(19) Europäisches Patentamt
European Patent Office
Office européen des brevets

(11) EP 3 932 923 B1

(12) EUROPEAN PATENT SPECIFICATION

(45) Date of publication and mention of the grant of the patent:
**03.05.2023 Bulletin 2023/18**

(21) Application number: **20774324.6**

(22) Date of filing: **19.03.2020**

(51) International Patent Classification (IPC):
***C07D 487/04*** *(2006.01)* ***A61P 35/00*** *(2006.01)*
***A61K 31/519*** *(2006.01)*

(52) Cooperative Patent Classification (CPC):
**C07D 487/04; A61P 35/00**

(86) International application number:
**PCT/CN2020/080198**

(87) International publication number:
**WO 2020/187291 (24.09.2020 Gazette 2020/39)**

(54) **PYRAZOLOPYRIMIDINE COMPOUND, PHARMACEUTICAL COMPOSITION, AND APPLICATION THEREFOR**

PYRAZOLOPYRIMIDINVERBINDUNG, PHARMAZEUTISCHE ZUSAMMENSETZUNG UND DEREN ANWENDUNG

COMPOSÉ DE PYRAZOLOPYRIMIDINE, COMPOSITION PHARMACEUTIQUE ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2019 CN 201910210021**
**19.03.2019 CN 201910210020**
**19.03.2019 CN 201910210015**

(43) Date of publication of application:
**05.01.2022 Bulletin 2022/01**

(73) Proprietor: **Central China Normal University**
**Wuhan, Hubei 430079 (CN)**

(72) Inventors:
- **YANG, Guangfu**
  **wuhan, Hubei 430079 (CN)**
- **HUANG, Wei**
  **wuhan, Hubei 430079 (CN)**
- **ZHUO, Linsheng**
  **wuhan, Hubei 430079 (CN)**
- **XU, Hongchuang**
  **wuhan, Hubei 430079 (CN)**
- **WANG, Mingshu**
  **wuhan, Hubei 430079 (CN)**

(74) Representative: **reuteler & cie SA**
**Chemin de la Vuarpillière 29**
**1260 Nyon (CH)**

(56) References cited:
**WO-A1-2016/097869**

- **ZHUO LIN-SHENG ET AL.: "Discovery of Next-Generation Tropomyosin Receptor Kinase Inhibitors for Combating Multiple Resistance Associated with Protein Mutation", J. MED. CHEM., vol. 64, no. 20, 20 October 2021 (2021-10-20), pages 15503-15514, XP055911444, US ISSN: 0022-2623, DOI: 10.1021/acs.jmedchem.1c01539**
- **BAILEY ET AL.: "Tropomyosin receptor kinase inhibitors: an updated patent review for 2010-2016 ? Part I", EXPERT OPIN. THER. PATENTS, vol. 27, no. 6, 8 March 2017 (2017-03-08), pages 733-751, XP055453922, GB ISSN: 1354-3776, DOI: 10.1080/13543776.2017.1297796**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 932 923 B1

## Description

### Technical Field

The invention relates to the field of biomedicine, and particularly relates to a pyrazolopyrimidine compound, a pharmaceutical composition containing the pyrazolopyrimidine compound, and application of the pyrazolopyrimidine compound and the pharmaceutical composition.

### Background

NTRK/TRK (Tropomosin receptor kinase) is neurotrophic factor tyrosine receptor, belonging to receptor tyrosine kinase family. The TRK family consists primarily of 3 members, NTRK1/TRKA, NTRK2/TRKB, and NTRK 3/TRKC. The complete TRK kinase comprises three parts, namely an extracellular region, a transmembrane region and an intracellular region. After the extracellular region of TRK kinase is combined with a corresponding ligand, the configuration change of the kinase can be caused, and a dimer is formed. The intracellular region of TRK kinase is autophosphorylated to activate the kinase activity of itself, and further activate the downstream signal transduction pathway (such as MAPK, AKT, PKC and the like) to generate corresponding biological functions; wherein NGF (nerve growth factor) binds TRKA, BDNF (derived neurotrophic factor) binds TRKB, and NT3 (neurotrophic factor 3) binds TRKC.

TRK kinases play important physiological roles in the development of nerves, including the growth and functional maintenance of neuronal axons, the development of memory, and the protection of neurons from injury, among others. Meanwhile, a large number of researches show that activation of TRK signal transduction pathways is closely related to occurrence and development of tumors, and activated TRK signal proteins are found in neurocytoma, prostatic cancer, breast cancer and the like.

In recent years, the discovery of various TRK fusion proteins further shows the biological function of promoting tumorigenesis. The earliest TPM3-TRKA fusion protein was found in colon cancer cells, with an incidence of about 1.5% in the clinical patients tested. Later, different types of TRK fusion proteins were found in different types of clinical tumor patient samples, such as lung cancer, head and neck cancer, breast cancer, thyroid cancer, glioma, etc., such as CD74-NTRK1, MPRIP-NTRK1, QKI-NTRK2, ETV6-NTRK3, BTB1-NTRK3, etc. Under the condition that ligand binding is not needed, the different NTRK fusion proteins are in a highly activated kinase activity state, so that downstream signal pathways can be continuously phosphorylated, cell proliferation is induced, and the generation and development of tumors are promoted.

Therefore, in recent years, TRK fusion proteins have become an effective anticancer target and a research hotspot, for example, WO2010048314, WO2012116217, WO2011146336, WO2010033941, WO2018077246, WO2016097869A1 and the like all disclose TRK kinase inhibitors with different structural types, and *Bailey et al.* reviewed the development and application of tropomyosin receptor kinase (TRK) inhibitors (Bailey et al. EXPERT OPIN. THER. PATENTS 2017, 27, 733-751, XP055453922).

Furthermore, the occurrence of target mutations after continuous administration is a significant cause of tumor resistance, and recent clinical cases of TRK mutations, such as TRKA G595R, G667C, G667S and F589L (Russo M et al; Cancer Discovery, 2016, 6(1), 36-44), TRKC G623R and G696A (Drilon A. et al Annals of Oncology 2016, 27(5), 920-926), have been observed, and the search for new TRK kinase inhibitors is expected to solve the problem of tumor resistance caused by TRK mutations.

In addition, nitrogen-containing aromatic heterocycles are generally preferred for their potency, a typical example being the ALK kinase inhibitor crizotinib (Cui J. et al. J. Med. chem. 2011, 54, 6342-6363). WO2007147647 and WO2007025540 also disclose pyrazole substituted pyrazolopyridine compounds and pyrazole substituted imidazopyridazine compounds, respectively, as ALK kinase inhibitors and their use in the treatment of disease.

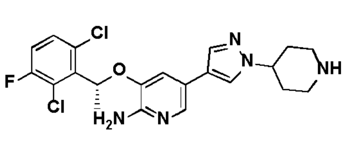

Crizotinib

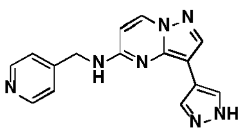

typical compound A
WO2007147647

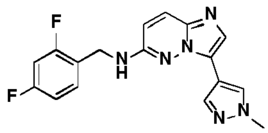

typical compound B
WO2007025540

### Disclosure of Invention

An object of the present invention is to provide a novel pyrazolopyrimidine compound having an excellent antitumor activity.

Although the typical compound A and the typical compound B provided in the prior art have good inhibitory

activity on ALK kinase, the inhibitory effect of structural analogs represented by the typical compound A and the typical compound B on TRK kinase is not good. Through a large number of scientific researches, the inventor of the invention finds that the pyrazolopyrimidine compound having the structure shown in formula (I) of the invention has excellent inhibitory activity on TRK kinase, and the inhibitory activity is obviously superior to that of the typical compound A and the typical compound B in the prior art. More importantly, the pyrazolopyrimidine compound of the invention has obviously better antitumor activity on animal level than the typical compound A and the typical compound B, thereby showing more excellent tumor treatment effect than the prior art.

In order to achieve the above objects, a first aspect of the present invention provides a pyrazolopyrimidine compound having a structure represented by formula (I) or a pharmaceutically acceptable salt thereof, or a stereoisomer, a geometric isomer, a tautomer, a nitrogen oxide, a hydrate, a solvate, thereof,

formula (I) 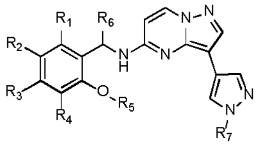 ,

wherein, in the formula (I),

$R_1$, $R_2$, $R_3$ and $R_4$ are each independently selected from H, halogen, alkyl of $C_{1-12}$, alkyl of $C_{1-12}$ substituted by 1-6 halogen;
$R_5$ is selected from H, alkyl of $C_{1-12}$, alkyl of $C_{1-12}$ substituted by 1-6 halogens, alkyl of $C_{1-12}$ substituted by hydroxyl, alkyl of $C_{2-12}$ substituted by alkoxy, alkyl of $C_{2-12}$ substituted by cyano, and cycloalkyl of $C_{2-12}$ containing 1-3 heteroatoms selected from N, O and S;
$R_6$ is selected from the group consisting of H, alkyl of $C_{1-12}$, alkyl of $C_{1-12}$ substituted by hydroxyl, and halogen;
$R_7$ is selected from H, alkyl of $C_{1-12}$, alkyl of $C_{1-12}$ substituted by 1-6 halogens, alkyl of $C_{1-12}$ substituted by hydroxyl, alkyl of $C_{2-12}$ substituted by cyano, cycloalkyl of $C_{2-12}$ containing 1-3 hetero atoms selected from N, O and S, acyl of $C_{2-12}$ and sulfonyl.

A third aspect of the present invention provides a pharmaceutical composition, which comprises a pharmaceutically acceptable carrier, excipient or diluent, and as an active ingredient, a pyrazolopyrimidine compound having a structure represented by formula (I) according to the first aspect of the present invention or a pharmaceutically acceptable salt thereof, or a stereoisomer, a geometric isomer, a tautomer, a nitrogen oxide, a hydrate, a solvate, thereof.

In a fifth aspect, the present invention provides a pyrazolopyrimidine compound having a structure represented by formula (I) or a pharmaceutically acceptable salt thereof, or a stereoisomer, a geometric isomer, a tautomer, an oxynitride, a hydrate, a solvate as described in the first aspect, or an application of a composition of matter as described in the third aspect, useful in the preparation of a medicament for preventing and/or treating tumors.

The pyrazolopyrimidine compound having a structure shown in formula (I) or a pharmaceutically acceptable salt thereof, or a stereoisomer, a geometric isomer, a tautomer, a nitrogen oxide, a hydrate, a solvate, provided by the invention, has excellent inhibitory activity on TRK kinase, and simultaneously has good antitumor activity on an animal level.

Detailed Description

The endpoints of the ranges and any values disclosed herein are not limited to the precise range or value, and these ranges or values should be understood to encompass values close to these ranges or values. For numerical ranges, each range between its endpoints and individual point values, and each individual point value can be combined with each other to give one or more new numerical ranges, and such numerical ranges should be construed as specifically disclosed herein.

As previously noted, a first aspect of the present invention provides a pyrazolopyrimidine compound or a pharmaceutically acceptable salt thereof, or a stereoisomer, a geometric isomer, a tautomer, an oxynitride, a hydrate, a solvate thereof.

Some terms involved in the present invention are explained below:

"$C_{1-12}$ alkyl" refers to alkyl groups having a total number of carbon atoms of 1 to 12, including straight chain, branched chain or cyclic alkyl groups, for example straight chain, branched chain or cyclic alkyl groups which may be 1, 2, 3, 4, 5, 6, 7, 8, 9, 10, 11 or 12 total carbon atoms, for example methyl, ethyl, n-propyl, isopropyl, n-butyl, isobutyl, tert-butyl, n-pentyl, isopentyl, n-hexyl, cyclopropyl, methylcyclopropyl, ethylcyclopropyl, cyclopentyl, methylcyclopentyl, cyclohexyl and the like. The same applies to "$C_{1-8}$ alkyl" and "$C_{1-6}$ alkyl", except that the number of carbon atoms is different.

" $C_{1-12}$ alkyl substituted with 1-6 halogens " refers to alkyl groups having a total number of carbon atoms of 1 to 12, including straight chain alkyl, branched chain alkyl, or cycloalkyl groups, and at least one H in the $C_{1-12}$ alkyl group is substituted by a halogen atom selected from halogen. For example, 1, 2, 3, 4, 5 or 6 H in the $C_{1-12}$ alkyl group are substituted by any one or more of the halogen atoms selected from fluorine, chlorine, bromine, and iodine. For example, trifluoromethyl, difluoromethyl, monofluoromethyl, monofluoroethyl, difluoroethyl, trifluoroethyl, etc. There are similar explanations for "$C_{1-8}$ alkyl substituted with 1-6 halogens" and "$C_{1-6}$ alkyl substituted with 1-6 halogens", except that the number of carbon atoms is different.

"$C_{1-12}$ alkyl group substituted with hydroxyl" means an alkyl group having a total of 1-12 carbon atoms, including straight chain alkyl, branched chain alkyl or cycloalkyl, and at least one H in the $C_{1-12}$ alkyl groups is substituted by hydroxyl.

"$C_{2-12}$ alkyl group substituted with alkoxy" represents a group having 2 to 12 carbon atoms in total, and the structural formula of the group may be represented by $-R^1OR^2$, wherein the sum of the carbon atoms in $R^1$ and $R^2$ is 2 to 12, and $R^1$ is directly bonded to the phenoxy group in the pyrazolopyrimidine compound of the structure represented by formula (I) in the present invention.

"cyano-substituted $C_{2-12}$ alkyl" means an alkyl group having a total number of carbon atoms of 2 to 12, including straight chain alkyl, branched chain alkyl, or cycloalkyl, and at least one H in the $C_{2-12}$ alkyl group is substituted with a cyano group, and the number of carbon atoms in the "cyano" is counted in the total number of carbon atoms in the group.

"$C_{2-12}$ cycloalkyl group containing 1 to 3 heteroatoms selected from N, O and S" means a cycloalkyl group having a total number of carbon atoms of 2 to 12, and 1 to 3 of the atoms forming the ring are heteroatoms selected from N, O and aforementioned range of the total number of carbon atoms. The "$C_{2-12}$ cycloalkyl group containing 1 to 3 heteroatoms selected from N, O and S "may be, for example, a three-membered ring, a four-membered ring, a five-membered ring, a six-membered ring, a seven-membered ring, an eight-membered ring, a nine-membered ring, a ten-membered ring, an eleven-membered ring or a twelve-membered ring, and H in the cycloalkyl group may be optionally substituted or unsubstituted, if substituted, with at least one substituent independently selected from halogen, hydroxyl, nitro and mercapto.

"$C_{2-12}$ acyl" means an acyl group having 2-12 carbon atoms in total, and may be, for example, acetyl group, propionyl group or the like.

The "sulfonyl group" may contain a $C_{1-6}$ alkyl group, and the sulfonyl group may be represented by-$SO_2R^3$, wherein $R^3$ may be a $C_{1-6}$ alkyl group.

In the pyrazolopyrimidine compound of the formula (I) according to the present invention, $R_1$, $R_2$, $R_3$ and $R_4$ are preferably independently selected from the group consisting of H, fluoro, chloro, bromo, $C_{1-8}$ alkyl, $C_{1-8}$ alkyl substituted with 1-6 halogens selected from fluoro, chloro and bromo; more preferably, $R_1$, $R_2$, $R_3$ and $R_4$ are each independently selected from the group consisting of H, fluorine, chlorine, bromine, $C_{1-6}$ alkyl, $C_{1-6}$ alkyl substituted with 1-4 halogens selected from the group consisting of fluorine, chlorine and bromine; further preferably, $R_1$, $R_3$ and $R_4$ are each independently selected from the group consisting of H, fluorine, chlorine, bromine, $C_{1-8}$ alkyl, $C_{1-8}$ alkyl substituted by 1-6 halogens selected from the group consisting of fluorine, chlorine and bromine; and $R_2$ is H or F.

According to another preferred embodiment, in the pyrazolopyrimidine compound of the formula (I) according to the present invention, $R_1$, $R_3$ and $R_4$ are preferably each independently selected from the group consisting of H, halogen, $C_{1-12}$ alkyl, $C_{1-12}$ alkyl substituted with 1-6 halogen; and $R_2$ is halogen; more preferably, $R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo; and $R_2$ is F; further preferably, $R_1$, $R_3$ and $R_4$ are all H; $R_2$ is F.

In the pyrazolopyrimidine compound of the formula (I) according to the present invention, $R_5$ is preferably selected from the group consisting of H, $C_{1-8}$ alkyl, $C_{1-8}$ alkyl substituted with 1-6 halogens selected from fluorine, chlorine and bromine, $C_{1-8}$ alkyl substituted with hydroxy, $C_{2-8}$ alkyl substituted with alkoxy, $C_{2-8}$ alkyl substituted with cyano, and $C_{2-10}$ cycloalkyl containing 1-3 heteroatoms selected from N, O and S; more preferably, $R_5$ is selected from the group consisting of H, $C_{1-6}$ alkyl, $C_{1-6}$ alkyl substituted with 1-4 halogens selected from fluorine, chlorine and bromine, $C_{1-6}$ alkyl substituted with hydroxy, $C_{2-8}$ alkyl substituted with alkoxy, $C_{2-6}$ alkyl substituted with cyano, $C_{2-8}$ cycloalkyl containing 1-3 heteroatoms selected from N, O and S; further preferably, $R_5$ is selected from $C_{1-12}$ alkyl substituted with 1-6 halogens; further preferably, $R_5$ is selected from $C_{1-8}$ alkyl substituted with 1-6 halogens selected from fluorine, chlorine and bromine; more preferably, $R_5$ is $-CH_2CHF_2$.

In the pyrazolopyrimidine compound of the formula (I) according to the present invention, $R_6$ is preferably selected from the group consisting of H, $C_{1-12}$ alkyl, hydroxy-substituted $C_{1-12}$ alkyl and halogen; more preferably, $R_6$ is selected from the group consisting of H, $C_{1-8}$ alkyl, hydroxy substituted $C_{1-8}$ alkyl and halogen; further preferably, $R_6$

is selected from the group consisting of H, $C_{1-6}$ alkyl, hydroxy substituted $C_{1-6}$ alkyl and halogen.

In the pyrazolopyrimidine compound of the formula (I) according to the present invention, $R_7$ is preferably selected from the group consisting of H, $C_{1-12}$ alkyl, $C_{1-12}$ alkyl substituted with 1-6 halogens, $C_{1-12}$ alkyl substituted with hydroxy, $C_{2-12}$ alkyl substituted with cyano, $C_{2-12}$ cycloalkyl containing 1-3 heteroatoms selected from N, O and S, $C_{2-12}$ acyl, and sulfonyl; more preferably, $R_7$ is selected from the group consisting of H, $C_{1-8}$ alkyl, $C_{1-8}$ alkyl substituted with 1-6 halogens selected from fluorine, chlorine and bromine, $C_{1-8}$ alkyl substituted with hydroxy, $C_{2-8}$ alkyl substituted with cyano, $C_{2-10}$ cycloalkyl containing 1-3 heteroatoms selected from N, O and S, $C_{2-8}$ acyl, sulfonyl; further preferably, $R_7$ is selected from the group consisting of H, $C_{1-6}$ alkyl, $C_{1-6}$ alkyl substituted with 1-4 halogens selected from fluorine, chlorine and bromine, $C_{1-6}$ alkyl substituted with hydroxy, $C_{2-6}$ alkyl substituted with cyano, $C_{2-8}$ cycloalkyl containing 1-3 heteroatoms selected from N, O and S, $C_{2-6}$ acyl, sulfonyl; $R_7$ is particularly preferably H.

In particular, the inventors of the present invention found that when $R_2$ is halogen, the pyrazolopyrimidine compound of the structure represented by formula (I) provided by the present invention exhibits higher inhibitory activity against TRK kinase, particularly against mutated TRK kinase; meanwhile, the compound has more reasonable pharmacokinetic property and more excellent in-vivo antitumor activity.

Several preferred embodiments of the pyrazolopyrimidine compound of the structure of formula (I) of the invention are provided below:

Embodiment mode 1:
in the formula (I),

$R_1$, $R_2$, $R_3$ and $R_4$ are each independently selected from the group consisting of H, fluorine, chlorine, bromine, $C_{1-8}$ alkyl, $C_{1-8}$ alkyl substituted with 1-6 halogens selected from the group consisting of fluorine, chlorine and bromine;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and cycloalkyl of $C_{2-10}$ containing 1-3 hetero atoms selected from N, O and S;
$R_6$ is selected from the group consisting of H, $C_{1-8}$ alkyl, hydroxy substituted $C_{1-8}$ alkyl and halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 hetero atoms selected from N, O and S, $C_{2-8}$ acyl and sulfonyl.

Embodiment mode 2:
in the formula (I),

$R_1$, $R_2$, $R_3$ and $R_4$ are each independently selected from the group consisting of H, fluorine, chlorine, bromine, $C_{1-6}$ alkyl, $C_{1-6}$ alkyl substituted with 1-4 halogens selected from the group consisting of fluorine, chlorine and bromine;
$R_5$ is selected from H, $C_{1-6}$ alkyl, $C_{1-6}$ alkyl substituted by 1-4 halogens selected from fluorine, chlorine and bromine, $C_{1-6}$ alkyl substituted by hydroxyl, $C_{2-8}$ alkyl substituted by alkoxy, $C_{2-6}$ alkyl substituted by cyano, and $C_{2-8}$ cycloalkyl containing 1-3 heteroatoms selected from N, O and S;
$R_6$ is selected from the group consisting of H, $C_{1-6}$ alkyl, $C_{1-6}$ alkyl substituted by hydroxyl, halogen;
$R_7$ is selected from H, $C_{1-6}$ alkyl, $C_{1-6}$ alkyl substituted by 1-4 halogens selected from fluorine, chlorine and bromine, $C_{1-6}$ alkyl substituted by hydroxyl, $C_{2-6}$ alkyl substituted by cyano, $C_{2-8}$ cycloalkyl containing 1-3 hetero atoms selected from N, O and S, $C_{2-6}$ acyl, sulfonyl.
Further preferably, in the foregoing embodiment mode 1 and the foregoing embodiment mode 2, it is more preferable that at least one group of $R_1$, $R_2$, $R_3$, $R_4$, and $R_5$ contains a F atom; and $R_5$ is selected from $C_{1-12}$ alkyl substituted with 1-6 halogens; further preferably, at least one of $R_1$, $R_2$, $R_3$, $R_4$, and $R_5$ contains an F atom; and $R_5$ is selected from $C_{1-8}$ alkyl substituted with 1-6 halogens selected from fluorine, chlorine and bromine.

Embodiment mode 3:
in the formula (I),

$R_1$, $R_2$, $R_3$ and $R_4$ are each independently selected from H, halogen, $C_{1-12}$ alkyl, $C_{1-12}$ alkyl substituted by 1-6 halogen;
$R_5$ is $-CH_2CHF_2$;
$R_6$ is selected from the group consisting of $C_{1-12}$ alkyl, $C_{1-12}$ alkyl substituted with hydroxyl, halogen;
$R_7$ is selected from H, $C_{1-12}$ alkyl, $C_{1-12}$ alkyl substituted by 1-6 halogens, $C_{1-12}$ alkyl substituted by hydroxyl, $C_{2-12}$ alkyl substituted by cyano, $C_{2-12}$ cycloalkyl containing 1-3 hetero atoms selected from N, O and S, $C_{2-12}$

acyl, sulfonyl.

Embodiment mode 4:
in the formula (I),

$R_1$, $R_2$, $R_3$ and $R_4$ are each independently selected from the group consisting of H, fluorine, chlorine, bromine, $C_{1-8}$ alkyl, $C_{1-8}$ alkyl substituted with 1-6 halogens selected from the group consisting of fluorine, chlorine and bromine;
$R_5$ is $-CH_2CHF_2$;
$R_6$ is selected from the group consisting of $C_{1-8}$ alkyl, $C_{1-8}$ alkyl substituted by hydroxyl, halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{1-8}$ alkyl substituted by cyano, $C_{2-10}$ cycloalkyl containing 1-3 hetero atoms selected from N, O and S, $C_{2-8}$ acyl, sulfonyl.

Embodiment mode 5:

$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;
$R_2$ is H or F;
$R_5$ is-$CH_2CHF_2$;
$R_6$ is selected from the group consisting of $C_{1-8}$ alkyl, $C_{1-8}$ alkyl substituted with hydroxy, halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 hetero atoms selected from N, O and S, $C_{2-8}$ acyl, sulfonyl.

Embodiment mode 6: The compound with the structure shown in the formula (I) is selected from at least one of the following compounds:

compound 1： 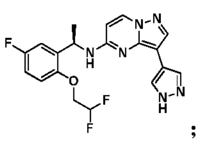 ; compound 2： 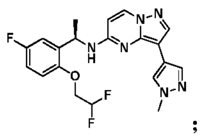 ; compound 3： 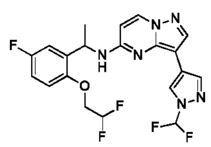 ;

compound 4： 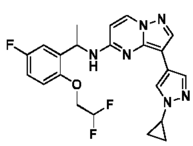 ; compound 5： 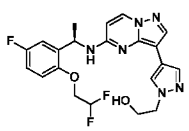 ; compound 6： 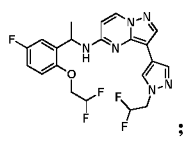 ;

compound 7： 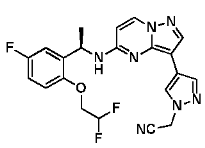 ; compound 8： 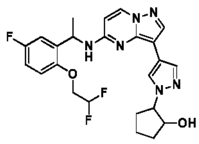 ; compound 9： 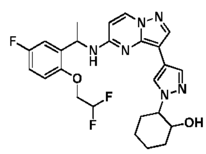 ;

compound 10： 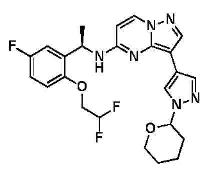 ; compound 11： 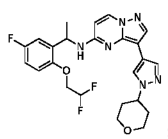 ; compound 12： 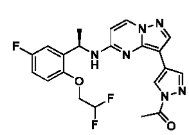 ;

compound 13： 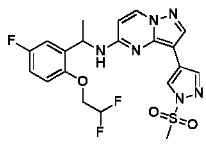 ; compound 14： 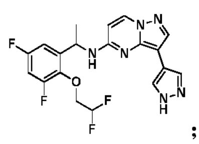 ; compound 15： 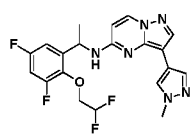 ;

compound 16: 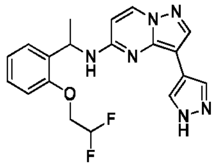 ; compound 17: 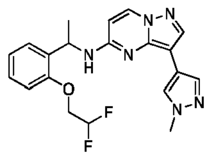 .

More preferably, in the aforementioned embodiment mode 1 and the aforementioned embodiment mode 2, $R_7$ is more preferably selected from the group consisting of $C_{1-12}$ alkyl, $C_{1-12}$ alkyl substituted with 1-6 halogens, $C_{1-12}$ alkyl substituted with hydroxy, $C_{2-12}$ alkyl substituted with cyano, $C_{2-12}$ cycloalkyl containing 1-3 heteroatoms selected from N, O and S, $C_{2-12}$ acyl, sulfonyl; further preferably, $R_7$ is selected from the group consisting of $C_{1-6}$ alkyl, $C_{1-6}$ alkyl substituted with 1-4 halogens selected from fluorine, chlorine and bromine, $C_{1-6}$ alkyl substituted with hydroxy, $C_{2-6}$ alkyl substituted with cyano, $C_{2-8}$ cycloalkyl containing 1-3 heteroatoms selected from N, O and S, $C_{2-6}$ acyl, sulfonyl.

Embodiment mode 7:

in the formula (I),

$R_1$, $R_3$ and $R_4$ are each independently selected from the group consisting of H, halogen, $C_{1-12}$ alkyl, $C_{1-12}$ alkyl substituted with 1-6 halogen;

$R_2$ is halogen;

$R_5$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by alkoxy, $C_{2-12}$ alkyl substituted by cyano, and $C_{2-12}$ cycloalkyl containing 1-3 heteroatoms selected from N, O and S;

$R_6$ is selected from the group consisting of $C_{1-12}$ alkyl, $C_{1-12}$ alkyl substituted with hydroxyl, halogen;

$R_7$ is selected from $C_{1-12}$ alkyl, $C_{1-12}$ alkyl substituted by 1-6 halogens, $C_{1-12}$ alkyl substituted by hydroxy, $C_{2-12}$ alkyl substituted by cyano, $C_{2-12}$ cycloalkyl containing 1-3 heteroatoms selected from N, O and S, $C_{2-12}$ acyl, sulfonyl.

Embodiment mode 8:

in the formula (I),

$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;

$R_2$ is F;

$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;

$R_6$ is selected from the group consisting of $C_{1-8}$ alkyl, hydroxy substituted $C_{1-8}$ alkyl, halogen;

$R_7$ is selected from $C_{1-8}$ alkyl, $C_{1-8}$ alkyl substituted by 1-6 halogens selected from fluorine, chlorine and bromine, $C_{1-8}$ alkyl substituted by hydroxyl, $C_{1-8}$ alkyl substituted by cyano, $C_{2-10}$ cycloalkyl containing 1-3 heteroatoms selected from N, O and S, $C_{2-8}$ acyl, sulfonyl.

Embodiment mode 9:

in the formula (I),

$R_1$, $R_3$ and $R_4$ are all H; $R_2$ is F;

$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and cycloalkyl of C2-10 containing 1-3 hetero atoms selected from N, O and S;

$R_6$ is selected from the group consisting of $C_{1-8}$ alkyl, $C_{1-8}$ alkyl substituted with hydroxyl, halogen;

$R_7$ is selected from $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 heteroatoms selected from N, O and S, $C_{2-8}$ acyl, sulfonyl.

Embodiment mode 10: The compound with the structure shown in the formula (I) is selected from at least one of the following compounds:

compound a1-2： 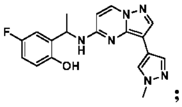 ； compound a2-3： 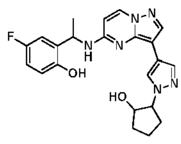 ； compound a3-4： 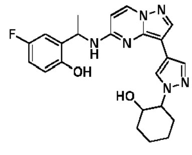 ；

compound a4-8： 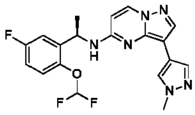 ； compound a5-9： 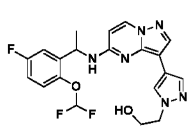 ； compound a6-10： 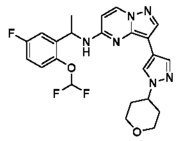 ；

compound a7-12： 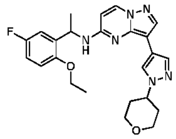 ； compound a8-13： 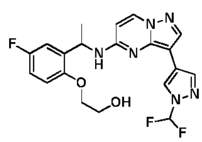 ； compound a9-14： 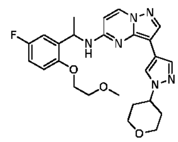 ；

compound a10-17： 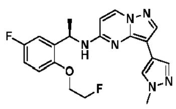 ； compound a11-18： 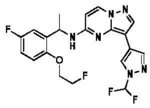 ； compound a12-19： 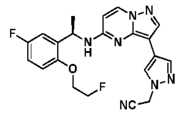 ；

compound a13-20： 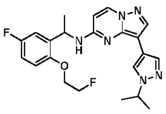 ； compound a14-21： 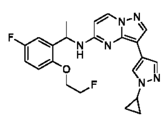 ； compound a15-22： 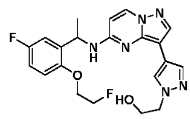 ；

compound a16-23： 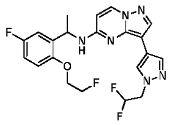 ； compound a17-24： 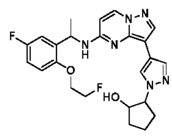 ； compound a18-25： 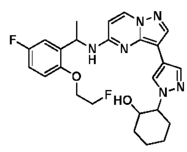 ；

compound a19-26： 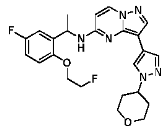 ； compound a20-27： 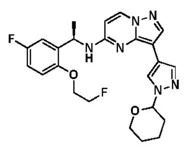 ； compound a21-28： 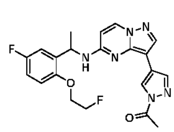 ；

compound a22-29： 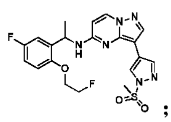 ； compound a23-31： 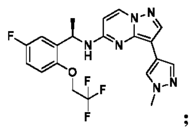 ； compound a24-32： 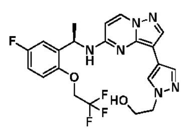 ；

compound a25-35： 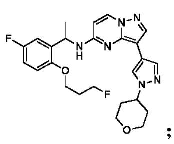 ; compound a26-38： 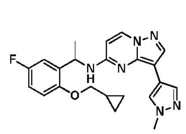 ; compound a27-41： 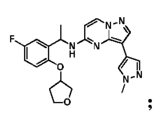 ; compound a28-43： 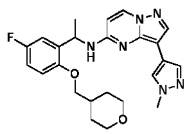 .

More preferably, in the above mode embodiment 1 and embodiment mode 2, more preferably, $R_7$ is H.
Embodiment mode 11:
in the formula (I),

$R_1$, $R_3$ and $R_4$ are each independently selected from the group consisting of H, halogen, $C_{1-12}$ alkyl, $C_{1-12}$ alkyl substituted with 1-6 halogen;
$R_2$ is halogen;
$R_5$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by alkoxy, $C_{2-12}$ alkyl substituted by cyano, and $C_{2-12}$ cycloalkyl containing 1-3 heteroatoms selected from N, O and S;
$R_6$ is selected from the group consisting of $C_{1-12}$ alkyl, hydroxy substituted $C_{1-12}$ alkyl and halogen.
Embodiment mode 12:
in the formula (I),
$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;
$R_2$ is F;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted with hydroxyl, halogen.

Embodiment mode 13:
in the formula (I),

$R_1$, $R_3$ and $R_4$ are all H; $R_2$ is F;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted with hydroxyl, halogen.

Embodiment mode 14: The compound with the structure shown in the formula (I) is selected from at least one of the following compounds:

compound b1-1: 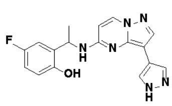; compound b2-5: 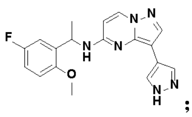; compound b3-6: 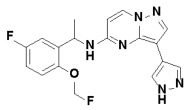; compound b4-7: 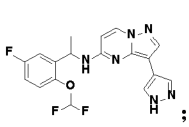; compound b5-11: 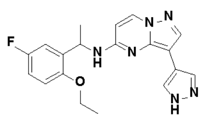; compound b6-15: 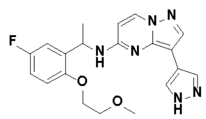;

compound b7-16: 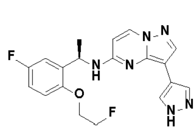; compound b8-30: 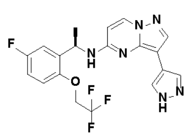; compound b9-33: 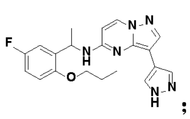;

compound b10-34: 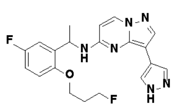; compound b11-36: 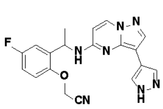; compound b12-37: 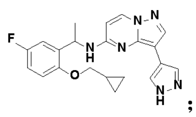;

compound b13-39: 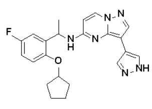 ; compound b14-40: 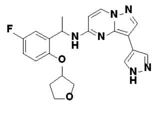 ; compound b15-42: 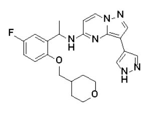 ; compound b16-44: 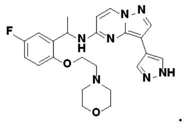 .

Among the above specific compounds, those having no specific configuration of the chiral center are represented as racemates.

The process for producing a pyrazolopyrimidine compound having the structure represented by formula (I) in the present invention is not particularly limited, and can be produced, for example, by the following production process:

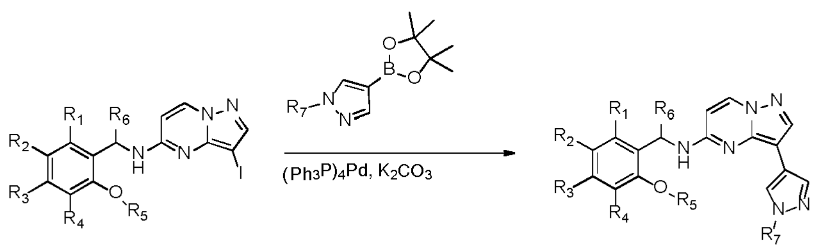


The preparation method involves Suzuki coupling reaction, the reaction conditions of the coupling reaction are not particularly limited, and those skilled in the art can obtain appropriate reaction conditions according to common general knowledge in the field of organic synthesis and specific examples provided in the examples section of the present invention.

As described above, the third aspect of the present invention provides a pharmaceutical composition comprising a pharmaceutically acceptable carrier, excipient or diluent, and, as an active ingredient, a pyrazolopyrimidine compound having a structure represented by formula (I) or a pharmaceutically acceptable salt thereof, or a stereoisomer, a geometric isomer, a tautomer, a nitrogen oxide, a hydrate, a solvate thereof, according to the first aspect of the present invention.

As described above, the fifth aspect of the present invention provides a pyrazolopyrimidine compound having a structure represented by formula (I) or a pharmaceutically acceptable salt thereof, or a stereoisomer, a geometric isomer, a tautomer, a nitrogen oxide, a hydrate, a solvate, according to the first aspect of the present invention, or a pharmaceutical composition according to the third aspect of the present invention, for use in the preparation of a medicament for the prevention and/or treatment of tumors.

Preferably, the tumor is at least one of breast cancer, large intestine cancer, lung cancer, thyroid cancer, skin cancer, bone cancer, melanoma, leukemia, salivary gland tumor, neuroendocrine tumor, lymphoma, brain tumor, neuroblastoma, ovarian cancer, pancreatic cancer, mesothelioma, esophageal cancer, pulmonary sarcoma, medulloblastoma, glioblastoma, colon cancer, hepatoma, retinoblastoma, renal carcinoma, bladder cancer, osteosarcoma, gastric cancer, uterine cancer, vulval cancer, small intestine cancer, prostate cancer, bile duct cancer, ureter cancer, adrenal cortex cancer, or head and neck cancer.

The present invention will be described in detail below by way of examples. In the following examples, the various starting materials used are commercially available and of analytical purity, unless otherwise specified.

Example 1: Preparation of (R) -N- (1- (2- (2, 2-difluoroethoxy) -5-fluorophenyl) ethyl) -3- (1H-pyrazol-4-yl) pyrazolo [1,5-a] pyrimidin-5-amine (compound 1)

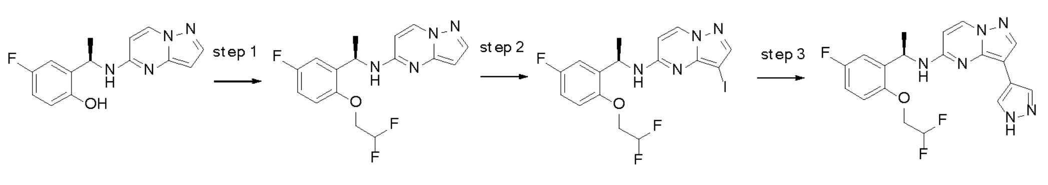

Step 1): (R) 4-fluoro-2-(1-(pyrazolo [1,5-a] pyrimidin-5-ylamino) ethyl) phenol (11.0 mmol), 1,1-difluoro-2-iodoethane (16.5 mmol), cesium carbonate (22 mmol) were added to a 200 mL pear-shaped bottle, to which DMF (50 mL) was added. Heated overnight in an oil bath (100 °C). The reaction was cooled to ambient temperature and the crude product was purified by column chromatography to give a white solid with a yield of 90%.

Step 2): (R) -N- (1- (2- (2, 2-difluoroethoxy) -5-fluorophenyl) ethyl) pyrazolo [1,5-a] pyrimidin-5-amine (9.8 mmol) was added to a 200 mL pear-shaped bottle, to which was added acetonitrile (50 mL). N-iodosuccinimide (NIS, 14.85 mmol) was added under magnetic stirring at room temperature. The reaction was carried out at room temperature for 1h and TLC monitored for completion. After removing acetonitrile as much as possible under reduced pressure, the mixture was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 3): (R) -N- (1- (2- (2, 2-difluoroethoxy) -5-fluorophenyl) ethyl) -3-iodopyrazolo [1,5-a] pyrimidin-5-amine (0.50 mmol), 1-Boc-pyrazole-4-boronic acid pinacol ester (0.75 mmol), anhydrous potassium carbonate (2.00 mmol), tetrakis (triphenylphosphine) palladium (0.05 mmol) were added to a 100 mL reaction tube, replaced with argon for 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 50%.

$^1$H NMR (400 MHz, DMSO-$d_6$) δ 12.71 (s, 1 H), 8.49 (d, *J* = 7.6 Hz, 1 H), 8.08 (s, 1 H), 8.04 (d, *J* = 6.8 Hz, 1 H), 7.85 (s, 2 H), 7.16 - 6.99 (m, 3 H), 6.70 - 6.32 (m, 2 H), 5.63 - 5.44 (m, 1 H), 4.58 - 4.44 (m, 2 H), 1.47 (d, *J* = 6.8 Hz, 3 H).

Example 2: Preparation of (R) -N- (1- (2- (2, 2-difluoroethoxy) -5-fluorophenyl) ethyl) -3-(1-methyl-1H-pyrazol-4-yl) pyrazolo [1,5-a] pyrimidin-5-amine (compound 2)

The preparation method was the same as in example 1 except that 1-Boc-pyrazole-4-boronic acid pinacol ester was changed to 1-methylpyrazole-4-boronic acid pinacol ester.

$^1$H NMR (400 MHz, DMSO-$d_6$) δ 8.49 (d, *J* = 7.6 Hz, 1 H), 8.05 (s, 1 H), 8.02 (d, *J* = 9.2 Hz, 1 H), 7.75 (s, 2 H), 7.23 - 6.99 (m, 3 H), 6.70 - 6.30 (m, 2 H), 5.57 - 5.46 (m, 1 H), 4.49 (t, *J* = 14.4 Hz, 2 H), 3.85 (s, 3 H), 1.48 (d, *J* = 6.8 Hz, 3 H).

Example 3: Preparation of N- (1- (2- (2, 2-difluoroethoxy) -5-fluorophenyl) ethyl) -3- (1- (difluoromethyl) -1H-pyrazol-4-yl) pyrazolo [1,5-a] pyrimidin-5-amine (compound 3)

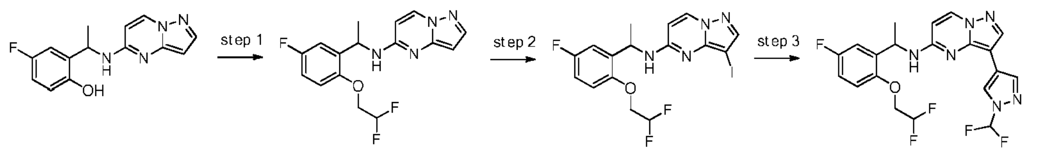


Step 1): the procedure of step 1 in example 1 was used.

Step 2): synthesis of N- (1- (2- (2, 2-difluoroethoxy) -5-fluorophenyl) ethyl) -3-iodopyrazolo [1,5-a] pyrimidin-5-amine using the procedure of step 2 in example 1.

And step 3): the synthesis method is the same as step 3 in example 1. Except that 1-Boc-pyrazole-4-boronic acid pinacol ester was replaced with 1- (difluoromethyl) -4- (4,4,5, 5-tetramethyl-1, 3, 2-dioxaborolan-2-yl) -1H-pyrazole.

$^1$H NMR (400 MHz, DMSO-$d_6$) δ 8.53 (d, *J* = 7.6 Hz, 1 H), 8.24 (s, 1 H), 8.19 (s, 1 H), 8.13 (d, *J* = 6.0 Hz, 2 H), 7.99 - 7.53 (m, 1 H), 7.21 - 6.96 (m, 3 H), 6.64 - 6.30 (m, 2 H), 5.55 - 5.46 (m, 1 H), 4.45 (ddq, *J* = 17.6, 11.2, 3.2 Hz, 2 H), 1.48 (d, *J* = 6.8 Hz, 3 H).

Example 4: Preparation of 3- (1-cyclopropyl-1H-pyrazol-4-yl) -N- (1- (2- (2, 2-difluoroethoxy) -5-fluorophenyl) ethyl) pyrazolo [1,5-a] pyrimidin-5-amine (compound 4)

The synthesis procedure is the same as in example 3, except that 1- (difluoromethyl) -4- (4,4,5, 5-tetramethyl-1, 3, 2-dioxaborolan-2-yl) -1H-pyrazole is replaced by 1-cyclopropylpyrazole-4-boronic acid pinacol ester.

$^1$H NMR (400 MHz, DMSO-$d_6$) δ 8.48 (d, *J* = 7.6 Hz, 1 H), 8.04 (s, 1 H), 8.00 (d, *J* = 6.8 Hz, 1 H), 7.81 (s, 1 H), 7.73 (s, 1 H), 7.25 - 6.96 (m, 3 H), 6.67 - 6.29 (m, 2 H), 5.55 - 5.44 (m, 1 H), 4.48 (t, *J* = 14.4 Hz, 2 H), 3.77 - 3.54 (m, 1 H), 1.47 (d, *J* = 6.8 Hz, 3 H), 1.08 - 0.96 (d, *J* = 6.4 Hz, 4 H).

Example 5: Preparation of (R) -2- (4- (5- ((1- (2- (2, 2-difluoroethoxy) -5-fluorophenyl) ethyl) amino) pyrazolo [1,5-a] pyrimidin-3-yl) -1H-pyrazol-1-yl) ethanol (compound 5)

The preparation was identical to example 1, except that 1-Boc-pyrazole-4-boronic acid pinacol ester was replaced with 4- (4,4,5, 5-tetramethyl-1, 3, 2-dioxaborolan-2-yl) -1H-pyrazole-1-ethanol.

$^1$H NMR (400 MHz, DMSO-$d_6$) δ 8.47 (d, *J* = 7.6 Hz, 1 H), 8.04 (s, 1 H), 7.98 (d, *J* = 6.8 Hz, 1 H), 7.77 (d, *J* = 11.2 Hz, 2 H), 7.19 - 6.96 (m, 3 H), 6.67 - 6.30 (m, 2 H), 5.60 - 5.41 (m, 1 H), 4.94 (t, *J* = 5.2 Hz, 1 H), 4.47 (qd, *J* = 12.8, 10.8, 5.6 Hz, 2 H), 4.11 (t, *J* = 5.6 Hz, 2 H), 3.73 (q, *J* = 5.6 Hz, 2 H), 1.45 (d, *J* = 6.8 Hz, 3 H).

Example 6: Preparation of N- (1- (2- (2, 2-difluoroethoxy) -5-fluorophenyl) ethyl) -3- (1- (2, 2-difluoroethyl) -1H-pyrazol-4-yl) pyrazolo [1,5-a] pyrimidin-5-amine (compound 6)

The procedure is as in example 3, except that 1- (difluoromethyl) -4- (4,4,5, 5-tetramethyl-1, 3, 2-dioxaborolan-2-yl) -1H-pyrazole is replaced by 1- (2, 2-difluoroethyl) -4- (4,4,5, 5-tetramethyl-1, 3, 2-dioxaborolan-2-yl) -1H-pyrazole. $^1$H NMR (400 MHz, DMSO-$d_6$) δ 8.50 (d, *J* = 7.6 Hz, 1 H), 8.09 (s, 1 H), 8.02 (d, *J* = 7.2 Hz, 1 H), 7.88 (d, *J* = 7.6 Hz, 2 H), 7.22 - 6.96 (m, 3 H), 6.70 - 6.14 (m, 3 H), 5.58 - 5.48 (m, 1 H), 4.59 (dt, *J* = 15.2, 9.2 Hz, 2 H), 4.46 (dt, *J* = 14.4, 3.6 Hz, 2 H), 1.48 (d, *J* = 6.8 Hz, 3 H).

Example 7: Preparation of ((R) -2- (4- (5- ((1- (2- (2, 2-difluoroethoxy) -5-fluorophenyl) ethyl) amino) pyrazolo [1,5-a] pyrimidin-3-yl) -1H-pyrazol-1-yl) acetonitrile (Compound 7)

The preparation was carried out as in example 1, except that 1-Boc-pyrazole-4-boronic acid pinacol ester was replaced by 2- [4- (4,4,5, 5-tetramethyl-1, 3, 2-dioxaborolan-2-yl) pyrazol-1-yl] acetonitrile.

$^1$H NMR (400 MHz, DMSO-$d_6$) δ 8.49 (d, *J* = 7.6 Hz, 1 H), 8.09 (s, 1 H), 8.05 (d, *J* = 7.2 Hz, 1 H), 7.91 (d, *J* = 7.6 Hz, 2 H), 7.21 - 6.97 (m, 3 H), 6.67 - 6.30 (m, 2 H), 5.54 - 5.42 (m, 3 H), 4.61 - 4.35 (m, 2 H), 1.45 (d, *J* = 7.2 Hz, 3 H).

Example 8: Preparation of 2- (4- (5- ((1- (2- (2, 2-difluoroethoxy) -5-fluorophenyl) ethyl) amino) pyrazolo [1,5-a] pyrimidin-3-yl) -1H-pyrazol-1-yl) cyclopentan-1-ol (compound 8)

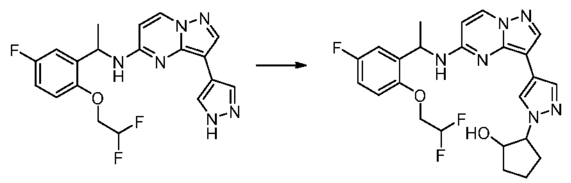

To a jar, N- (1- (2- (2, 2-difluoroethoxy) -5-fluorophenyl) ethyl) -3- (1H-pyrazol-4-yl) pyrazolo [1,5-a] pyrimidin-5-amine (0.22 mmol), 1, 2-epoxycyclopentane (0.22 mmol), anhydrous DMF (10 mL), and cesium carbonate (0.6 mmol) were added. Heated to reflux overnight in an oil bath (100 °C). The reaction was cooled to ambient temperature, concentrated under reduced pressure to remove DMF as much as possible to give a yellow oily crude product, which was purified by column chromatography to give a pale yellow solid with a yield of 60%.

$^1$H NMR (400 MHz, DMSO-$d_6$) δ 8.46 (d, *J* = 7.6 Hz, 1 H), 8.03 (d, *J* = 1.2 Hz, 1 H), 7.95 (t, *J* = 7.6 Hz, 1 H), 7.81 (d, *J* = 10.0 Hz, 1 H), 7.77 (s, 1 H), 7.20 - 6.97 (m, 3 H), 6.67 - 6.31 (m, 2 H), 5.55 - 5.44 (m, 1 H), 5.07 (dd, *J* = 6.4, 4.8 Hz, 1 H), 4.60 - 4.36 (m, 2 H), 4.34 - 4.10 (m, 2 H), 2.22 - 2.06 (m, 1 H), 2.03 - 1.88 (m, 2 H), 1.78 (p, *J* = 7.2 Hz, 2 H), 1.63 - 1.52 (m, 1 H), 1.45 *(d, J* = 6.8 Hz, 3 H).

Example 9: Preparation of 2- (4- (5- ((1- (2- (2, 2-difluoroethoxy) -5-fluorophenyl) ethyl) amino) pyrazolo [1,5-a] pyrimidin-3-yl) -1H-pyrazol-1-yl) cyclohexan-1-ol (compound 9)

The preparation was carried out as in example 8, but replacing 1, 2-epoxycyclopentane by 1, 2-epoxycyclohexane.

$^1$H NMR (400 MHz, DMSO-$d_6$) δ 8.49 (d, *J* = 7.6 Hz, 1 H), 8.05 (s, 1 H), 7.98 (t, *J* = 7.6 Hz, 1 H), 7.84 - 7.74 (m, 2 H), 7.23 - 6.97 (m, 3 H), 6.72 - 6.29 (m, 2 H), 5.60 - 5.47 (m, 1 H), 4.79 (dd, *J* = 11.6, 5.2 Hz, 1 H), 4.59 - 4.40 (m, 2 H), 3.86 - 3.67 (m, 2 H), 2.07 - 1.64 (m, 5 H), 1.58 - 1.17 (m, 6 H).

Example 10: Preparation of N- ((R) -1- (2- (2, 2-difluoroethoxy) -5-fluorophenyl) ethyl) -3- (1-(tetrahydro-2H-pyran-2-yl) -1H-pyrazol-4-yl) pyrazolo [1,5-a] pyrimidin-5-amine (compound 10)

The preparation method was the same as in example 1 except that 1-Boc-pyrazole-4-boronic acid pinacol ester was replaced with 1- (tetrahydropyran-2-yl) -1H-pyrazole-4-boronic acid pinacol ester.

$^1$H NMR (400 MHz, DMSO-$d_6$) δ 8.48 (d, *J* = 7.6 Hz, 1 H), 8.12 - 8.06 (m, 1 H), 8.04 - 7.96 (m, 1 H), 7.93 (s, 1 H), 7.83 (s, 1 H), 7.20 - 6.94 (m, 3 H), 6.68 - 6.30 (m, 2 H), 5.54 - 5.41 (m, 1 H), 5.41 - 5.28 (m, 1 H), 4.45 (td, *J* = 14.4, 3.6 Hz, 2 H), 3.98 - 3.84 (m, 1 H), 3.70 - 3.58 (m, 1 H), 2.15 - 1.84 (m, 3 H), 1.74 - 1.63 (m, 1 H), 1.58 - 1.50 (m, 2 H), 1.45 (d, *J* = 6.8 Hz, 3 H).

Example 11: Preparation of N- (1- (2- (2, 2-difluoroethoxy) -5-fluorophenyl) ethyl) -3- (1-(tetrahydro-2H-pyran-4-yl) -1H-pyrazol-4-yl) pyrazolo [1,5-a] pyrimidin-5-amine (compound 11)

The preparation is as in example 3, except that 1- (difluoromethyl) -4- (4,4,5, 5-tetramethyl-1, 3, 2-dioxaborolan-2-yl) -1H-pyrazole is replaced by 1- (tetrahydropyran-4-yl) -1H-pyrazole-4-boronic acid pinacol ester.

$^1$H NMR (400 MHz, DMSO-$d_6$) δ 8.47 (d, *J* = 7.6 Hz, 1 H), 8.04 (s, 1H), 7.96 (d, *J* = 7.2 Hz, 1 H), 7.82 (s, 1 H), 7.76 (s, 1 H), 7.20 - 6.99 (m, 3 H), 6.66 - 6.32 (m, 2 H), 5.54 - 5.39 (m, 1 H), 4.57 - 4.26 (m, 3 H), 4.07 - 3.92 (m, 2 H), 3.57 - 3.44 (m, 2 H), 2.03 - 1.88 (m, 4 H), 1.46 (d, *J* = 6.8 Hz, 3 H).

Example 12: preparation of (R) -1- (4- (5- ((1- (2- (2, 2-difluoroethoxy) -5-fluorophenyl) ethyl) amino) pyrazolo [1,5-a] pyrimidin-3-yl) -1H-pyrazol-1-yl) ethanone (compound 12)

To an eggplant-shaped bottle were added N- (1- (5-fluoro-2- (2, 2-difluoroethoxy) phenyl) ethyl) -3-(1H-pyrazol-4-yl) pyrazolo [1,5-a] pyrimidin-5-amine (0.26 mmol), acetyl chloride (0.26 mmol), anhydrous DCM (10 mL) and triethylamine (0.52 mmol). The reaction was carried out at 0 °C for 4 h. Vacuum concentrating to obtain crude product, and purifying by column chromatography to obtain light yellow solid with yield of 70%.

$^1$H NMR (400 MHz, DMSO-$d_6$) δ 8.53 (d, *J* = 7.6 Hz, 1 H), 8.41 (s, 1 H), 8.25 (d, *J* = 8.0 Hz, 2 H), 8.19 (d, *J* = 6.8 Hz, 1 H), 7.18 - 6.93 (m, 3 H), 6.68 - 6.32 (m, 2 H), 5.50 (t, *J* = 7.2 Hz, 1 H), 4.65 - 4.36 (m, 2 H), 2.64 (s, 3 H),1.45 (d, *J* = 6.8 Hz, 3 H).

Example 13: Preparation of N- (1- (2- (2, 2-difluoroethoxy) -5-fluorophenyl) ethyl) -3- (1- (methylsulfonyl) -1H-pyrazol-4-yl) pyrazolo [1,5-a] pyrimidin-5-amine (compound 13)

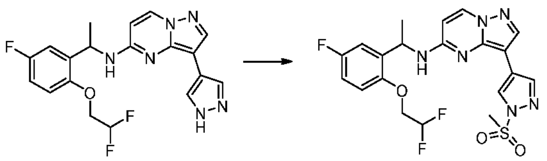

To an eggplant-shaped bottle were added N- (1- (2- (2, 2-difluoroethoxy) -5-fluorophenyl) ethyl) -3-(1H-pyrazol-4-yl) pyrazolo [1,5-a] pyrimidin-5-amine (0.26 mmol), methanesulfonyl chloride (0.26 mmol), anhydrous DCM (10 mL) and triethylamine (0.52 mmol). The reaction was carried out at 0 °C for 4 h. Vacuum concentrating to obtain crude product, and purifying by column chromatography to obtain light yellow solid with yield of 70%.

$^1$H NMR (400 MHz, DMSO-$d_6$) δ 8.52 (d, *J* = 7.6 Hz, 1 H), 8.31 (d, *J* = 11.2 Hz, 2 H), 8.24 (s, 1 H), 8.13 (d, *J* = 7.2 Hz, 1 H), 7.19 - 6.94 (m, 3 H), 6.63 - 6.27 (m, 2 H), 5.56 - 5.42 (m, 1 H), 4.58 - 4.32 (m, 2 H), 3.49 (s, 3 H), 1.46 (d, *J* = 6.8 Hz, 3 H).

Example 14: Preparation of N- (1- (2- (2, 2-difluoroethoxy) -3, 5-difluorophenyl) ethyl) -3- (1H-pyrazol-4-yl) pyrazolo [1,5-a] pyrimidin-5-amine (compound 14)

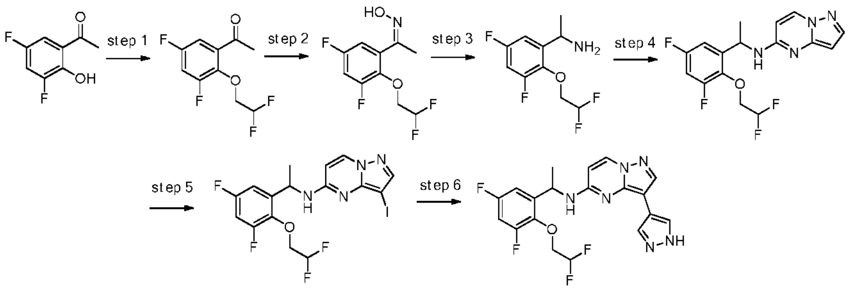


Step 1): 1- (3, 5-difluoro-2-hydroxyphenyl) ethan-1-one (29.0 mmol), 1,1-difluoro-2-iodoethane (43.5 mmol), cesium carbonate (58 mmol) were added to a 200 mL pear-shaped bottle to which DMF (50 mL) was added. Heated overnight in an oil bath (80 °C). The reaction was cooled to ambient temperature and the crude product was purified by column chromatography to give a white solid with 78% yield.

Step 2): 1- (2- (2, 2-difluoroethoxy) -3, 5-difluorophenyl) ethanone (22.3 mmol), hydroxylamine hydrochloride (33.9 mmol), and cesium carbonate (44.6 mmol) were added to a 200 mL pear-shaped bottle, to which methanol (50 mL) was added. The reaction was carried out for 4h at room temperature with magnetic stirring. The reaction solution was poured into water, filtered under suction, and dried to give a white solid with a yield of 98%.

And step 3): 1- (2- (2, 2-difluoroethoxy) -3, 5-difluorophenyl) ethan-1-one oxime (21.4 mmol), zinc powder (321 mmol), ammonium chloride (321 mmol) and acetic acid (321 mmol) were charged to a 200 mL pear bottle, to which methanol (50 mL) was added. Heated overnight in an oil bath (80 °C). The reaction was cooled to ambient temperature, neutralized, filtered through celite, and the filtrate was extracted with water and ethyl acetate. The organic phase was washed twice with saturated brine, dried over anhydrous sodium sulfate and concentrated to give a yellow liquid in 77% yield.

Step 4): 5-Chloropyrazolo [1,5-a] pyrimidine (16.5 mmol), 1- (2- (2, 2-difluoroethoxy) -3, 5-difluorophenyl) ethan-1-amine (16.5 mmol), anhydrous n-butanol (50 mL) and N, N-diisopropylethylamine (DIPEA, 49.5 mmol) were added to a 200 mL pear. The mixture was heated overnight in an oil bath (140 °C). The reaction was cooled to ambient temperature and concentrated under reduced pressure to remove n-butanol and N, N-Diisopropylethylamine (DIPEA) as much as possible to give a crude yellow oil which was purified by column chromatography to give a pale yellow solid with a yield of 68%.

Step 5): N- (1- (2- (2, 2-difluoroethoxy) -3, 5-difluorophenyl) ethyl) pyrazolo [1,5-a] pyrimidin-5-amine (11.2 mmol) was added to a 200 mL pear-shaped bottle, to which acetonitrile (50 mL) was added. N-iodosuccinimide (NIS, 13.4 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 6): N- (1- (2- (2, 2-difluoroethoxy) -3, 5-difluorophenyl) ethyl) -3-iodopyrazolo [1,5-a] pyrimidin-5-amine (0.50 mmol), 1-Boc-pyrazole-4-boronic acid pinacol ester (0.75 mmol), anhydrous potassium carbonate (2.00 mmol), tetrakis (triphenylphosphine) palladium (0.05 mmol) were added to a 100 mL reaction tube, replaced with argon 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 50%.

$^1$H NMR (400 MHz, DMSO-$d_6$) $\delta$ 12.71 (s, 1 H), 8.49 (d, $J$ = 7.6 Hz, 1 H), 8.07 (s, 1 H), 7.99 (d, $J$ = 6.8 Hz, 1 H), 7.87 (s, 2 H), 7.26 - 7.18 (m, 1 H), 7.07 - 7.01 (m, 1 H), 6.63 - 6.25 (m, 2 H), 5.56 - 5.50 (m, 1 H), 4.62 - 4.30 (m, 2 H), 1.49 (d, $J$ = 6.8 Hz, 3 H).

Example 15: Preparation of N- (1- (2- (2, 2-difluoroethoxy) -3, 5-difluorophenyl) ethyl) -3-(1-methyl-1H-pyrazol-4-yl) pyrazolo [1,5-a] pyrimidin-5-amine (compound 15)

The preparation method was the same as in example 14, except that 1-Boc-pyrazole-4-boronic acid pinacol ester was replaced with 1-methylpyrazole-4-boronic acid pinacol ester.

$^1$H NMR (400 MHz, DMSO-$d_6$) $\delta$ 8.47 (d, $J$ = 7.6 Hz, 1 H), 8.02 (d, $J$ = 16.4 Hz, 2 H), 7.82 (s, 1 H), 7.68 (s, 1 H), 7.24 (d, $J$ = 9.6 Hz, 1 H), 7.02 (d, $J$ = 9.2 Hz, 1 H), 6.62 - 6.26 (m, 2 H), 5.59 - 5.39 (m, 1 H), 4.55 - 4.31 (m, 2 H), 3.84 (s, 3 H), 1.49 (d, $J$ = 6.8 Hz, 3 H).

Example 16: Preparation of N- (1- (2- (2, 2-difluoroethoxy) phenyl) ethyl) -3- (1H-pyrazol-4-yl) pyrazolo [1,5-a] pyrimidin-5-amine (compound 16)

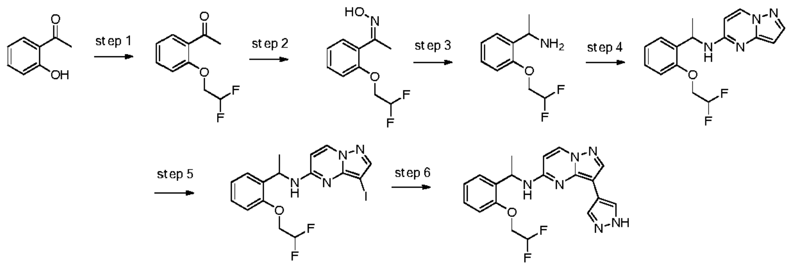


Step 1): the procedure of step 1 in example 14 was used except that 1- (3, 5-difluoro-2-hydroxyphenyl) ethan-1-one was replaced with 1- (2-hydroxyphenyl) ethan-1-one.
Step 2): the procedure of step 2 in example 14 was used.
Step 3): the procedure of step 3 in example 14 was used.
Step 4): the method of step 4 in example 14 was used.
Step 5): the procedure of step 5 in example 14 was used.
Step 6): the synthesis method is the same as step 6 in example 14.
$^1$H NMR (400 MHz, DMSO-$d_6$) δ 12.68 (s, 1 H), 8.43 (d, *J* = 7.6 Hz, 1 H), 8.02 (s, 1 H), 7.99 (d, *J* = 7.2 Hz, 1 H), 7.83 (s, 2 H), 7.31 (dd, *J* = 7.6, 1.6 Hz, 1 H), 7.23 - 7.12 (m, 1 H), 7.06 (d, *J* = 8.0 Hz, 1 H), 6.92 (t, *J* = 7.6 Hz, 1 H), 6.67 - 6.28 (m, 2 H), 5.58 - 5.51 (m, 1 H), 4.50 - 4.40 (m, 2 H), 1.43 (d, *J* = 6.4 Hz, 3 H).
Example 17: Preparation of N- (1- (2- (2, 2-difluoroethoxy) phenyl) ethyl) -3- (1-methyl-1H-pyrazol-4-yl) pyrazolo [1,5-a] pyrimidin-5-amine (compound 17)
The preparation method was the same as in example 16, except that 1-Boc-pyrazole-4-boronic acid pinacol ester was replaced with 1-methylpyrazole-4-boronic acid pinacol ester.
$^1$H NMR (400 MHz, DMSO-$d_6$) δ 8.43 (d, *J* = 7.2 Hz, 1 H), 7.98 (m, 2 H), 7.72 (d, *J* = 7.2 Hz, 2 H), 7.31 (m, 1 H), 7.19 (m, 1 H), 7.11 (m, 1 H), 6.93 (m, 1 H), 6.71 - 6.23 (m, 2 H), 5.59 - 5.47 (m, 1 H), 4.55 - 4.38 (m, 2 H), 3.81 (s, 3 H), 1.44 (d, *J* = 7.2 Hz, 3 H).

Example 18: Preparation of 4-fluoro-2- (1- ((3- (1-methyl-1H-pyrazol-4-yl) pyrazolo [1,5-a] pyrimidin-5-yl) amino) ethyl) phenol (compound a1-2)

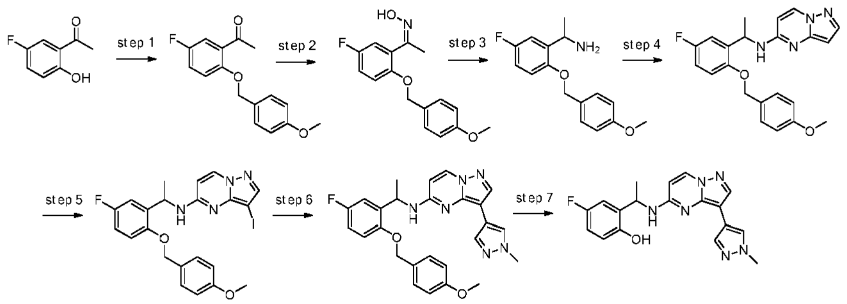


Step 1): 1- (5-fluoro-2-hydroxyphenyl) ethanone (32.4 mmol), 4-methoxybenzyl bromide (38.9 mmol), cesium carbonate (48.6 mmol) were added to a 200 mL pear-shaped vial, to which was added acetonitrile (50 mL). Heated overnight in an oil bath (80 °C). The reaction was cooled to ambient temperature and the crude product was purified by column chromatography to give a white solid with 78% yield.
Step 2): 1- (5-fluoro-2- ((4-methoxybenzyl) oxy) phenyl) ethanone (25.3 mmol), hydroxylamine hydrochloride (30.4 mmol), cesium carbonate (38.0 mmol) were added to a 200 mL pear-shaped bottle, to which methanol (50 mL) was added. The reaction was carried out for 4h at room temperature with magnetic stirring. The reaction solution was poured into water, filtered under suction, and dried to give a white solid with a yield of 98%.
Step 3): 1- (5-fluoro-2- ((4-methoxybenzyl) oxy) phenyl) ethanone oxime (24.8 mmol), zinc powder (372 mmol),

Step 5): N- (1- (5-fluoro-2- ((4-methoxybenzyl) oxy) phenyl) ethyl) pyrazolo [1,5-a] pyrimidin-5-amine (13.0 mmol) was added to a 200 mL pear-shaped vial, to which was added acetonitrile (50 mL). N-iodosuccinimide (NIS, 14.3 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 6): N- (1- (5-fluoro-2- ((4-methoxybenzyl) oxy) phenyl) ethyl) -3-iodopyrazolo [1,5-a] pyrimidin-5-amine (0.77 mmol), 1-methylpyrazole-4-boronic acid pinacol ester (1.16 mmol), anhydrous potassium carbonate (2.00 mmol), tetrakis (triphenylphosphine) palladium (0.08 mmol) were added to a 100 mL reaction tube, replaced with argon for 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 52%.

Step 7): N- (1- (5-fluoro-2- ((4-methoxybenzyl) oxy) phenyl) ethyl) -3- (1-methyl-1H-pyrazol-3-yl) pyrazolo [1,5-a] pyrimidin-5-amine (0.22 mmol) was added to a 200 mL pear-shaped bottle, dichloromethane (10 mL) was added thereto, and trifluoroacetic acid (3.3 mmol) was added dropwise. The reaction was carried out for 4h at room temperature with magnetic stirring. Neutralized and extracted with ethyl acetate. The organic phase was washed twice with saturated brine, dried over anhydrous sodium sulfate, and purified by column chromatography to give a white solid with a yield of 86%.

$^1$H NMR (400 MHz, DMSO-$d_6$) δ 9.68 - 9.65 (m, 1 H), 8.44 (d, $J$ = 7.6 Hz, 1 H), 8.02 (s, 1 H), 7.96 (d, $J$ = 6.8 Hz, 1 H), 7.84 (s, 1 H), 7.75 (s, 1 H), 7.04 - 6.77 (m, 3 H), 6.33 (d, $J$ = 7.6 Hz, 1 H), 5.44 (d, $J$ = 7.2 Hz, 1 H), 3.84 (s, 3 H), 1.43 (d, $J$ = 6.8 Hz, 3 H).

Example 19: Preparation of 4-fluoro-2- (1- ((3- (1- (2-hydroxycyclopentyl) -1H-pyrazol-4-yl) pyrazolo [1,5-a] pyrimidin-5-yl) amino) ethyl) phenol (compound a 2-3)

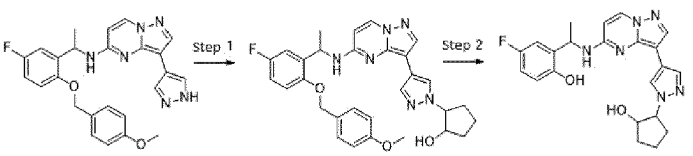


Step 1): to a jar of eggplant shape were added N- (1- (5-fluoro-2- ((4-methoxybenzyl) oxy) phenyl) ethyl) -3- (1H-pyrazol-4-yl) pyrazolo [1,5-a] pyrimidin-5-amine (0.22 mmol), 1, 2-epoxycyclopentane (0.22 mmol), anhydrous DMF (10 mL) and cesium carbonate (0.6 mmol). Heated to reflux overnight in an oil bath (100 °C). The reaction was cooled to ambient temperature, concentrated under reduced pressure to remove DMF as much as possible to give a yellow oily crude product, which was purified by column chromatography to give a pale yellow solid with a yield of 60%.

Step 2): the procedure of step 7 in example 18 was used.

$^1$H NMR (400 MHz, DMSO-$d_6$) δ 9.60 (d, $J$ = 16.4 Hz, 1 H), 8.43 (d, $J$ = 7.6 Hz, 1 H), 8.03 (d, $J$ = 2.0 Hz, 1 H), 7.96 - 7.86 (m, 2 H), 7.82 (s, 1 H), 7.04 - 6.94 (m, 1 H), 6.88 - 6.77 (m, 2 H), 6.33 (d, $J$ = 7.6 Hz, 1 H), 5.54 - 5..44 (m, 1 H), 5.09 - 5..02 (m, 1 H), 4.42 - 4.14 (m, 2 H), 2.21 - 2.09 (m, 2 H), 2.02 - 1.87 (m, 2 H), 1.83 - 1.73 (m, 2 H), 1.63 - 1.54 (m, 2 H), 1.43 (d, $J$ = 6.8 Hz, 3 H).

Example 20: Preparation of 4-fluoro-2- (1- ((3- (1- (2-hydroxycyclohexyl) -1H-pyrazol-4-yl) pyrazolo [1,5-a] pyrimidin-5-yl) amino) ethyl) phenol (compound a 3-4)

The preparation was carried out as in example 19, except that 1, 2-epoxycyclopentane was replaced by 1,2-epoxycyclohexane. yellow oily crude product, which was purified by column chromatography to give a pale yellow solid with a yield of 60%.

Step 2): the procedure of step 7 in example 18 was used.

$^1$H NMR (400 MHz, DMSO-$d_6$) δ 9.60 (d, $J$ = 16.4 Hz, 1 H), 8.43 (d, $J$ = 7.6 Hz, 1 H), 8.03 (d, $J$ = 2.0 Hz, 1 H), 7.96 - 7.86 (m, 2 H), 7.82 (s, 1 H), 7.04 - 6.94 (m, 1 H), 6.88 - 6.77 (m, 2 H), 6.33 (d, $J$ = 7.6 Hz, 1 H), 5.54 - 5..44 (m, 1 H), 5.09 - 5..02 (m, 1 H), 4.42 - 4.14 (m, 2 H), 2.21 - 2.09 (m, 2 H), 2.02 - 1.87 (m, 2 H), 1.83 - 1.73 (m, 2 H), 1.63 - 1.54 (m, 2 H), 1.43 (d, $J$ = 6.8 Hz, 3 H).

Example 20: Preparation of 4-fluoro-2- (1- ((3- (1- (2-hydroxycyclohexyl) -1H-pyrazol-4-yl) pyrazolo [1,5-a] pyrimidin-5-yl) amino) ethyl) phenol (compound a 3-4)

The preparation was carried out as in example 19, except that 1, 2-epoxycyclopentane was replaced by 1,2-epoxycyclohexane.

$^1H$ NMR (400 MHz, DMSO-$d_6$) $\delta$ 9.59 (d, *J* = 18.0 Hz, 1 H), 8.43 (d, *J* = 7.6 Hz, 1 H), 8.01 (d, *J* = 2.8 Hz, 1 H), 7.96 - 7.85 (m, 2 H), 7.80 (s, 1H), 7.06 - 6.95 (m, 1 H), 6.82 (dt, *J* = 6.4, 1.6 Hz, 2 H), 6.42 - 6.22 (m, 1 H), 5.54 - 5.41 (m, 1 H), 4.84 - 4.64 (m, 1 H), 3.92 - 3.64 (m, 2 H), 2.03 - 1.65 (m, 6 H), 1.52 - 1.27 (m, 7 H).

Example 21: Preparation of (R)-N- (1- (2- (difluoromethoxy) -5-fluorophenyl) ethyl) -3- (1-methyl-1H-pyrazol-3-yl) pyrazolo [1,5-a] pyrimidin-5-amine (compound a 4-8)

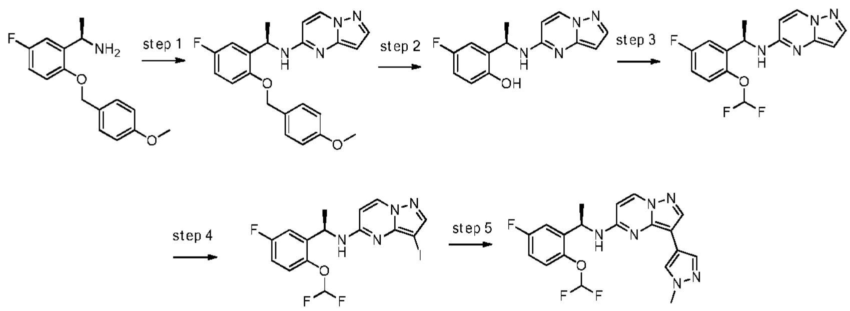


Step 1): 5-Chloropyrazolo [1,5-a] pyrimidine (19.1 mmol), (R)-1- (5-fluoro-2- ((4-methoxybenzyl) oxy) phenyl) ethylamine (19.1 mmol), anhydrous n-butanol (50 mL) and N,N-diisopropylethylamine (DIPEA, 38.2 mmol) were added to a 200 mL pear-shaped bottle. The mixture was heated overnight in an oil bath (140 °C). The reaction was cooled to ambient temperature and concentrated under reduced pressure to remove n-butanol and N, N-Diisopropylethylamine (DIPEA) as much as possible to give a crude yellow oil which was purified by column chromatography to give a pale yellow solid with a yield of 68%.

Step 2): (R) -N- (1- (5-fluoro-2- ((4-methoxybenzyl) oxy) phenyl) ethyl) pyrazolo [1,5-a] pyrimidin-5-amine (13.0 mmol) was added to a 200 mL pear-shaped bottle, methylene chloride (50 mL) was added thereto, and trifluoroacetic acid (195 mmol) was added dropwise. The reaction was carried out for 4h at room temperature with magnetic stirring. Neutralized and extracted with ethyl acetate. The organic phase was washed twice with saturated brine, dried over anhydrous sodium sulfate, and purified by column chromatography to give a white solid with a yield of 85%.

Step 3): (R) -4-fluoro-2- (1- (pyrazolo [1,5-a] pyrimidin-5-ylamino) ethyl) phenol (11.0 mmol), difluoromethane (16.5 mmol), cesium carbonate (22 mmol) were added to a 200 mL pear-shaped bottle, to which DMF (50 mL) was added. Heated overnight in an oil bath (100 °C). The reaction was cooled to ambient temperature and the crude product was purified by column chromatography to give a white solid with a yield of 91%.

Step 4): (R) -N- (1- (2-(difluoromethoxy) -5-fluorophenyl) ethyl) pyrazolo [1,5-a] pyrimidin-5-amine (9.8 mmol) was added to a 200 mL pear-shaped bottle, to which was added acetonitrile (50 mL). N-iodosuccinimide (NIS, 14.85 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 5): (R) -N- (1- (2- (difluoromethoxy) -5-fluorophenyl) ethyl) -3-iodopyrazolo [1,5-a] pyrimidin-5-amine (0.50 mmol), 1-methylpyrazole-4-boronic acid pinacol ester (0.75 mmol), anhydrous potassium carbonate (2.00 mmol), tetrakis (triphenylphosphine) palladium (0.05 mmol) were added to a 100 mL reaction tube, replaced with argon for 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 52%.

$^1H$ NMR (600 MHz, DMSO-$d_6$) $\delta$ 8.48 (s, 1 H), 8.07 (d, *J* = 6.0 Hz, 2 H), 7.80 (s, 1 H), 7.71 (s, 2 H), 7.39 - 7.07 (m, 3 H), 6.34 (s, 1 H), 5.52 - 5.39 (m, 1 H), 3.83 (s, 3 H), 1.47 d, *J* = 6.6 Hz, 3 H).

Example 22: Preparation of 2- (4- (5- ((1- (2- (difluoromethoxy) -5-fluorophenyl) ethyl) amino) pyrazolo [1,5-a] pyrimidin-3-yl) -1H-pyrazol-1-yl) ethan-1-ol (compound a 5-9)

Step 1): 4-fluoro-2- (1- (pyrazolo [1,5-a] pyrimidin-5-ylamino) ethyl) phenol (11.0 mmol), difluoromethane (16.5 mmol), cesium carbonate (22 mmol) were added to a 200 mL pear-shaped bottle, to which DMF (50 mL) was added. Heated overnight in an oil bath (100 °C). The reaction was cooled to ambient temperature and the crude product was purified by column chromatography to give a white solid with a yield of 91%.

Step 2): N- (1- (-5-fluoro-2-difluoromethoxyphenyl) ethyl) pyrazolo [1,5-a] pyrimidin-5-amine (9.8 mmol) was added to a 200 mL pear-shaped bottle, to which was added acetonitrile (50 mL). N-iodosuccinimide (NIS, 14.85 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 65%.

Step 3): N- (1- (5-fluoro-2-difluoromethoxyphenyl) ethyl) -3-iodopyrazolo [1,5-a] pyrimidin-5-amine (0.50 mmol), 4- (4,4,5, 5-tetramethyl-1, 3, 2-dioxaborolan-2-yl) -1H-pyrazole-1-ethanol (0.75 mmol), anhydrous potassium carbonate (2.00 mmol), tetrakis (triphenylphosphine) palladium (0.05 mmol) were added to a 100 mL reaction tube, replaced with argon 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 52%.

$^1$H NMR (400 MHz, DMSO-d6) δ 8.50 (d, *J* = 7.6 Hz, 1 H), 8.07 (s, 1 H), 8.06 (s, 1 H), 7.86 (s, 1 H), 7.76 (s, 1 H), 7.55 - 7.10 (m, 4 H), 6.37 (d, *J* = 7.6 Hz, 1 H), 5.49 (t, *J* = 7.2 Hz, 1 H), 4.95 - 4.88 (m, 1 H), 4.15 (t, *J* = 6.0 Hz, 2 H), 3.77 (q, *J* = 5.6 Hz, 2 H), 1.50 (d, *J* = 7.2 Hz, 3 H).

Example 23: Preparation of N- (1- (2- (difluoromethoxy) -5-fluorophenyl) ethyl) -3- (1-(tetrahydro-2H-pyran-4-yl) -1H-pyrazol-4-yl) pyrazolo [1,5-a] pyrimidin-5-amine (compound a 6-10)

A method similar to example 22 was used, except that 4-(4,4,5, 5-tetramethyl-1, 3, 2-dioxaborolan-2-yl) -1H-pyrazole-1-ethanol was replaced with 1- (tetrahydropyran-4-yl) -1H-pyrazole-4-boronic acid pinacol ester.

$^1$H NMR (400 MHz, DMSO-$d_6$) δ 8.50 (d, *J* = 7.6 Hz, 1 H), 8.08 (s, 1 H), 8.06 (s, 1 H), 7.90 (s, 1 H), 7.78 (s, 1 H), 7.54 - 7.11 (m, 4 H), 6.38 (d, *J* = 7.6 Hz, 1 H), 5.49 (t, *J* = 7.0 Hz, 1 H), 4.36 (dt, *J* = 10.0, 4.4 Hz, 1 H), 4.03 (dt, *J* = 11.2, 3.2 Hz, 2 H), 3.50 (dp, *J* = 11.2, 4.4 Hz, 2 H), 1.98 (td, *J* = 8.8, 7.2, 3.6 Hz, 4 H), 1.50 (d, *J* = 6.8 Hz, 3 H).

Example 24: Preparation of N- (1- (2-ethoxy-5-fluorophenyl) ethyl) -3- (1- (tetrahydro-2H-pyran-4-yl) -1H-pyrazol-4-yl) pyrazolo [1,5-a] pyrimidin-5-amine (compound a 7-12)

Step 1): 4-fluoro-2- (1- (pyrazolo [1,5-a] pyrimidin-5-ylamino) ethyl) phenol (11.0 mmol), iodoethane (16.5 mmol), cesium carbonate (22 mmol) were added to a 200 mL pear-shaped bottle, to which DMF (50 mL) was added. Heated overnight in an oil bath (100 °C). The reaction was cooled to ambient temperature and the crude product was purified by column chromatography to give a white solid with 88% yield.

Step 2): N- (1- (5-fluoro-2-ethoxyphenyl) ethyl) pyrazolo [1,5-a] pyrimidin-5-amine (9.8 mmol) was added to a 200 mL pear-shaped bottle, to which acetonitrile (50 mL) was added. N-iodosuccinimide (NIS, 14.85 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 68%.

Step 3): N- (1- (5-fluoro-2-ethoxyphenyl) ethyl) -3-iodopyrazolo [1,5-a] pyrimidin-5-amine (0.50 mmol), 1-(tetrahydropyran-4-yl) -1H-pyrazole-4-boronic acid pinacol ester (0.75 mmol), anhydrous potassium carbonate (2.00 mmol), tetrakis (triphenylphosphine) palladium (0.05 mmol) were added to a 100 mL reaction tube, replaced with argon for 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 52%.

$^1$H NMR (400 MHz, DMSO-*d6*) δ 8.48 (d, *J* = 7.2 Hz, 1 H), 8.06 (s, 1 H), 7.98 (d, *J* = 7.0 Hz, 1 H), 7.87 (s, 1

H), 7.82 (s, 1 H), 7.15 - 6.92 (m, 3 H), 6.38 (d, $J$ = 7.2 Hz, 1 H), 5.61 - 5.44 (m, 1 H), 4.44 - 3.92 (m, 5 H), 3.59 - 3.43 (m, 2 H), 1.96 (s, 4 H), 1.45 (d, $J$ = 7.2 Hz, 6 H).

Example 25: Preparation of 2- (2- (1- ((3- (1- (difluoromethyl) -1H-pyrazol-4-yl) pyrazolo [1,5-a] pyrimidin-5-yl) amino) ethyl) -4-fluorophenoxy) ethanol (compound a 8-13)

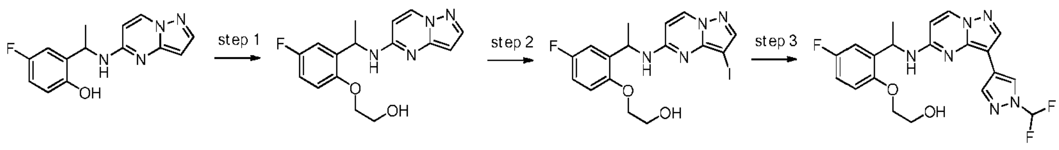


A method similar to example 24 was used, except that iodoethane was replaced with iodoethanol, and that 1-(tetrahydropyran-4-yl) -1H-pyrazole-4-boronic acid pinacol ester was replaced with 1- (difluoromethyl) -4-(4,4,5,5-tetramethyl-1, 3, 2-dioxaborolan-2-yl) -1H-pyrazole.

$^1$H NMR (400 MHz, DMSO-$d_6$) $\delta$ 8.52 (d, $J$ = 7.6 Hz, 1 H), 8.29 (s, 1 H), 8.19 (s, 1 H), 8.16 (s, 1 H), 8.09 (d, $J$ = 7.2 Hz, 1 H), 7.82 (t, $J$ = 59.6 Hz, 1 H), 7.12 - 6.91 (m, 3 H), 6.41 (d, $J$ = 7.6 Hz, 1 H), 5.59 - 5.46 (m, 1 H), 5.00 (t, $J$ = 5.6 Hz, 1 H), 4.23 - 4.03 (m, 2 H), 3.90 - 3.80 (m, 2 H), 1.48 (d, $J$ = 6.8 Hz, 3 H).

Example 26: Preparation of N- (1- (5-fluoro-2- (2-methoxyethoxy) phenyl) ethyl) -3- (1-(tetrahydro-2H-pyran-4-yl) -1H-pyrazol-4-yl) pyrazolo [1,5-a] pyrimidin-5-amine (compound a 9-14)

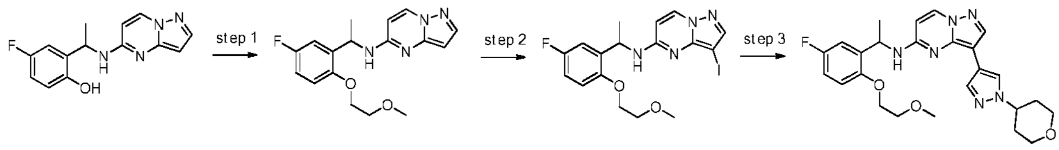


A method similar to that of example 24 was used, except that iodoethane was replaced with 2-iodoethyl methyl ether.

$^1$H NMR (400 MHz, DMSO-$d_6$) $\delta$ 8.45 (d, $J$ = 7.6 Hz, 1 H), 8.03 (s, 1 H), 7.90 (d, $J$ = 7.2 Hz, 1 H), 7.82 (s, 1 H), 7.77 (s, 1 H), 7.08 - 6.94 (m, 3 H), 6.35 (d, $J$ = 7.6 Hz, 1 H), 5.54 - 5.43 (m, 1 H), 4.39 - 4.13 (m, 3 H), 4.03 - 3.94 (m, 2 H), 3.81 - 3.69 (m, 2 H), 3.53 - 3.44 (m, 2 H), 3.33 (s, 3 H), 1.99 - 1.83 (m, 4 H), 1.45 (d, $J$ = 6.8 Hz, 3 H).

Example 27: Preparation of (R) -N- (1- (5-fluoro-2- (2-fluoroethoxy) phenyl) ethyl) -3-(1-methyl-1H-pyrazol-4-yl) pyrazolo [1,5-a] pyrimidin-5-amine (compound a 10-17)

A method similar to that of example 21 was used, except that difluoromethane monoiodo was replaced with 1-fluoro-2-iodoethane.

$^1$H NMR (400 MHz, DMSO-$d_6$) $\delta$ 8.49 (d, $J$ = 7.2 Hz, 1 H), 8.05 (s, 1 H), 8.01 (d, $J$ = 7.2 Hz, 1 H), 7.76 (s, 2 H), 7.17 - 6.95 (m, 3 H), 6.38 (d, $J$ = 7.6 Hz, 1 H), 5.61 - 5.49 (m, 1 H), 4.99 - 4.90 (m, 1 H), 4.87 - 4.78 (m, 1 H), 4.51 - 4.32 (m, 2 H), 3.84 (s, 3 H), 1.47 (d, $J$ = 6.8 Hz, 3 H).

Example 28: Preparation of 3- (1- (difluoromethyl) -1H-pyrazol-4-yl) -N- (1- (5-fluoro-2- (2-fluoroethoxy) phenyl) ethyl) pyrazolo [1,5-a] pyrimidin-5-amine (compound a 11-18)

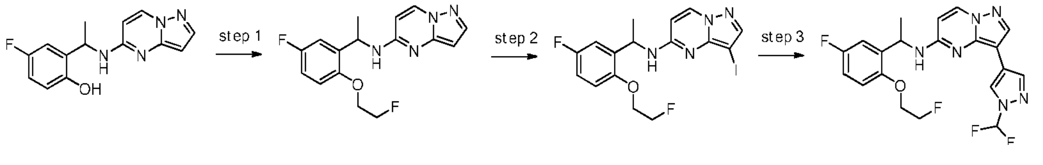

A method similar to example 24 was used, except that iodoethane was replaced with 1-fluoro-2-iodoethane, and that 1- (tetrahydropyran-4-yl) -1H-pyrazole-4-boronic acid pinacol ester was replaced with 1- (difluoromethyl) -4- (4,4,5, 5-tetramethyl-1, 3, 2-dioxaborolan-2-yl) -1H-pyrazole.

$^{1}$H NMR (400 MHz, DMSO-$d_6$) δ 8.53 (d, *J* = 7.2 Hz, 1 H), 8.26 (s, 1 H), 8.19 (s, 1 H), 8.12 (d, *J* = 8.0 Hz, 2 H), 7.80 (t, *J* = 59.2 Hz, 1 H), 7.05 (m, 3 H), 6.43 (d, *J* = 7.6 Hz, 1 H), 5.58 - 5.49 (m, 1 H), 4.96 - 4.85 (m, 1 H), 4.83 - 4.74 (m, 1 H), 4.48 - 4.37 (m, 1 H), 4.38 - 4.30 (m, 1 H), 1.48 (d, *J* = 6.8 Hz, 3 H).

Example 29: Preparation of (R) -2- (4- (5- ((1- (5-fluoro-2- (2-fluoroethoxy) phenyl) ethyl) amino) pyrazolo [1,5-a] pyrimidin-3-yl) -1H-pyrazol-1-yl) acetonitrile (compound a 12-19)

A method similar to that in example 21 was conducted, except that difluoromethane was replaced with 1- fluoro-2-iodoethane and that 1- (tetrahydropyran-4-yl) -1H-pyrazole-4-boronic acid pinacol ester was replaced with 2-[4- (4,4,5, 5-tetramethyl-1, 3, 2-dioxolan-2-yl) pyrazol-1-yl] acetonitrile, respectively.

$^{1}$H NMR (400 MHz, DMSO-$d_6$) δ 8.48 (d, *J* = 7.2 Hz, 1 H), 8.08 (s, 1 H), 8.04 (s, 1 H), 7.91 (d, *J* = 8.0 Hz, 2 H), 7.20 - 6.93 (m, 3 H), 6.37 (d, *J* = 7.2 Hz, 1 H), 5.59 - 5.50 (m, 1 H), 5.47 - 5.40 (m, 2 H), 4.96 -4.87 (m, 1 H), 4.84 - 4.74 (m, 1 H), 4.48 -4.30 (m, 2 H), 1.45 (d, *J* = 6.8 Hz, 3 H).

Example 30: Preparation of N- (1- (5-fluoro-2- (2-fluoroethoxy) phenyl) ethyl) -3- (1-isopropyl-1H-pyrazol-4-yl) pyrazolo [1,5-a] pyrimidin-5-amine (compound a 13-20)

The preparation is as in example 28, but replacing 1-(difluoromethyl) -4-(4,4,5,5-tetramethyl-1,3,2-dioxaborolan-2-yl) -1H-pyrazole by 1-isopropyl-4- (4,4,5, 5-tetramethyl-1, 3, 2-dioxaborolan-2-yl) -1H-pyrazole.

1H NMR (400 MHz, DMSO-d6) δ 8.45 (d, *J* = 7.6 Hz, 1 H), 8.01 (s, 1 H), 7.91 (d, *J* = 7.2 Hz, 1 H), 7.81 (s, 1 H), 7.73 (s, 1 H), 7.13 - 6.94 (m, 3 H), 6.35 (d, *J* = 7.6 Hz, 1 H), 5.55 - 5.42 (m, 1 H), 4.89 (t, *J* = 4.0 Hz, 1 H), 4.77 (t, *J* = 4.0 Hz, 1 H), 4.49 - 4.28 (m, 3 H), 1.45 (d, *J* = 6.8 Hz, 3 H), 1.40 (d, *J* = 6.8 Hz, 6 H).

Example 31: Preparation of 3- (1-cyclopropyl-1H-pyrazol-4-yl) -N- (1- (5-fluoro-2- (2-fluoroethoxy) phenyl) ethyl) pyrazolo [1,5-a] pyrimidin-5-amine (compound a 14-21)

The procedure is as in example 28, except that 1- (difluoromethyl) -4- (4,4,5, 5-tetramethyl-1, 3, 2-dioxaborolan-2-yl) -1H-pyrazole is replaced by 1-cyclopropylpyrazole-4-boronic acid pinacol ester.

$^{1}$H NMR (400 MHz, DMSO-$d_6$) δ 8.48 (d, *J* = 7.6 Hz, 1 H), 8.04 (s, 1 H), 7.99 (d, *J* = 6.8 Hz, 1 H), 7.81 (s, 1 H), 7.74 (s, 1 H), 7.17 - 6.97 (m, 3 H), 6.38 (d, *J* = 7.6 Hz, 1 H), 5.57 - 5.46 (m, 1 H), 4.97 - 4.89 (m, 1 H), 4.84 - 4.78 (m, 1 H), 4.51 - 4.31 (m, 2 H), 3.71 - 3.60 (m, 1 H), 1.47 (d, *J* = 6.8 Hz, 3 H), 1.06 - 0.95 (m, 4 H).

Example 32: Preparation of 2- (4- (5- ((1- (5-fluoro-2- (2-fluoroethoxy) phenyl) ethyl) amino) pyrazolo [1,5-a] pyrimidin-3-yl) -1H-pyrazol-1-yl) ethanol (compound a 15-22)

The preparation is as in example 28, except that 1- (difluoromethyl) -4- (4,4,5, 5-tetramethyl-1, 3, 2-dioxaborolan-2-yl) -1H-pyrazole is replaced by 4- (4,4,5, 5-tetramethyl-1, 3, 2-dioxaborolan-2-yl) -1H-pyrazole-1-ethanol.

$^{1}$H NMR (400 MHz, DMSO-$d_6$) δ 8.48 (d, *J* = 7.6 Hz, 1 H), 8.05 (s, 1 H), 7.98 (d, *J* = 7.2 Hz, 1 H), 7.80 (s, 1 H), 7.77 (s, 1 H),7.11 - 7.00 (m, 3 H), 6.37 (d, *J* = 7.6 Hz, 1 H), 5.60 - 5.51 (m, 1 H), 4.96 - 4.91 (m, 1 H), 4.84 - 4.79 (m, 1 H), 4.54 - 4.31 (m, 2 H), 4.11 (t, *J* = 5.6 Hz, 2 H), 3.73 (t, *J* = 5.6 Hz, 2 H), 1.47 (d, *J* = 6.8 Hz, 3 H).

Example 33: Preparation of 3- (1- (2, 2-difluoroethyl) -1H-pyrazol-4-yl) -N- (1- (5-fluoro-2- (2-fluoroethoxy) phenyl) ethyl) pyrazolo [1,5-a] pyrimidin-5-amine (compound a 16-23)

The preparation is as in example 28, but replacing 1- (difluoromethyl) -4- (4,4,5, 5-tetramethyl-1, 3, 2-dioxaborolan-2-yl) -1H-pyrazole by 1- (2, 2-difluoroethyl) -4- (4,4,5, 5-tetramethyl-1, 3, 2-dioxaborolan-2-yl) -1H-pyrazole.

$^{1}$H NMR (400 MHz, DMSO-$d_6$) δ 8.49 (d, *J* = 7.6 Hz, 1 H), 8.08 (s, 1 H), 8.01 (d, *J* = 7.2 Hz, 1 H), 7.89 (s, 1 H), 7.87 (s, 1 H), 7.19 - 6.96 (m, 3 H), 6.55 - 6.19 (m, 2 H), 5.60 - 5.50 (m, 1 H), 4.92 (t, *J* = 4.0 Hz, 1 H), 4.80 (t, *J* = 3.6 Hz, 1 H), 4.67 - 4.52 (m, 2 H), 4.42 (d, *J* = 4.8 Hz, 1 H), 4.35 (d, *J* = 4.8 Hz, 1 H), 1.47 (d, *J* = 6.8 Hz, 3 H).

Example 34: preparation of 2- (4- (5- ((1- (5-fluoro-2- (2-fluoroethoxy) phenyl) ethyl) amino) pyrazolo [1,5-a] pyrimidin-3-yl) -1H-pyrazol-1-yl) cyclopentan-1-ol (compound a 17-24)

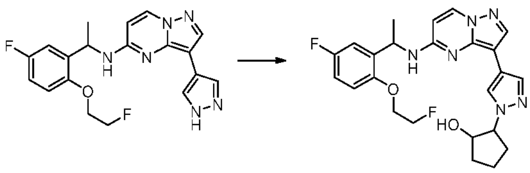

N- (1- (5-fluoro-2- (2-fluoroethoxy) phenyl) ethyl) -3- (1H-pyrazol-4-yl) pyrazolo [1,5-a] pyrimidin-5-amine (0.22 mmol), 1, 2-epoxycyclopentane (0.22 mmol), anhydrous DMF (10 mL), and cesium carbonate (0.6 mmol) were added to an eggplant-shaped bottle. Heated to reflux overnight in an oil bath (100 °C). The reaction was cooled to ambient temperature, concentrated under reduced pressure to remove DMF as much as possible to give a yellow oily crude product, which was purified by column chromatography to give a pale yellow solid with a yield of 65%.

$^1$H NMR (400 MHz, DMSO-$d_6$) δ 8.47 (d, *J* = 7.6 Hz, 1 H), 8.04 (s, 1 H), 7.97 (s, 1 H), 7.83 (d, *J* = 5.6 Hz, 1 H), 7.79 (s, 1 H), 7.15 - 6.97 (m, 3 H), 6.37 (d, *J* = 7.6 Hz, 1 H), 5.58 - 5.49 (m, 1 H), 5.07 (t, *J* = 5.6 Hz, 1 H), 4.97 - 4.89 (m, 1 H), 4.84 - 4.77 (m, 1 H), 4.51 - 4.15 (m, 4 H), 2.21 - 2.10 (m, 1 H), 2.02 - 1.90 (m, 2 H), 1.85 - 1.73 (m, 2 H), 1.64 - 1.54 (m, 1H), 1.47 (d, *J* = 6.8 Hz, 3 H).

Example 35: Preparation of 2- (4- (5- ((1- (5-fluoro-2- (2-fluoroethoxy) phenyl) ethyl) amino) pyrazolo [1,5-a] pyrimidin-3-yl) -1H-pyrazol-1-yl) cyclohexan-1-ol (compound a 18-25)

The preparation was carried out as in example 34, except that 1, 2-epoxycyclopentane was replaced by 1, 2-epoxycyclohexane.

$^1$H NMR (400 MHz, DMSO-$d_6$) δ 8.47 (d, *J* = 7.6 Hz, 1 H), 8.03 (s, 1 H), 7.95 (d, *J* = 7.2 Hz, 1 H), 7.79 (s, 1 H), 7.75 (d, *J* = 2.4 Hz, 1 H), 7.16 - 6.97 (m, 3 H), 6.37 (d, *J* = 7.6 Hz, 1 H), 5.59 - 5.48 (m, 1 H), 4.98 - 4.69 (m, 3 H), 4.51 - 4.31 (m, 2 H), 3.86 - 3.66 (m, 2 H), 2.07 - 1.67 (m, 5 H), 1.47 (d, *J* = 7.2 Hz, 3 H), 1.40 - 1.24 (m, 3 H).

Example 36: Preparation of N- (1- (5-fluoro-2- (2-fluoroethoxy) phenyl) ethyl) -3- (1- (tetrahydro-2H-pyran-4-yl) -1H-pyrazol-4-yl) pyrazolo [1,5-a] pyrimidin-5-amine (compound a 19-26)

The preparation was carried out as in example 28, except that 1- (difluoromethyl) -4- (4,4,5, 5-tetramethyl-1, 3, 2-dioxaborolan-2-yl) -1H-pyrazole was replaced with 1- (tetrahydropyran-4-yl) -1H-pyrazole-4-boronic acid pinacol ester.

$^1$H NMR (400 MHz, DMSO-$d_6$) δ 8.46 (d, *J* = 7.8 Hz, 1 H), 8.04 (s, 1 H), 7.94 (d, *J* = 7.2 Hz, 1 H), 7.83 (s, 1 H), 7.78 (s, 1 H), 7.16 - 6.94 (m, 3 H), 6.38 (d, *J* = 7.6 Hz, 1 H), 5.59 - 5.41 (m, 1 H), 4.91 (dt, *J* = 4.8, 2.4 Hz, 1 H), 4.79 (dt, *J* = 4.4, 2.4 Hz, 1 H), 4.51 - 4.25 (m, 3 H), 4.00 (dq, *J* = 10.8, 3.2 Hz, 2 H), 3.49 (dp, *J* = 10.8, 3.6 Hz, 2 H), 1.99 - 1.81 (m, 4 H), 1.47 (d, *J* = 6.8 Hz, 3 H).

Example 37: Preparation of N- ((R) -1- (5-fluoro-2- (2-fluoroethoxy) phenyl) ethyl) -3- (1-(tetrahydro-2H-pyran-2-yl) -1H-pyrazol-4-yl) pyrazolo [1,5-a] pyrimidin-5-amine (compound a 20-27)

The preparation was carried out as in example 27, except that 1-methylpyrazole-4-boronic acid pinacol ester was replaced by 1- (tetrahydropyran-2-yl) -4- (4,4,5, 5-tetramethyl-1, 3, 2-dioxaborolan-2-yl) -1H-pyrazole.

$^1$H NMR (400 MHz, DMSO-$d_6$) δ 8.49 (d, *J* = 7.6 Hz, 1 H), 8.09 (s, 1 H), 8.00 (d, *J* = 6.8 Hz, 1 H), 7.96 (s, 1 H), 7.85 (s, 1 H), 7.15 - 6.96 (m, 3 H), 6.38 (d, *J* = 7.6 Hz, 1 H), 5.57 - 5.47 (m, 1 H), 5.39 - 5.32 (m, 1 H), 4.96 - 4.91 (m, 1 H), 4.84 - 4.78 (m, 1 H), 4.46 - 4.41 (m, 1H), 4.39 - 4.34 (m, 1 H), 3.99 - 3.91 (m, 1 H), 3.71 - 3.62 (m, 1 H), 2.16 - 1.88 (m, 3 H), 1.78 - 1.68 (m, 1 H), 1.61 - 1.54 (m, 2 H), 1.47 (d, *J* = 6.8 Hz, 3 H).

Example 38: Preparation of 1- (4- (5- ((1- (5-fluoro-2- (2-fluoroethoxy) phenyl) ethyl) amino) pyrazolo [1,5-a] pyrimidin-3-yl) -1H-pyrazol-1-yl) ethanone (compound a 21-28)

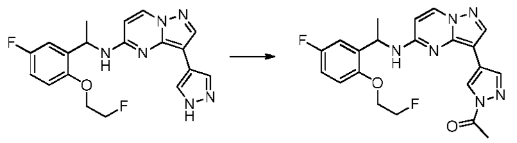

To a jar-shaped bottle were added N- (1- (5-fluoro-2- (2-fluoroethoxy) phenyl) ethyl) -3- (1H-pyrazol-4-yl) pyrazolo [1,5-a] pyrimidin-5-amine (0.26 mmol), acetyl chloride (0.26 mmol), anhydrous DCM (10 mL), and triethylamine (0.52 mmol). The reaction was carried out at 0 °C for 4 h. Vacuum concentrating to obtain crude product, and purifying by column chromatography to obtain light yellow solid with yield of 70%.
$^1$H NMR (400 MHz, DMSO-$d_6$) δ 8.54 (d, *J* = 7.6 Hz, 1 H), 8.47 (s, 1 H), 8.29 (s, 1 H), 8.27 (s, 1 H), 8.16 (d, *J* = 7.2 Hz, 1 H), 7.15 - 6.96 (m, 3 H), 6.43 (d, *J* = 7.6 Hz, 1 H), 5.62 - 5.53 (m, 1 H), 4.99 - 4.90 (m, 1 H), 4.87 - 4.78 (m, 1H), 4.57 - 4.32 (m, 2 H), 2.66 (s, 3 H), 1.47 (d, *J* = 6.8 Hz, 3 H).

Example 39: Preparation of N- (1- (5-fluoro-2- (2-fluoroethoxy) phenyl) ethyl) -3- (1- (methylsulfonyl) -1H-pyrazol-4-yl) pyrazolo [1,5-a] pyrimidin-5-amine (compound a 22-29)

The preparation was carried out as in example 38, except that acetyl chloride was replaced by methanesulfonyl chloride.
$^1$H NMR (400 MHz, DMSO-$d_6$) δ 8.54 (d, *J* = 7.6 Hz, 1 H), 8.37 (s, 1 H), 8.32 (s, 1 H), 8.26 (s, 1 H), 8.15 (d, *J* = 7.2 Hz, 1 H), 7.11 - 6.99 (m, 3 H), 6.44 (d, *J* = 7.6 Hz, 1 H), 5.61 - 5.51 (m, 1 H), 4.97 - 4.89 (m, 1 H), 4.86 - 4.75 (m, 1 H), 4.53 - 4.29 (m, 2 H), 3.51 (s, 3 H), 1.48 (d, *J* = 6.8 Hz, 3 H)..

Example 40: Preparation of (R)-N- (1- (5-fluoro-2- (2, 2, 2-trifluoroethoxy) phenyl) ethyl) -3-(1-methyl-1H-pyrazol-4-yl) pyrazolo [1,5-a] pyrimidin-5-amine (compound a 23-31)

A method similar to that of example 21 was used, except that difluoromethane was replaced with 2-iodo-1,1,1-trifluoroethane.
$^1$H NMR (400 MHz, DMSO-$d_6$) δ 8.47 (d, *J* = 7.6 Hz, 1 H), 8.02 (s, 1 H), 8.00 (d, *J* = 6.8 Hz, 1 H), 7.70 (d, *J* = 2.4 Hz, 2 H), 7.25 - 7.01 (m, 3 H), 6.35 (d, *J* = 7.6 Hz, 1 H), 5.53 - 5.41 (m, 1 H), 4.99 - 4.81 (m, 2 H), 3.80 (s, 3 H), 1.44 (d, *J* = 6.4 Hz, 3 H).

Example 41: Preparation of (R) -2- (4- (5- ((1- (5-fluoro-2- (2, 2, 2-trifluoroethoxy) phenyl) ethyl) amino) pyrazolo [1,5-a] pyrimidin-3-yl) -1H-pyrazol-1-yl) ethanol (compound a 24-32)

A method similar to example 21 was used, except that iodoethane was replaced with 2-iodo-1, 1, 1-trifluoroethane, and 1-methylpyrazole-4-boronic acid pinacol ester was replaced with 4- (4,4,5, 5-tetramethyl-1, 3, 2-dioxaborolan-2-yl) -1H-pyrazole-1-ethanol.
$^1$H NMR (400 MHz, DMSO-$d_6$) δ 8.47 (d, *J* = 7.2Hz, 1 H), 8.03 (s, 1 H), 7.98 (d, *J* = 6.4Hz, 1 H), 7.73 (d, *J* = 11.2Hz, 2 H), 7.27 - 7.00 (m, 3 H), 6.35 (d, *J* = 7.6 Hz, 1 H), 5.51 - 5.41 (m, 1 H), 5.00 - 4.78 (m, 3 H), 4.08 (t, *J* = 5.6Hz, 2 H), 3.71 (q, *J* = 5.6 Hz, 2 H), 1.44 (d, *J* = 6.8Hz, 3 H).

Example 42: Preparation of N- (1- (5-fluoro-2- (3-fluoropropoxy) phenyl) ethyl) -3- (1- (tetrahydro-2H-pyran-4-yl) -1H-pyrazol-4-yl) pyrazolo [1,5-a] pyrimidin-5-amine (compound a 25-35)

A method similar to example 24 was used, except that iodoethane was replaced with 3-fluoro-1-iodopropane.
1H NMR (400 MHz, DMSO-d6) δ 8.46 (d, *J* = 7.6 Hz, 1 H), 8.03 (s, 1 H), 7.93 (d, *J* = 7.2 Hz, 1 H), 7.84 (s, 1 H), 7.79 (s, 1 H), 7.12 - 6.94 (m, 3 H), 6.36 (d, *J* = 7.6 Hz, 1 H), 5.49 (t, *J* = 7.2 Hz, 1 H), 4.73 (td, *J* = 6.0, 2.4 Hz, 1 H), 4.61 (td, *J* = 6.0, 2.4 Hz, 1 H), 4.40 - 4.07 (m, 3 H), 3.99 (dq, *J* = 11.2, 3.2 Hz, 2 H), 3.49 (tt, *J* = 11.2, 3.2 Hz, 2 H), 2.22 (dp, *J* = 24.4, 6.0 Hz, 2 H), 1.92 (td, *J* = 11.2, 10.4, 4.4 Hz, 4 H), 1.45 (d, *J* = 6.8 Hz, 3 H).

Example 43: Preparation of N- (1- (2- (cyclopropylmethoxy) -5-fluorophenyl) ethyl) -3-(1-methyl-1H-pyrazol-4-yl) pyrazolo [1,5-a] pyrimidin-5-amine (compound a 26-38)

A method similar to example 24 was used, except that iodoethane was replaced with iodomethylcyclopropane, and that 1- (tetrahydropyran-4-yl) -1H-pyrazole-4-boronic acid pinacol ester was replaced with 1-methylpyrazole-4-boronic acid pinacol ester.
$^1$H NMR (400 MHz, DMSO-$d_6$) δ 8.48 (d, *J* = 7.6 Hz, 1 H), 8.03 (s, 1 H), 7.99 (d, *J* = 6.8 Hz, 1 H), 7.76 (d, *J* = 2.8 Hz, 2 H), 7.14 - 6.90 (m, 3 H), 6.37 (d, *J* = 7.6 Hz, 1 H), 5.58 - 5.49 (m, 1 H), 4.09 - 3.93 (m, 2 H), 3.84 (s, 3 H), 1.47 (d, *J* = 6.8 Hz, 3 H), 1.39 - 1.29 (m, 1 H), 0.64 - 0.56 (m, 2 H), 0.46 - 0.39 (m, 2 H).

Example 44: Preparation of N- (1- (5-fluoro-2- ((tetrahydrofuran-3-yl) oxy) phenyl) ethyl) -3-(1-methyl-1H-pyrazol-4-yl) pyrazolo [1,5-a] pyrimidin-5-amine (compound a 27-41)

A method similar to example 24 was conducted, except that iodoethane was replaced with 3-iodotetrahydrofuran, and that 1- (tetrahydropyran-4-yl) -1H-pyrazole-4-boronic acid pinacol ester was replaced with 1-methylpyrazole-4-boronic acid pinacol ester.

$^1$H NMR (400 MHz, DMSO-$d_6$) δ 8.47 (d, *J* = 7.6 Hz, 1 H), 8.03 (s, 1 H), 7.94 (d, *J* = 7.0 Hz, 1 H), 7.78 (s, 1 H), 7.75 (s, 1 H), 7.16 - 6.94 (m, 3 H), 6.37 (d, *J* = 7.6 Hz, 1 H), 5.51 - 5.43 (m, 1 H), 5.21 - 5.15 (m, 1 H), 4.10 - 3.76 (m, 7 H), 2.40 - 1.96 (m, 2 H), 1.44 (d, *J* = 6.8 Hz, 3 H).

Example 45: Preparation of N- (1- (5-fluoro-2- ((tetrahydro-2H-pyran-4-yl) methoxy) phenyl) ethyl) -3-(1-methyl-1H-pyrazol-4-yl) pyrazolo [1,5-a] pyrimidin-5-amine (compound a 28-43)

A method similar to example 24 was conducted, except that iodoethane was replaced with 4-iodomethyltetrahydropyran, and that 1- (tetrahydropyran-4-yl) -1H-pyrazole-4-boronic acid pinacol ester was replaced with 1-methylpyrazole-4-boronic acid pinacol ester.

$^1$H NMR (400 MHz, DMSO-$d_6$) δ 8.47 (d, *J* = 7.6 Hz, 1 H), 8.02 (s, 1 H), 7.95 (d, *J* = 6.8 Hz, 1 H), 7.75 (s, 2 H), 7.14 - 6.93 (m, 3 H), 6.36 (d, *J* = 7.6 Hz, 1 H), 5.58 - 5.48 (m, 1 H), 4.07 - 3.98 (m, 1 H), 3.97 - 3.77 (m, 6 H), 3.31 - 3.28 (m, 2 H), 2.18 - 2.06 (m, 1 H), 1.83 - 1.67 (m, 2 H), 1.55 - 1.35 (m, 5 H).

Example 46: Preparation of 2- (1- ((3- (1H-pyrazol-4-yl) pyrazolo [1,5-a] pyrimidin-5-yl) amino) ethyl) -4-fluorophenol (compound b1-1)

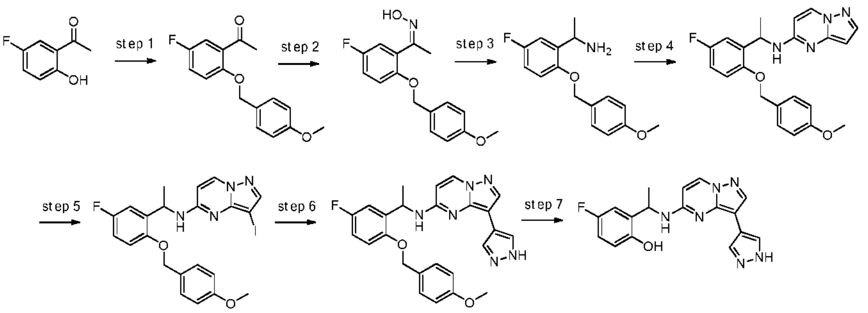


Step 1): 1- (5-fluoro-2-hydroxyphenyl) ethanone (32.4 mmol), 4-methoxybenzyl bromide (38.9 mmol), cesium carbonate (48.6 mmol) were added to a 200 mL pear-shaped vial, to which was added acetonitrile (50 mL). Heated overnight in an oil bath (80 °C). The reaction was cooled to ambient temperature and the crude product was purified by column chromatography to give a white solid with 78% yield.

Step 2): 1- (5-fluoro-2- ((4-methoxybenzyl) oxy) phenyl) ethanone (25.3 mmol), hydroxylamine hydrochloride (30.4 mmol), cesium carbonate (38.0 mmol) were added to a 200 mL pear-shaped bottle, to which methanol (50 mL) was added. The reaction was carried out for 4h at room temperature with magnetic stirring. The reaction solution was poured into water, filtered under suction, and dried to give a white solid with a yield of 98%.

Step 3): 1- (5-fluoro-2- ((4-methoxybenzyl) oxy) phenyl) ethanone oxime (24.8 mmol), zinc powder (372 mmol), ammonium chloride (372 mmol), acetic acid (372 mmol) were added to a 200 mL pear-shaped bottle, to which methanol (50 mL) was added. Heated overnight in an oil bath (80 °C). The reaction was cooled to ambient temperature, neutralized, filtered through celite, and the filtrate was extracted with water and ethyl acetate. The organic phase was washed twice with saturated brine, dried over anhydrous sodium sulfate and concentrated to give a yellow liquid in 77% yield.

Step 4): 5-Chloropyrazolo [1,5-a] pyrimidine (19.1 mmol), 1- (5-fluoro-2- ((4-methoxybenzyl) oxy) phenyl) ethylamine (19.1 mmol), anhydrous n-butanol (50 mL) and N, N-diisopropylethylamine (DIPEA, 38.2 mmol) were added to a 200 mL pear-shaped bottle. The mixture was heated overnight in an oil bath (140 °C). The reaction was cooled to ambient temperature and concentrated under reduced pressure to remove n-butanol and N, N-Diisopropylethylamine (DIPEA) as much as possible to give a crude yellow oil which was purified by column chromatography to give a pale yellow solid with a yield of 68%.

Step 5): N- (1- (5-fluoro-2- ((4-methoxybenzyl) oxy) phenyl) ethyl) pyrazolo [1,5-a] pyrimidin-5-amine (13.0 mmol) was added to a 200 mL pear-shaped vial, to which was added acetonitrile (50 mL). N-iodosuccinimide (NIS, 14.3 mmol) was added under magnetic stirring at room temperature. The reaction was carried out at room temperature for

1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 6): N- (1- (5-fluoro-2- ((4-methoxybenzyl) oxy) phenyl) ethyl) -3-iodopyrazolo [1,5-a] pyrimidin-5-amine (0.77 mmol), 1-Boc-pyrazole-4-boronic acid pinacol ester (1.16 mmol), anhydrous potassium carbonate (2.00 mmol), tetrakis (triphenylphosphine) palladium (0.08 mmol) were added to a 100 mL reaction tube, replaced with argon for 3 times, and 10mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 50%.

Step 7): N- (1- (5-fluoro-2- ((4-methoxybenzyl) oxy) phenyl) ethyl) pyrazolo [1,5-a] pyrimidin-5-amine (0.22 mmol) was added to a 200 mL pear-shaped bottle, to which dichloromethane (10 mL) was added, and trifluoroacetic acid (3.3 mmol) was added dropwise. The reaction was carried out for 4h at room temperature with magnetic stirring. Neutralized and extracted with ethyl acetate. The organic phase was washed twice with saturated brine, dried over anhydrous sodium sulfate, and purified by column chromatography to give a white solid with a yield of 85%.

$^1$H NMR (400 MHz, DMSO-$d_6$) $\delta$ 12.63 (s, 1 H), 9.66 - 9.56 (m, 1 H), 8.44 (d, *J* = 7.6 Hz, 1 H), 8.05 (s, 1 H), 7.97 - 7.82 (m, 3 H), 7.08 - 6.98 (m, 1 H), 6.89 - 6.76 (m, 2 H), 6.32 (d, *J* = 7.6 Hz, 1 H), 5.57 - 5.46 (m, 1 H), 1.43 (d, *J* = 6.8 Hz, 3 H).

Example 47: Preparation of N- (1- (5-fluoro-2-methoxyphenyl) ethyl) -3- (1H-pyrazol-4-yl) pyrazolo [1,5-a] pyrimidin-5-amine (compound b 2-5)

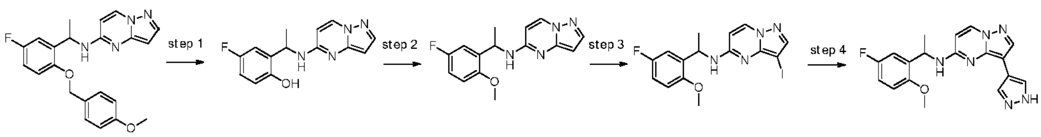


Step 1): N- (1- (5-fluoro-2- ((4-methoxybenzyl) oxy) phenyl) ethyl) pyrazolo [1,5-a] pyrimidin-5-amine (13.0 mmol) was added to a 200 mL pear-shaped bottle, to which dichloromethane (50 mL) was added, and trifluoroacetic acid (195 mmol) was added dropwise. The reaction was carried out for 4h at room temperature with magnetic stirring. Neutralized and extracted with ethyl acetate. The organic phase was washed twice with saturated brine, dried over anhydrous sodium sulfate, and purified by column chromatography to give a white solid with a yield of 85%.

Step 2): 4-fluoro-2- (1- (pyrazolo [1,5-a] pyrimidin-5-ylamino) ethyl) phenol (11.0 mmol), iodomethane (16.5 mmol), and cesium carbonate (22 mmol) were added to a 200 mL pear-shaped bottle, to which DMF (50 mL) was added. Heated overnight in an oil bath (100 °C). The reaction was cooled to ambient temperature and the crude product was purified by column chromatography to give a white solid with a yield of 90%.

Step 3): N- (1- (5-fluoro-2-methoxyphenyl) ethyl) pyrazolo [1,5-a] pyrimidin-5-amine (9.8 mmol) was added to a 200 mL pear-shaped bottle, to which acetonitrile (50 mL) was added. N-iodosuccinimide (NIS, 14.85 mmol) was added under magnetic stirring at room temperature. The reaction was carried out at room temperature for 1h and TLC monitored for completion. After the acetonitrile was removed as much as possible under reduced pressure, it was diluted with 250mL of ethyl acetate and transferred to a separatory funnel. Washing with 1 mol/L NaOH for 3 times, washing with saturated salt for two times, drying with anhydrous sodium sulfate, concentrating to obtain red oily crude product, and purifying the crude product by column chromatography to obtain light yellow solid with yield of 67%.

Step 4): N- (1- (5-fluoro-2-methoxyphenyl) ethyl) -3-iodopyrazolo [1,5-a] pyrimidin-5-amine (0.50 mmol), 1-Boc-pyrazole-4-boronic acid pinacol ester (0.75 mmol), anhydrous potassium carbonate (2.00 mmol), tetrakis (triphenylphosphine) palladium (0.05 mmol) were added to a 100 mL reaction tube, replaced with argon for 3 times, and 10 mL anhydrous DMF, 2 mL water were added. The reaction was carried out at 100 °C for 2h under argon atmosphere and the completion of the reaction was monitored by TLC. Cooled to 50 °C, filtered through celite, and the filtrate was extracted with water and ethyl acetate. The organic phase was washed twice with saturated brine, dried over anhydrous sodium sulfate, concentrated to give a crude product as a black oil, which was purified by column chromatography to give a pale yellow solid with a yield of 50%.

$^1$H NMR (400 MHz, DMSO-d6) $\delta$ 12.70 (s, 1 H), 8.44 (d, *J* = 7.6 Hz, 1 H), 8.14 - 7.91 (m, 2 H), 7.82 (s, 2 H), 7.16 - 6.87 (m, 3 H), 6.32 (d, *J* = 8.0 Hz, 1 H), 5.57 - 5.40 (m, 1 H), 3.95 (s, 3 H), 1.40 (d, *J* = 7.2 Hz, 3 H).

Example 48: Preparation of N- (1- (5-fluoro-2- (fluoromethoxy) phenyl) ethyl) -3- (1H-pyrazol-4-yl) pyrazolo [1,5-a] pyrimidin-5-amine (compound b 3-6)

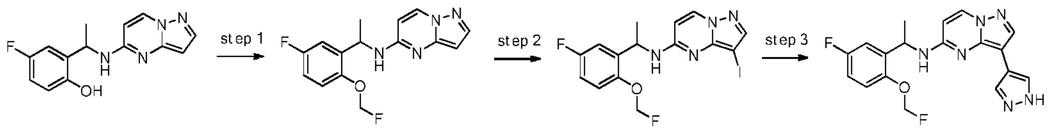


Step 1): the procedure as in step 2 of example 47 was used except that methyl iodide was replaced with fluoromethyl iodide.
Step 2): the procedure of step 3 in example 47 was used.
Step 3): the synthesis procedure used was step 4 of example 47.
$^1H$ NMR (400 MHz, DMSO-$d_6$) $\delta$ 12.68 (s, 1 H), 8.46 (d, *J* = 7.6 Hz, 1 H), 8.04 (d, *J* = 3.2 Hz, 1 H), 8.02 (s, 1 H), 7.86 (s, 1 H), 7.79 (s, 1 H), 7.27 - 7.02 (m, 3 H), 6.33 (d, *J* = 7.6 Hz, 1 H), 6.07 (s, 1 H), 5.93 (s, 1 H), 5.58 - 5.47 (m, 1 H), 1.45 (d, *J* = 6.8 Hz, 3 H).

Example 49: Preparation of N- (1- (2- (difluoromethoxy) -5-fluorophenyl) ethyl) -3- (1H-pyrazol-4-yl) pyrazolo [1,5-a] pyrimidin-5-amine (compound b 4-7)

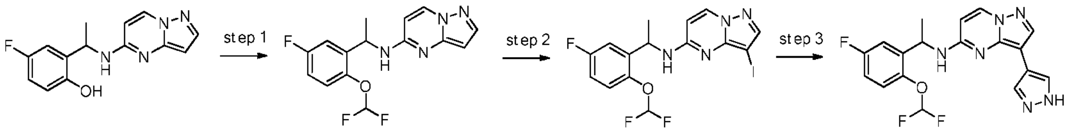


Step 1): the procedure as in step 2 of example 47 was used except that iodomethane was replaced with difluoromethane monoiodomethane.
Step 2): the procedure of step 3 in example 47 was used.
Step 3): the synthesis procedure used was step 4 of example 47.
$^1H$ NMR (400 MHz, DMSO-$d_6$) $\delta$ 12.70 (s, 1 H), 8.50 (d, *J* = 7.6 Hz, 1 H), 8.08 (d, *J* = 1.6 Hz, 1 H), 8.03 (d, *J* = 6.8 Hz, 1 H), 7.85 (s, 2 H), 7.52 - 7.07 (m, 4 H), 6.36 (d, *J* = 7.6 Hz, 1 H), 5.50 (t, *J* = 7.2 Hz, 1 H), 1.50 (d, *J* = 6.8 Hz, 3 H).

Example 50: Preparation of N- (1- (2-ethoxy-5-fluorophenyl) ethyl) -3- (1H-pyrazol-4-yl) pyrazolo [1,5-a] pyrimidin-5-amine (compound b 5-11)

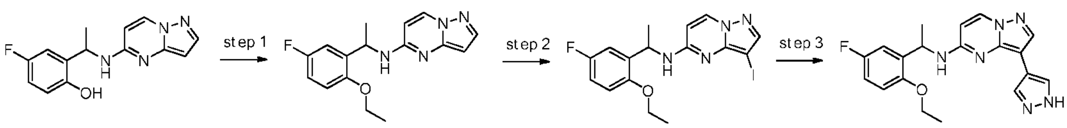


Step 1): the procedure as in step 2 of example 47 was used except that methyl iodide was replaced with ethyl iodide.
Step 2): the procedure of step 3 in example 47 was used.
Step 3): the synthesis procedure used was step 4 of example 47.
$^1H$ NMR (400 MHz, DMSO-$d_6$) $\delta$12.70 (s, 1 H) , 8.47 (d, *J* = 7.2 Hz, 1 H), 8.06 (s, 1 H), 8.00 (d, *J* = 7.2 Hz, 1 H), 7.87 (s, 2 H), 7.09 (dd, *J* = 9.6, 3.0 Hz, 1 H), 7.04 - 6.91 (m, 2 H), 6.35 (d, *J* = 7.2 Hz, 1 H), 5.65 - 5.53 (m, 1 H), 4.27 - 4.10 (m, 2 H), 1.51 - 1.41 (m, 6 H).

Example 51: Preparation of N- (1- (5-fluoro-2- (2-methoxyethoxy) phenyl) ethyl) -3- (1H-pyrazol-4-yl) pyrazolo [1,5-a] pyrimidin-5-amine (compound b 6-15)

Step 1): the procedure as in step 2 of example 47 was used except that methyl iodide was replaced with 2-iodoethyl methyl ether.

Step 2): the procedure of step 3 in example 47 was used.

Step 3): the synthesis procedure used was step 4 of example 47.

1H NMR (400 MHz, DMSO-d6) δ 12.67 (s, 1 H), 8.45 (d, *J* = 7.6 Hz, 1 H), 8.05 (s, 1 H), 7.94 (d, *J* = 7.2 Hz, 1 H), 7.86 (s, 2 H), 7.16 - 6.87 (m, 3 H), 6.34 (d, *J* = 7.6 Hz, 1 H), 5.63 - 5.46 (m, 1 H), 4.33 - 4.12 (m, 2 H), 3.85 - 3.68 (m, 2 H), 3.44 - 3.38 (m, 3 H), 1.44 (d, *J* = 6.8 Hz, 3 H).

Example 52: Preparation of (R) -N- (1- (5-fluoro-2- (2-fluoroethoxy) phenyl) ethyl) -3- (1H-pyrazol-4-yl) pyrazolo [1,5-a] pyrimidin-5-amine (compound b 7-16)

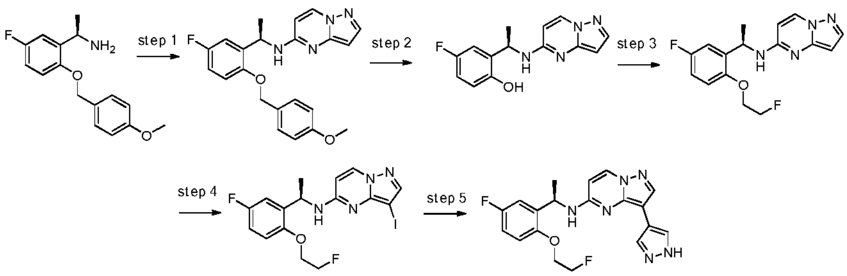


Step 1): 5-Chloropyrazolo [1,5-a] pyrimidine (19.1 mmol), (R) -1- (5-fluoro-2- ((4-methoxybenzyl) oxy) phenyl) ethylamine (19.1 mmol), anhydrous n-butanol (50 mL) and N, N-diisopropylethylamine (DIPEA, 38.2 mmol) were added to a 200 mL pear-shaped bottle. The mixture was heated overnight in an oil bath (140 °C). Cooling the reaction to ambient temperature, concentrating under reduced pressure to remove n-butanol and N, N-Diisopropylethylamine (DIPEA) to obtain yellow oily crude product, purifying by column chromatography to obtain light yellow solid with yield of 68%.

Step 2): (R) -N- (1- (5-fluoro-2- ((4-methoxybenzyl) oxy) phenyl) ethyl) pyrazolo [1,5-a] pyrimidin-5-amine (13.0 mmol) was added to a 200 mL pear-shaped bottle, methylene chloride (50 mL) was added thereto, and trifluoroacetic acid (195 mmol) was added dropwise. The reaction was carried out for 4h at room temperature with magnetic stirring. Neutralized and extracted with ethyl acetate. The organic phase was washed twice with saturated brine, dried over anhydrous sodium sulfate, and purified by column chromatography to give a white solid with a yield of 85%.

Step 3): the procedure as in step 2 of example 47 was used except that methyl iodide was replaced with 1-fluoro-2-iodoethane.

Step 4): the procedure of step 3 in example 47 was used.

Step 5): the synthesis was performed as in step 4 of example 47.

$^1$H NMR (400 MHz, DMSO-$d_6$) δ 12.68 (s, 1 H), 8.48 (d, *J* = 7.2 Hz, 1 H), 8.07 (s, 1 H), 8.02 (d, *J* = 7.2 Hz, 1 H), 7.86 (s, 2 H), 7.16 - 6.95 (m, 3 H), 6.36 (d, *J* = 7.6 Hz, 1 H), 5.64 - 5.54 (m, 1 H), 4.94 (t, *J* = 3.6 Hz, 1 H), 4.82 (t, *J* = 3.6 Hz, 1 H), 4.48 - 4.44 (m, 1 H), 4.41 - 4.36 (m, 1 H), 1.46 (d, *J* = 7.2 Hz, 3 H).

Example 53: Preparation of (R) -N- (1- (5-fluoro-2- (2, 2, 2-trifluoroethoxy) phenyl) ethyl) -3- (1H-pyrazol-4-yl) pyrazolo [1,5-a] pyrimidin-5-amine (compound b 8-30)

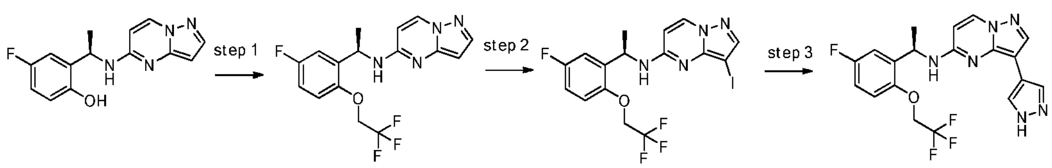


Step 1): the procedure as in step 2 of example 47 was used except that methyl iodide was replaced with 2-iodo-1, 1, 1-trifluoroethane.

Step 2): the procedure of step 3 in example 47 was used.

Step 3): the synthesis was performed as in step 4 of example 47.

$^1$H NMR (400 MHz, DMSO-$d_6$) δ12.67 (s, 1 H), 8.46 (d, *J* = 7.6 Hz, 1 H), 8.04 (s, 1 H), 8.02 (d, *J* = 7.2 Hz, 1 H), 7.80 (s, 2 H), 7.08 (m, 3 H), 6.34 (d, *J* = 7.6 Hz, 1H), 5.52 - 5.44 (m, 1 H), 5.04 - 4.70 (m, 2 H), 1.43 (d, *J* = 6.4 Hz, 3 H).

Example 54: Preparation of N- (1- (5-fluoro-2-propoxyphenyl) ethyl) -3- (1H-pyrazol-4-yl) pyrazolo [1,5-a] pyrimidin-5-amine (compound b 9-33)

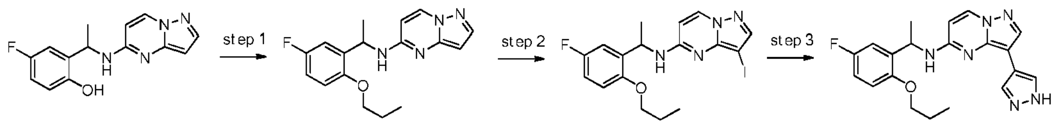


Step 1): the procedure as in step 2 of example 47 was used except that methyl iodide was replaced with iodopropane.

Step 2): the procedure of step 3 in example 47 was used.

Step 3): the synthesis was performed as in step 4 of example 47.

$^1$H NMR (400 MHz, DMSO-$d_6$) δ 12.67 (s, 1 H), 8.45 (d, *J* = 7.6 Hz, 1 H), 8.04 (s, 1 H), 7.98 (d, *J* = 7.2 Hz, 1 H), 7.83 (s, 2 H), 7.10 - 6.90 (m, 3 H), 6.33 (d, *J* = 7.6 Hz, 1 H), 5.61 - 5.48 (m, 1 H), 4.13 (dt, *J* = 9.2, 6.0 Hz, 1 H), 4.03 (dt, *J* = $9_2$, 6.4 Hz, 1 H), 1.84 (q, *J* = 6.8 Hz, 2 H), 1.42 (d, *J* = 6.8 Hz, 3 H), 1.05 (t, *J* = 7.6 Hz, 3 H).

Example 55: Preparation of N- (1- (5-fluoro-2- (3-fluoropropoxy) phenyl) ethyl) -3- (1H-pyrazol-4-yl) pyrazolo [1,5-a] pyrimidin-5-amine (compound b 10-34)

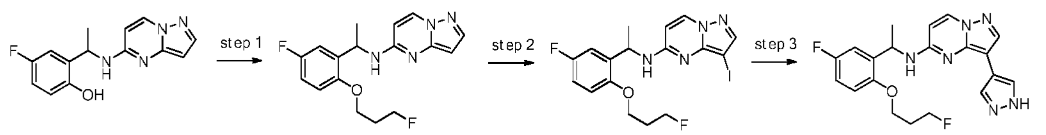


Step 1): the procedure of step 2 in example 47 was used except that methyl iodide was replaced with 3-fluoro-1-iodopropane.

Step 2): the procedure of step 3 in example 47 was used.

Step 3): the synthesis was performed as in step 4 of example 47.

$^1$H NMR (400 MHz, DMSO-$d_6$) δ 12.70 (s, 1 H), 8.46 (d, *J* = 7.6 Hz, 1 H), 8.06 (s, 1 H), 7.98 (d, *J* = 7.2 Hz, 1 H), 7.86 (s, 2 H), 7.15 - 6.90 (m, 3 H), 6.35 (d, *J* = 7.6 Hz, 1 H), 5.60 - 5.46 (m, 1 H), 4.74 (tt, *J* = 5.6, 2.8 Hz, 1 H), 4.62 (td, *J* = 6.0, 2.4 Hz, 1 H), 4.29 - 4.15 (m, 2 H), 2.30 - 2.17 (m, 2 H), 1.44 (d, *J* = 6.8 Hz, 3 H).

Example 56: Preparation of 2- (2- (1- ((3- (1H-pyrazol-4-yl) pyrazolo [1,5-a] pyrimidin-5-yl) amino) ethyl) -4-fluorophenoxy) acetonitrile (compound b 11-36)

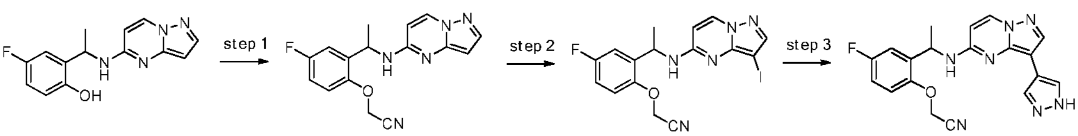


Step 1): the process of step 2 in example 47 was used, except that methyl iodide was replaced with iodoacetonitrile.

Step 2): the procedure of step 3 in example 47 was used.

Step 3): the synthesis procedure used was step 4 of example 47.

$^1$H NMR (400 MHz, DMSO-$d_6$) δ 12.64 (s, 1 H), 8.48 (d, *J* = 7.2 Hz, 1 H), 8.06 (s, 1 H), 7.86 (s, 1 H), 7.50 (s,

1 H), 7.41 (s, 1 H), 7.12 (d, $J$ = 9.0 Hz, 1 H), 7.09 - 6.92 (m, 2 H), 6.36 (d, $J$ = 7.2 Hz, 1 H), 5.66 - 5.49 (m, 1 H), 4.76 - 4.50 (m, 2 H), 1.5 1 (d, $J$ = 6.6 Hz, 3 H).

Example 57: Preparation of N- (1- (2- (cyclopropylmethoxy) -5-fluorophenyl) ethyl) -3- (1H-pyrazol-4-yl) pyrazolo [1,5-a] pyrimidin-5-amine (compound b 12-37)

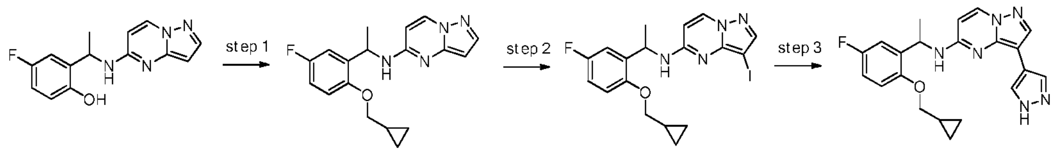


Step 1): the procedure as in step 2 of example 47 was used except that methyl iodide was replaced with (iodomethyl) cyclopropane.
Step 2): the procedure of step 3 in example 47 was used.
Step 3): the synthesis was performed as in step 4 of example 47.
$^1$H NMR (400 MHz, DMSO-$d_6$) δ 12.68 (s, 1 H), 8.47 (d, $J$ = 7.6 Hz, 1 H), 8.06 (s, 1 H), 7.98 (d, $J$ = 7.2 Hz, 1 H), 7.85 (s, 2 H), 7.12 - 6.91 (m, 3 H), 6.36 (d, $J$ = 7.6 Hz, 1 H), 5.66 - 5.50 (m, 1 H), 4.01 (qd, $J$ = 10.0, 6.8 Hz, 2 H), 1.46 (d, $J$ = 6.8 Hz, 3 H), 1.40 - 1.31 (m, 1 H), 0.65 - 0.54 (m, 2 H), 0.47 - 0.41 (m, 2 H).

Example 58: Preparation of N- (1- (2- (cyclopentyloxy) -5-fluorophenyl) ethyl) -3- (1H-pyrazol-4-yl) pyrazolo [1,5-a] pyrimidin-5-amine (compound b 13-39)

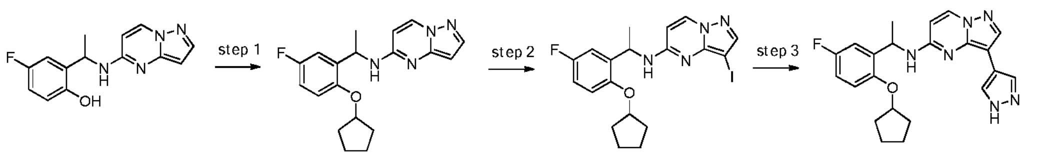


Step 1): the procedure of step 2 in example 47 was used except that methyl iodide was replaced with iodocyclopentane.
Step 2): the procedure of step 3 in example 47 was used.
Step 3): the synthesis was performed as in step 4 of example 47.
$^1$H NMR (600 MHz, DMSO-$d_6$) δ 12.66 (s, 1 H), 8.45 (d, $J$ = 7.2 Hz, 1 H), 8.05 (s, 1 H), 7.91 (s, 1 H), 7.85 (s, 2 H), 7.05 - 6.96 (m, 3 H), 6.37 - 6.28 (m, 1 H), 5.55 - 5.48 (m, 1 H), 4.99 - 4.89 (m, 1 H), 2.05 - 1.55 (m, 8 H), 1.40 (d, $J$ = 6.6 Hz, 3 H).

Example 59: Preparation of N- (1- (5-fluoro-2- ((tetrahydrofuran-3-yl) oxy) phenyl) ethyl) -3- (1H-pyrazol-4-yl) pyrazolo [1,5-a] pyrimidin-5-amine (compound b 14-40)

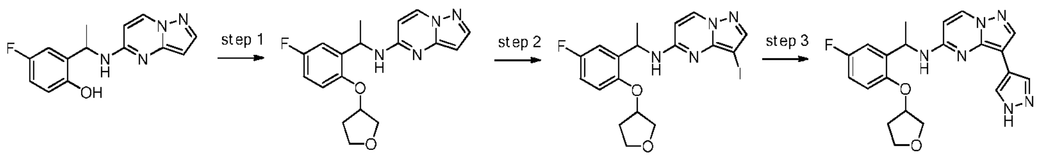


Step 1): the procedure as in step 2 of example 47 was used except that methyl iodide was replaced with 3 -iodotetrahydrofuran.
Step 2): the procedure of step 3 in example 47 was used.
Step 3): the synthesis was performed as in step 4 of example 47.
$^1$H NMR (400 MHz, DMSO-$d_6$) δ12.68 (s, 1 H), 8.47 (d, $J$ = 7.6 Hz, 1 H), 8.06 (s, 1 H), 7.96 (t, $J$ = 6.0 Hz, 1 H), 7.85 (s, 1 H), 7.84 (s, 1 H), 7.17 - 6.95 (m, 3 H), 6.36 (d, $J$ = 7.6 Hz, 1 H), 5.53 - 5.46 (m, 1 H), 5.21 - 5.16 (m, 1 H), 4.14

- 3.76 (m, 4 H), 2.37 -2.00(m, 2 H), 1.43 (d, *J* = 6.8 Hz, 3 H).

Example 60: Preparation of N- (1- (5-fluoro-2- ((tetrahydro-2H-pyran-4-yl) methoxy) phenyl) ethyl) -3-(1H-pyrazol-4-yl) pyrazolo [1,5-a] pyrimidin-5-amine (compound b 15-42)

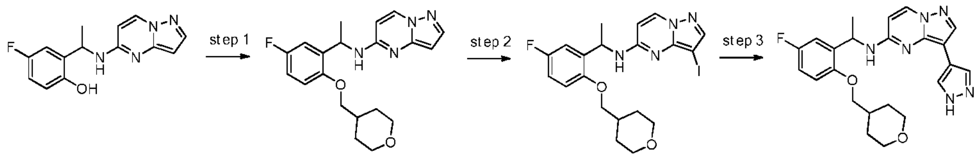


Step 1): the procedure as in step 2 of example 47 was used except that methyl iodide was replaced with 4-iodomethyltetrahydropyran.
Step 2): the procedure of step 3 in example 47 was used.
Step 3): the synthesis was performed as in step 4 of example 47.
$^1$H NMR (400 MHz, DMSO-$d_6$) δ 12.65 (s, 1 H), 8.47 (d, *J* = 7.6 Hz, 1 H), 8.06 (s, 1 H), 7.98 (d, *J* = 6.8 Hz, 1 H), 7.84 (s, 2 H), 7.13 - 6.94 (m, 3 H), 6.35 (d, *J* = 7.6 Hz, 1 H), 5.60 - 5.51 (m, 1 H), 4.11 - 3.99 (m, 1 H), 3.99 - 3.79 (m, 3 H), 3.32 - 3.30 (m, 2 H), 2.20 - 2.05 (m, 1 H), 1.77 (t, *J* = 12.4 Hz, 2 H), 1.50 - 1.41 (m, 5 H).

Example 61: Preparation of N- (1- (5-fluoro-2- (2-morpholinoethoxy) phenyl) ethyl) -3- (1H-pyrazol-4-yl) pyrazolo 1,5-a] pyrimidin-5-amine (compound b 16-44)

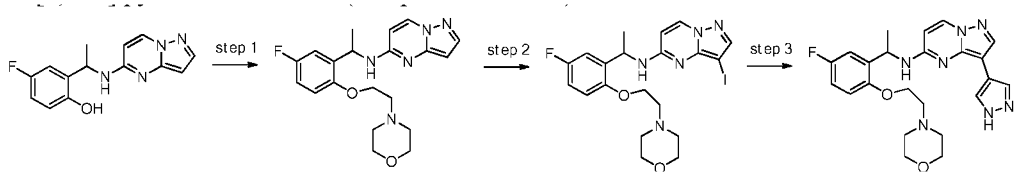


Step 1): the procedure as in step 2 of example 47 was used except that methyl iodide was replaced with 2-(4-morpholine) ethyl bromide.
Step 2): the procedure of step 3 in example 47 was used.
Step 3): the synthesis was performed as in step 4 of example 47.
$^1$H NMR (600 MHz, DMSO-$d_6$) δ 12.68 (s, 1 H), 8.46 (d, *J* = 7.8 Hz, 1 H), 8.04 (s, 1 H), 8.00 - 7.90 (m, 1 H), 7.83 (s, 2 H), 7.13 - 6.91 (m, 3 H), 6.33 (d, *J* = 7.8 Hz, 1 H), 5.60 - 5.45 (m, 1 H), 4.32 - 4.13 (m, 2 H), 3.59 - 3.44 (m, 4 H), 2.81 (t, *J* = 6.0 Hz, 2 H), 2.56 - 2.47 (m, 4 H), 1.43 (d, *J* = 6.6 Hz, 3 H).

Test example 1: in vitro biochemical level inhibition Protein Kinase (PK) activity experiment

The material and the method are as follows: wild-type kinases such as TRKA, TRKB and TRKC, and mutant kinases such as TRKA-G595R, TRKA-G667C, TRKA-F589L, TRKC-G623R and TRKC-G696A, and are derived from Carna Biosciences 08-186, 08-187, 08-197; HTRF KinEASE TKkit (Cisbio 62TK0 PEC); 384 well plates (Greiner corporation); ATP (Life technologies PV 3227), MgCl2 (sigma); PHERAstar FS Multifunctional microplate reader (BMG Co.); low speed centrifuge (StaiteXiangyi corporation); incubator (Binder company).
The control compounds selected were typical compound A disclosed in WO2007147647, typical compound B disclosed in WO2007025540, as well as comparative compound C, comparative compound D, wherein comparative compound C (nuclear magnetic data: $^1$H NMR (400 MHz, DMSO-$d_6$) δ 12.78 - 12.66 (m, 1 H), 8.45 (d, *J* = 7.6 Hz, 1 H), 8.05 (s, 1 H), 8.01 - 7.80 (m, 3 H), 6.94 (s, 1 H), 6.90 (d, *J* = 9.2 Hz, 1 H), 6.77 (dt, *J* = 10.8, 2.4 Hz, 1 H), 6.56 - 6.21 (m, 2 H), 5.20 (d, *J* = 6.0 Hz, 1 H), 4.31 (tt, *J* = 14.4, 3.6 Hz, 2 H), 1.48 (d, *J* = 6.8 Hz, 3 H)) and comparative compound D (nuclear magnetic data: preparation of $^1$H NMR (400 MHz, DMSO-$d_6$) δ 12.70 (br, 1 H), 8.44 (d, *J* = 7.6 Hz, 1 H), 8.05 (s, 1 H), 7.94 (d, *J* = 6.8 Hz, 2 H), 7.85 (s, 1 H), 7.35 - 7.14 (m, 3 H), 6.54 - 6.20 (m, 2 H), 5.28 - 5.11 (m, 1 H), 4.39 - 4.30 (m, 2 H), 1.48 (d, *J* = 6.8 Hz, 3 H)) reference the preparation of the foregoing example 1, with the following differences only in the starting materials and the structure is as follows:

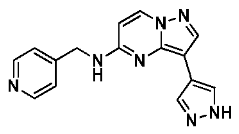

typical compound A

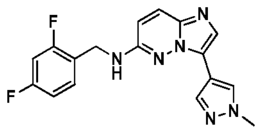

typical compound B

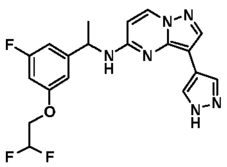

comparative Compound C、

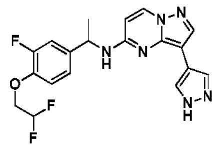

comparative Compound D

Compound dissolution and preservation: preparing a test compound into a mother solution of 10 mmol/L by dimethyl sulfoxide (DMSO) according to solubility, subpackaging and storing at -20 °C;

Preparing a compound working solution: the dispensed compounds were removed from the freezer prior to testing and diluted to 100× concentration with pure DMSO; then the compound was diluted to the desired concentration of 4× with deionized water;

Preparation of 1.33× enzyme buffer (enzyme buffer): 5× enzyme buffer (from HTRF kit) was diluted to 1.33× with deionized water and the corresponding ingredients were added at a final concentration of 1.33×: 1.33mmol/L Dithiothreitol (DTT), 1.33mmol/L MnCl2, 6.65mmol/L MgCl2 and 39.9nmol/L SEB;

Preparation of a kinase working solution: TRKA, TRKB and TRKC were diluted with 1.33× enzyme buffer to the required concentrations of 2× of 0.404 ng/μL, 0.304 ng/μL, and 0.236 ng/μL, respectively;

Preparing a substrate working solution: a mixture of TK Substrate Blr diluted to the desired final concentration of 4× (from HTRF kit) and ATP (10 mM) with 1.33× enzyme buffer; the final ATP concentrations for TRKA, TRKB, and TRKC were: 3.727μmol/L, 2.56μmol/L and 2.526μmol/L. The TK Substrate-biotin (from HTRF KinEASE TKkit) final concentrations were all: 0.2 μmol/L.

Preparing a detection working solution: 16.67 μmol/L of Streptavidin-XL665 (Streptavidin-XL 665) were diluted to the desired final concentration of 4× with HTRF test buffer and then mixed with an equal volume of Antibody-europium Cryptate (Antibody-Cryptate) (both from HTRF kits).

An enzyme reaction step: add 4 μL of kinase working solution to each well of a low volume 384 microwell plate along with 4 μL of 1.33× enzyme buffer as a Negative control (Negative); add 2 μl of compound working solution to the wells, while adding 2 μl of 8% DMSO aqueous solution as a zero compound concentration control (i.e., Positive control, Positive); incubating at 25 °C for 5 min; add 2 μL of substrate working solution to the wells to start the enzymatic reaction, shake the reaction for 30 min at 37 °C.

HTRF reagent detection step: adding 8 detection working solution to the hole to terminate the reaction; reacting for 1h at 25 °C;

Reading of HTRF signal: the PHERAstar FS reading is adopted to detect signals, and the corresponding setting of the instrument is as follows:

Optic module HTRF®
Integration delay, lag time: 50 μs
Integration time: 400 μs
Number of flashes: 200
For the raw data read out per well, the ratio =665 nm/620 nm;
Calculation of inhibition rate:

$$\text{Inhibition rate\%} = \left(1 - \frac{\text{Test ratio} - \text{Negative control ratio}}{\text{Positive control ratio} - \text{Negative control ratio}}\right) \text{X } 100\%$$

Calculation of $IC_{50}$ values: with the logarithm of the compound concentration as abscissa and the inhibition as ordinate, a non-linear curve was fitted in GraphPad Prism 5: log (inhibitor) vs. response -- Variable slope, and calculating the concentration of the compound to be detected, namely $IC_{50}$, when the enzyme activity inhibition rate is 50%.

The experimental results are as follows: TRKA, TRKB and TRKC kinase half inhibitory concentrations ($IC_{50}$, nM)

The present invention provides half maximal inhibitory concentrations ($IC_{50}$) of compounds having the structure

shown in formula (I) and control compounds on TRKA, TRKB and TRKC as shown in table 1:

Table 1: TRKA, TRKB and TRKC kinase inhibitory activity of compound

| Compound number | $IC_{50}$, nM | | |
|---|---|---|---|
| | TRKA | TRKB | TRKC |
| Compound 1 | 1.9 | 1.9 | 3.1 |
| Compound 2 | 2.8 | 2.8 | 3.7 |
| Compound 3 | 5.8 | 4.8 | 7.1 |
| Compound 4 | 12.2 | 15.9 | 24.9 |
| Compound 5 | 3.6 | 3.9 | 11.1 |
| Compound 6 | 12.4 | - | - |
| Compound 7 | 2.4 | 1.3 | 2.4 |
| Compound 8 | 5.4 | 5.7 | 9.8 |
| Compound 9 | 11.4 | - | - |
| Compound 10 | 3.8 | 2.7 | 5.5 |
| Compound 11 | 11.8 | - | - |
| Compound 12 | 3.3 | 3.5 | 4.7 |
| Compound 13 | 10.4 | - | - |
| Compound 14 | 11.3 | 4.2 | 6.7 |
| Compound 15 | 11.7 | - | - |
| Compound 16 | 8.5 | 8.6 | 12.2 |
| Compound 17 | 18.3 | - | - |
| Compound a1-2 | 9.0 | - | - |
| Compound a2-3 | 10.5 | 5.1 | 8.5 |
| Compound a3-4 | 24.2 | - | - |
| Compound a4-8 | 2.4 | 1.5 | 3.5 |
| Compound a5-9 | 4.0 | - | - |
| Compound a6-10 | 26.5 | - | - |
| Compound a7-12 | 23.0 | - | - |
| Compound a8-13 | 40.9 | - | - |
| Compound a9-14 | 67.8 | - | - |
| Compound a10-17 | 2.3 | 1.5 | 15.1 |
| Compound a11-18 | 10.9 | - | - |
| Compound a12-19 | 3.3 | - | - |
| Compound a13-20 | 20.2 | - | - |
| Compound a14-21 | 12.0 | - | - |
| Compound a15-22 | 7.9 | - | - |
| Compound a16-23 | 9.0 | - | - |
| Compound a17-24 | 7.6 | - | - |
| Compound a18-25 | 9.0 | - | - |
| Compound a19-26 | 15.8 | - | - |

(continued)

| Compound number | IC$_{50}$, nM | | |
|---|---|---|---|
| | TRKA | TRKB | TRKC |
| Compound a20-27 | 2.8 | - | - |
| Compound a21-28 | 5.3 | - | - |
| Compound a22-29 | 14.6 | - | - |
| Compound a23-31 | 4.0 | 3.0 | 6.1 |
| Compound a24-32 | 3.1 | 1.4 | 2.9 |
| Compound a25-35 | 24.8 | - | - |
| Compound a26-38 | 11.5 | 6.7 | 9.9 |
| Compound a27-41 | 7.7 | 5.8 | 7.1 |
| Compound a28-43 | 110.5 | - | - |
| Compound b1-1 | 9.5 | - | - |
| Compound b2-5 | 15.2 | - | - |
| Compound b3-6 | 18.1 | 13.0 | 15.5 |
| Compound b4-7 | 3.37 | 3.1 | 3.8 |
| Compound b5-11 | 8.4 | 4.3 | 8.4 |
| Compound b6-15 | 24.0 | - | - |
| Compound b7-16 | 2.5 | 2.5 | 3.0 |
| Compound b8-30 | 2.8 | 3.2 | 3.9 |
| Compound b9-33 | 6.0 | 5.5 | 12.2 |
| Compound b10-34 | 9.7 | 9.8 | 14.1 |
| Compound b11-36 | 88.6 | - | - |
| Compound b12-37 | 4.2 | - | - |
| Compound b13-39 | 7.9 | 5.9 | 8.0 |
| Compound b14-40 | 4.51 | 8.6 | 11.7 |
| Compound b15-42 | 55.0 | - | - |
| Compound b16-44 | 364.4 | - | - |
| Typical Compound A | >1000 | >1000 | >1000 |
| Typical Compound B | >1000 | >1000 | >1000 |
| Control Compound C | >1000 | >1000 | >1000 |
| Control Compound D | >1000 | >1000 | >1000 |

As shown in table 1, the compounds provided by the present invention all showed excellent inhibitory activity against wild TRKA, TRKB, and TRKC kinases, which is significantly superior to typical compound a, typical compound B, Control compound C, and Control compound D.

Test example 2: drug metabolism study in rats

The compounds provided in the previous section of the examples were administered to rats as polyethylene glycol 400 in water (70%). For oral administration, rats were given a dose of 5 mg/kg. Approximately 0.3 mL of blood samples were collected 15, 30, 45 min, 1, 2, 4, 6, 8, 10, 24 h after oral group administration into heparinized Eppendorf tubes, buffered on ice and centrifuged. The whole blood was centrifuged at 8000 rpm for 5min, and plasma was collected,

transferred to a 96-well plate, and stored at -20 °C until detection by LC-MS/MS.

Pharmacokinetic parameters of rats after administration were calculated using a non-compartmental model of the software WinNonlin software.

Peak concentration $C_{max}$: adopting an actual measurement value;

AUC0-t value of area under the curve at time of drug: calculating by adopting a trapezoidal method; $AUC_{0-\infty} = AUC_{0-t} + Ct/ke$, Ct is the blood concentration at the last measurable time point, and ke is the elimination rate constant;

elimination half-life $t_{1/2}$=0.693 / ke;

the absolute bioavailability $F = Dose_{iv} *AUC_{0-t,\ ig} / Dose_{ig} *AUC_{0-t,\ iv} \times 100\%$.

Table 2 lists the pharmacokinetic parameters of the compounds of the invention in rats after intravenous administration. The results indicate that the compounds of the invention have good pharmacokinetic properties including ideal $T_{1/2}$, half-life; $T_{max}$, time of maximum concentration; $C_{max}$, maximum concentration; $AUC_{0-t}$, area under the plasma concentration time curve; Vz, volume of distribution; CL, clearance; $MRT_{last}$, Mean residence time.

Table 3 lists the pharmacokinetic parameters of the compounds of the invention in rats after oral administration. The results indicate that the compounds of the invention have good pharmacokinetic properties including ideal $T_{1/2}$, half-life; $T_{max}$, time of maximum concentration; $C_{max}$, maximum concentration; $AUC_{0-t}$, area under the plasma concentration time curve; $V_Z$, volume of distribution; CL, clearance; $MRT_{last}$, Mean residence time; *F*, oral bioavailabilityy.

Table 2: primary pharmacokinetic parameters for rat intravenous administration of Compounds

| Intravenous injection group 1 mg/kg | | Compound 1 | Compound 2 | Compound 5 | Compound a24-32 | Compound b8-30 |
|---|---|---|---|---|---|---|
| $T_{1/2}$ | h | 5.10 | 1.71 | 5.7 | 5.51 | 4.95 |
| $T_{max}$ | h | - | - | - | - | - |
| $C_{max}$ | ng/mL | 259.67 | 297.7 | 104.1 | 154.7 | 174.2 |
| $AUC_{0-t}$ | h*ng/mL | 434.39 | 352.8 | 194.1 | 343.1 | **518.4** |
| $V_z$ | L/kg | 16.86 | 6.80 | 40.0 | 22.76 | 12.66 |
| CL | L/h/kg | 2.31 | 2.76 | 5.0 | 2.86 | 1.82 |
| $MRT_{last}$ | h | 3.62 | 1.49 | 3.89 | 4.29 | 4.20 |

Table 3: major pharmacokinetic parameters for oral rat administration of Compounds

| Oral group 5 mg/kg | | Compound 1 | Compound 2 | Compound 5 | Compound a24-32 | Compound b8-30 |
|---|---|---|---|---|---|---|
| $T_{1/2}$ | h | 4.44 | 2.85 | 5.7 | 3.2 | 4.00 |
| $T_{max}$ | h | 0.83 | 0.67 | 0.25 | 0.5 | 2.00 |
| $C_{max}$ | ng/mL | 109.33 | 149.9 | 217.7 | 195.4 | 282.7 |
| $AUC_{0-t}$ | h*ng/mL | 650.14 | 533.2 | 478.6 | 816.5 | **1457.1** |
| $V_z$ | L/kg | 48.49 | 37.61 | 79.26 | 28.2 | 19.23 |
| CL | L/h/kg | 7.57 | 10.12 | 9.5 | 6.3 | 3.46 |
| $MRT_{last}$ | h | 5.69 | 2.62 | 3.6 | 3.8 | 4.27 |
| *F* | % | 29.9 | 30.2 | 49.3 | 47.6 | 56.2 |

Test example 3: inhibitory Activity of the Compound of the present invention against five TRK kinase mutants This test example was carried out by the same test method as in test example 1.

The results of this test example are shown in Table 4.

TABLE 4

| Compound number | $IC_{50}$, nM | | | | |
|---|---|---|---|---|---|
| | TRKA-G595R | TRKA-G667C | TRKA-F589L | TRKC-G623R | TRKC-G696A |
| Compound 1 | 0.84 | 1.76 | 0.22 | 0.45 | 0.76 |
| Compound 2 | 1.0 | 0.94 | 0.27 | 1.7 | 0.20 |
| Compound 5 | 0.88 | 0.95 | 0.29 | 0.61 | 1.0 |
| Compound a4-8 | 1.2 | 3.2 | 0.40 | 2.9 | 1.1 |
| Compound a11-18 | 1.1 | 1.0 | 0.32 | 0.93 | 0.77 |
| Compound a24-32 | 0.94 | 1.39 | 0.44 | 1.1 | 0.75 |
| Compound a23-31 | 1.5 | 1.37 | 0.38 | 1.7 | 0.88 |
| Compound b7-16 | 1.1 | 1.0 | 0.32 | 0.93 | 0.77 |
| Compound b8-30 | 0.75 | 0.76 | 0.44 | 0.73 | 1.3 |
| Typical Compound A | >1000 | >1000 | >1000 | >1000 | >1000 |
| Typical Compound B | >1000 | >1000 | >1000 | >1000 | >1000 |
| Control Compound C | >1000 | >1000 | >1000 | >1000 | >1000 |
| Control Compound D | >1000 | >1000 | >1000 | >1000 | >1000 |

As can be seen from Table 4, the compound of the invention has better inhibitory activity on five TRK kinase mutants than on wild TRK kinase, and is expected to effectively overcome the clinically reported tumor drug resistance.

Test example 4: antitumor Activity of the Compounds of the invention on nude mouse xenograft tumor model

The efficacy of the compounds of the invention was evaluated by a standard murine model of the transplanted tumor. Human NSCLC H2228 was cultured, collected, and inoculated subcutaneously to 5-6 weeks old female nude mice (BALB/c, Shanghai Ling Chang Biotech, Ltd.). When the tumor volume reached 100-150mm$^3$, the animals were randomly divided into a solvent control group (70% PEG-400 in water) and a compound group (6 animals per group). Animals were subsequently gavaged with the compounds of the examples (corresponding dose, dissolved in 70% PEG-400 in water), starting anywhere from 0 to 7 days after tumor cell inoculation, and were performed twice daily in the experiment.

The experimental index is to examine the influence of the compound of the embodiment on the growth of the tumor, and the specific index is T/C% or tumor inhibition rate TGI (%).

Tumor diameters were measured twice weekly with a vernier caliper and tumor volume (V) was calculated as:

$V = 1/2 \times a \times b^2$ wherein a and b represent length and width, respectively.

$T/C(\%) = (T-T_0)/(C-C_0) \times 100$ where T, C is the tumor volume at the end of the experiment; $T_0$, $C_0$ are tumor volumes at the beginning of the experiment.

Tumor inhibition rate (TGI) (%) =100-T/C (%).

When tumor regression occurred, Tumor inhibition rate (TGI) (%) $=100-(T-T_0)/T_0 \times 100$

Partial tumor regression (PR) is defined if the tumor shrinks from the initial volume, i.e., $T<T_0$ or $C<C_0$; if the tumor completely disappears, it is defined as complete tumor regression (CR).

Comparison between the two groups of tumor volumes was tested using a two-tailed Student's t test, with P <0.05 defined as statistically significant differences.

The results of this test example are shown in Table 5.

BID below refers to twice daily dosing.

TABLE 5

| Grouping | Dosing | Tumor growth inhibition value D14 | complete regression | Partially regression |
|---|---|---|---|---|
| Solvent | BID, D0-7 | - | - | - |

(continued)

| Grouping | Dosing | Tumor growth inhibition value D14 | complete regression | Partially regression |
|---|---|---|---|---|
| Compound 5, 50mg/kg | BID, D0-7 | 155% | 3/6 | 2/6 |

As can be seen from table 5, the compounds of the present invention showed excellent antitumor activity in the human non-small cell lung cancer H2228 nude mouse xenograft tumor model. Wherein, the compound 5(50 mg/kg, BID×8) obviously inhibits the growth of human non-small cell lung cancer H2228 nude mouse subcutaneous transplantation tumor, when the compound is administrated to D7, the tumor inhibition rate is 182%, and 4/6 tumor is completely regressed; the dosing was stopped from D8 and to the end of the experiment (D14), the tumor inhibition rate was 155%, the tumors in 3/6 fully regressed and in 2/6 partially regressed.

Test example 5: antitumor Activity of the Compounds of the invention on nude mouse xenograft tumor model

The efficacy of the compounds of the invention was assessed by a standard murine model of transplanted tumors. Human NSCLC H2228 was cultured, collected, and inoculated subcutaneously to 5-6 weeks old female nude mice (BALB/c, Shanghai Ling Chang Biotech, Ltd.). When the tumor volume reached 100-150mm3, the animals were randomly divided into a solvent control group (70% PEG-400 in water) and a compound group (6 animals per group). Animals were subsequently gavaged with the compounds of the examples (corresponding doses, dissolved in 70% PEG-400 in water), starting anywhere from 0 to 13 days after tumor cell inoculation, and were performed once or twice daily in the experiment.
The experimental index is to examine the influence of the compound of the embodiment on the growth of the tumor, and the specific index is T/C% or tumor inhibition rate TGI (%).
Tumor diameters were measured twice weekly with a vernier caliper and tumor volume (V) was calculated as:

$V=1/2\times a\times b^2$ wherein a and b represent length and width, respectively.
$T/C(\%)=(T-T_0)/(C-C_0)\times 100$ where T, C is the tumor volume at the end of the experiment; $T_0$, $C_0$ are tumor volumes at the beginning of the experiment.
Tumor inhibition rate (TGI) (%) =100-T/C (%).
When tumor regression occurred, Tumor inhibition rate (TGI) (%) $=100-(T-T_0)/T_0\times 100$
Partial tumor regression (PR) is defined if the tumor shrinks from the initial volume, i.e., $T<T_0$ or $C<C_0$; if the tumor completely disappears, it is defined as complete tumor regression (CR).

Comparison between the two groups of tumor volumes was tested using a two-tailed Student's t test, with P <0.05 defined as statistically significant differences.
The results of this test example are shown in Table 6.
BID below indicates twice daily dosing and QD indicates once daily dosing.

TABLE 6

| Grouping | Dosing | Tumor growth inhibition value/ D14 | Partially regression |
|---|---|---|---|
| Solvent | BID, D0-7 | - | - |
| Compound b8-30 25mg/kg | BID, D0-13 | 127% | 6/6 |
| Compound b8-30 50mg/kg | QD, D0-10 | 110% | 3/6 |

As can be seen from table 6, the compounds of the present invention showed excellent antitumor activity in the human non-small cell lung cancer H2228 nude mouse xenograft tumor model. Wherein, the compound b8-30 (25 mg/kg, BID×14) obviously inhibits the growth of human non-small cell lung cancer H2228 nude mouse subcutaneous transplantation tumor, when the compound is administered to D7, the tumor inhibition rate is 95%, the dose is increased from D8 to 50 mg/kg, the tumor appears and regresses, and when the experiment is finished (D14), the tumor inhibition rate is 127%, and the tumor 6/6 appears and partially regresses; compound b8-30 (50 mg/kg QD, QD×11) showed 96% tumor suppression in H2228 nude mice subcutaneous transplantable tumors (D7), with tumor regression occurring from the initial dose of D8 to 100 mg/kg, and to the end of the experiment (D14), 110% tumor suppression and 3/6 tumor partial regression.
The results show that the pyrazolopyrimidine compound having the structure shown in formula (I) or a pharmaceutically acceptable salt thereof, or a stereoisomer, a geometric isomer, a tautomer, a nitrogen oxide, a hydrate, a

solvate thereof, provided by the invention, has excellent inhibitory activity on TRK kinase, and simultaneously, can show good antitumor activity on an animal level.

The preferred embodiments of the present invention have been described above in detail, but the present invention is not limited thereto.

## Claims

1. A pyrazolopyrimidine compound having a structure represented by formula (I), or a pharmaceutically acceptable salt thereof, or a stereoisomer, a geometric isomer, a tautomer, a nitrogen oxide, a hydrate, a solvate thereof,

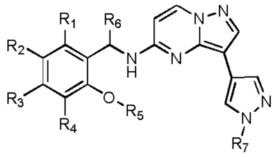

formula (I),

wherein, in the formula (I),

$R_1$, $R_2$, $R_3$ and $R_4$ are each independently selected from H, halogen, $C_{1-12}$ alkyl, $C_{1-12}$ alkyl substituted by 1-6 halogen;
$R_5$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by alkoxy, $C_{2-12}$ alkyl substituted by cyano, and $C_{2-12}$ cycloalkyl containing 1-3 heteroatoms selected from N, O and S;
$R_6$ is selected from the group consisting of H, $C_{1-12}$ alkyl, $C_{1-12}$ alkyl substituted by hydroxy, halogen;
$R_7$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by cyano, $C_{2-12}$ cycloalkyl containing 1-3 hetero atoms selected from N, O and S, $C_{2-12}$ acyl, sulfonyl.

2. The compound according to claim 1, wherein, in formula (I),

$R_1$, $R_2$, $R_3$ and $R_4$ are each independently selected from the group consisting of H, fluorine, chlorine, bromine, $C_{1-8}$ alkyl, $C_{1-8}$ alkyl substituted with 1-6 halogens selected from the group consisting of fluorine, chlorine and bromine;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of H, $C_{1-8}$ alkyl, $C_{1-8}$ alkyl substituted by hydroxy, halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 hetero atoms selected from N, O and S, $C_{2-8}$ acyl, sulfonyl;
preferably, in the formula (I),
$R_1$, $R_2$, $R_3$ and $R_4$ are each independently selected from the group consisting of H, fluorine, chlorine, bromine, $C_{1-6}$ alkyl, $C_{1-6}$ alkyl substituted with 1-4 halogens selected from the group consisting of fluorine, chlorine and bromine;
$R_5$ is selected from H, $C_{1-6}$ alkyl, $C_{1-6}$ alkyl substituted by 1-4 halogens selected from fluorine, chlorine and bromine, $C_{1-6}$ alkyl substituted by hydroxy, $C_{2-8}$ alkyl substituted by alkoxy, $C_{2-6}$ alkyl substituted by cyano, and $C_{2-8}$ cycloalkyl containing 1-3 heteroatoms selected from N, O and S;
$R_6$ is selected from the group consisting of H, $C_{1-6}$ alkyl, $C_{1-6}$ alkyl substituted by hydroxy, halogen;
$R_7$ is selected from H, $C_{1-6}$ alkyl, $C_{1-6}$ alkyl substituted by 1-4 halogens selected from fluorine, chlorine and bromine, $C_{1-6}$ alkyl substituted by hydroxy, $C_{2-6}$ alkyl substituted by cyano, $C_{2-8}$ cycloalkyl containing 1-3 hetero atoms selected from N, O and S, $C_{2-6}$ acyl, sulfonyl.

3. The compound according to claim 1 or 2, wherein, in formula (I),

at least one of $R_1$, $R_2$, $R_3$, $R_4$ and $R_5$ contains an F atom;
and $R_5$ is selected from $C_{1-12}$ alkyl substituted with 1-6 halogens;

preferably, in the formula (I),
at least one of $R_1$, $R_2$, $R_3$, $R_4$ and $R_5$ contains an F atom;
and $R_5$ is selected from $C_{1-8}$ alkyl substituted with 1-6 halogens selected from fluorine, chlorine and bromine.

**4.** The compound according to claim 3, wherein, in formula (I),

$R_1$, $R_2$, $R_3$ and $R_4$ are each independently selected from H, halogen, $C_{1-12}$ alkyl, $C_{1-12}$ alkyl substituted by 1-6 halogen;
$R_5$ is $-CH_2CHF_2$;
$R_6$ is selected from the group consisting of $C_{1-12}$ alkyl, $C_{1-12}$ alkyl substituted by hydroxy, halogen;
$R_7$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by cyano, $C_{2-12}$ cycloalkyl containing 1-3 hetero atoms selected from N, O and S, $C_{2-12}$ acyl, sulfonyl;
preferably, in the formula (I),
$R_1$, $R_2$, $R_3$ and $R_4$ are each independently selected from the group consisting of H, fluorine, chlorine, bromine, $C_{1-8}$ alkyl, $C_{1-8}$ alkyl substituted with 1-6 halogens selected from the group consisting of fluorine, chlorine and bromine;
$R_5$ is $-CH_2CHF_2$;
$R_6$ is selected from the group consisting of $C_{1-8}$ alkyl, $C_{1-8}$ alkyl substituted by hydroxy, halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{1-8}$ alkyl substituted by cyano, $C_{2-10}$ cycloalkyl containing 1-3 hetero atoms selected from N, O and S, $C_{2-8}$ acyl, sulfonyl;
preferably, in the formula (I),
$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;
$R_2$ is H or F;
$R_5$ is $-CH_2CHF_2$;
$R_6$ is selected from the group consisting of $C_{1-8}$ alkyl, $C_{1-8}$ alkyl substituted by hydroxy, halogen;
$R_7$ is selected from H, $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 hetero atoms selected from N, O and S, $C_{2-8}$ acyl, sulfonyl.

**5.** The compound of claim 4, wherein the compound of formula (I) is selected from at least one of the following compounds:

Compound 1： 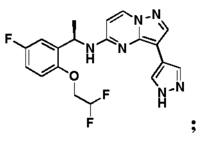 ； Compound 2： 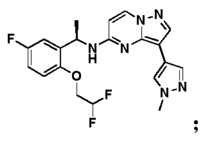 ； Compound 3： 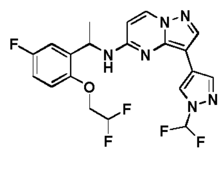 ；

Compound 4： 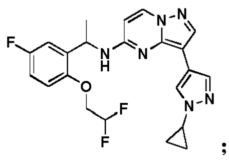 ； Compound 5： 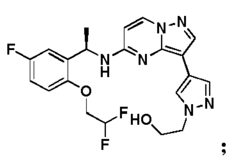 ； Compound 6： 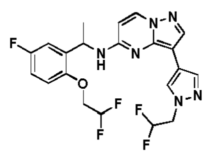 ；

Compound 7： 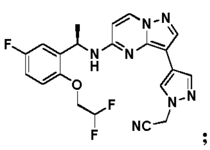 ； Compound 8： 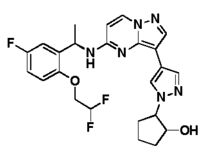 ； Compound 9： 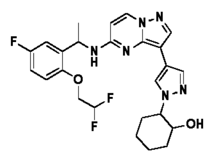 ；

Compound 10: 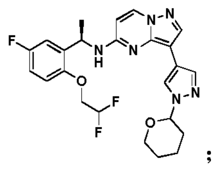 ; Compound 11: 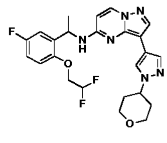 ; Compound 12: 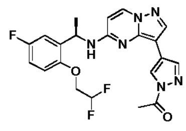 ;

Compound 13: 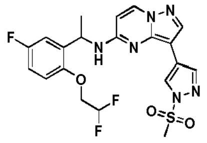 ; Compound 14: 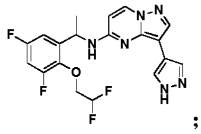 ; Compound 15: 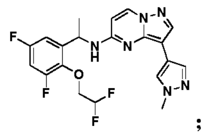 ;

Compound 16: 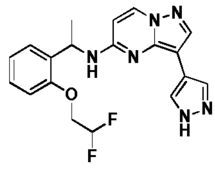 ; Compound 17: 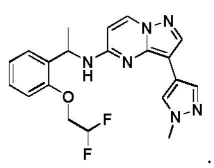 .

**6.** The compound according to claim 1 or 2, wherein, in formula (I),

$R_7$ is selected from $C_{1-12}$ alkyl, $C_{1-12}$ alkyl substituted by 1-6 halogens, $C_{1-12}$ alkyl substituted by hydroxy, $C_{2-12}$ alkyl substituted by cyano, $C_{2-12}$ cycloalkyl containing 1-3 heteroatoms selected from N, O and S, $C_{2-12}$ acyl, sulfonyl;
preferably, in the formula (I),
$R_7$ is selected from $C_{1-6}$ alkyl, $C_{1-6}$ alkyl substituted by 1-4 halogens selected from fluorine, chlorine and bromine, $C_{1-6}$ alkyl substituted by hydroxy, $C_{2-6}$ alkyl substituted by cyano, $C_{2-8}$ cycloalkyl containing 1-3 heteroatoms selected from N, O and S, $C_{2-6}$ acyl, sulfonyl.

**7.** The compound according to claim 6, wherein, in formula (I),

$R_1$, $R_3$ and $R_4$ are each independently selected from the group consisting of H, halogen, $C_{1-12}$ alkyl, $C_{1-12}$ alkyl substituted with 1-6 halogen;
$R_2$ is halogen;
$R_5$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by alkoxy, $C_{2-12}$ alkyl substituted by cyano, and $C_{2-12}$ cycloalkyl containing 1-3 heteroatoms selected from N, O and S;
$R_6$ is selected from the group consisting of $C_{1-12}$ alkyl, $C_{1-12}$ alkyl substituted by hydroxy, halogen;
$R_7$ is selected from $C_{1-12}$ alkyl, $C_{1-12}$ alkyl substituted by 1-6 halogens, $C_{1-12}$ alkyl substituted by hydroxy, $C_{2-12}$ alkyl substituted by cyano, $C_{2-12}$ cycloalkyl containing 1-3 heteroatoms selected from N, O and S, $C_{2-12}$ acyl, sulfonyl;
preferably, in the formula (I),
$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;
$R_2$ is F;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{1-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted by hydroxy, halogen;
$R_7$ is selected from $C_{1-8}$ alkyl, $C_{1-8}$ alkyl substituted by 1-6 halogens selected from fluorine, chlorine and bromine, $C_{1-8}$ alkyl substituted by hydroxy, $C_{1-8}$ alkyl substituted by cyano, $C_{2-10}$ cycloalkyl containing 1-3 heteroatoms selected from N, O and S, $C_{2-8}$ acyl, sulfonyl.

**8.** The compound according to claim 7, wherein, in formula (I),

$R_1$, $R_3$ and $R_4$ are all H; $R_2$ is F;
$R_5$ is selected from H, $C_{1-8}$ alkyl, $C_{1-8}$ alkyl substituted by 1-6 halogen atoms selected from fluorine, chlorine

and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted by hydroxy, halogen;
$R_7$ is selected from $C_{1-8}$ alkyl, $C_{1-8}$ alkyl substituted by 1-6 halogens selected from fluorine, chlorine and bromine, $C_{1-8}$ alkyl substituted by hydroxy, $C_{2-8}$ alkyl substituted by cyano, $C_{2-10}$ cycloalkyl containing 1-3 heteroatoms selected from N, O and S, $C_{2-8}$ acyl, sulfonyl;
preferably, the compound of formula (I) is selected from at least one of the following compounds:

Compound a1-2: 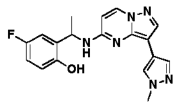 ; Compound a2-3: 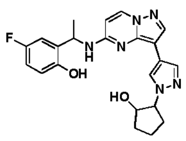 ; Compound a3-4: 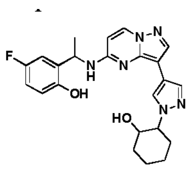 ;

Compound a4-8: 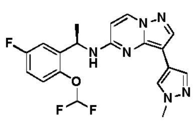 ; Compound a5-9: 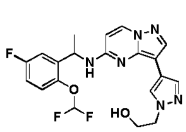 ; Compound a6-10: 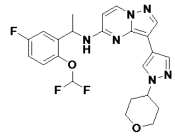 ;

Compound a7-12: 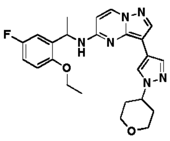 ; Compound a8-13: 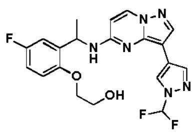 ; Compound a9-14: 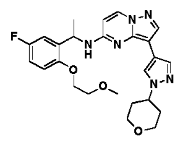 ;

Compound a10-17: 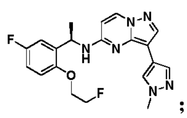 ; Compound a11-18: 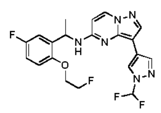 ; Compound a12-19: 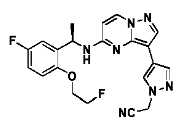 ;

Compound a13-20: 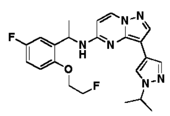 ; Compound a14-21: 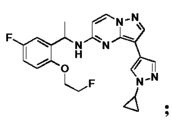 ; Compound a15-22: 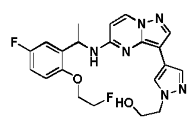 ;

Compound a16-23: 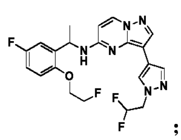 ; Compound a17-24: 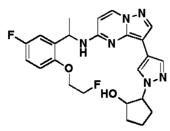 ; Compound a18-25: 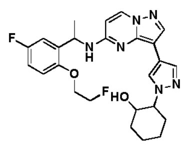 ;

Compound a19-26: 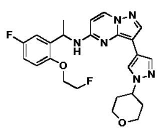 ; Compound a20-27: 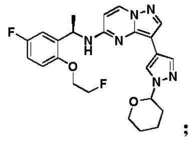 ; Compound a21-28: 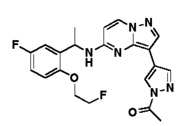 ;

Compound a22-29: 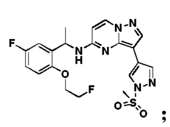 ; Compound a23-31: 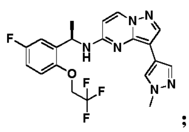 ; Compound a24-32: 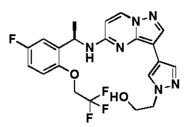 ;

Compound a25-35: 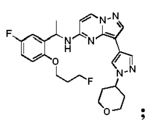; Compound a26-38: 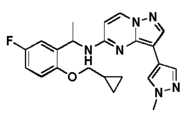; Compound a27-41: 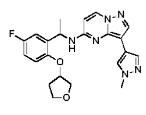; Compound a28-43: 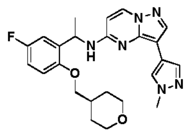.

9. The compound according to claim 1 or 2, wherein, in formula (I), $R_7$ is H.

10. The compound according to claim 9, wherein, in formula (I),

$R_1$, $R_3$ and $R_4$ are each independently selected from the group consisting of H, halogen, $C_{1-12}$ alkyl, $C_{1-12}$ alkyl substituted with 1-6 halogen;
$R_2$ is halogen;
$R_5$ is selected from H, $C_{1-12}$ alkyl, $C_{1-12}$ alkyl substituted by 1-6 halogens, $C_{1-12}$ alkyl substituted by hydroxy, $C_{2-12}$ alkyl substituted by alkoxy, $C_{2-12}$ alkyl substituted by cyano, and $C_{2-12}$ cycloalkyl containing 1-3 heteroatoms selected from N, O and S;
$R_6$ is selected from the group consisting of $C_{1-12}$ alkyl, $C_{1-12}$ alkyl substituted by hydroxy, halogen;
preferably, in the formula (I),
$R_1$, $R_3$ and $R_4$ are each independently selected from the group consisting of H, fluoro, chloro, bromo, $C_{1-8}$ alkyl, $C_{1-8}$ alkyl substituted with 1-6 halogens selected from fluoro, chloro and bromo;
$R_2$ is F;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted by hydroxy, halogen;
preferably, in the formula (I),
$R_1$, $R_3$ and $R_4$ are all H;$R_2$ is F;
$R_5$ is selected from H, $C_{1-8}$ alkyl, $C_{1-8}$ alkyl substituted by 1-6 halogen atoms selected from fluorine, chlorine and bromine, $C_{1-8}$ alkyl substituted by hydroxy, $C_{2-8}$ alkyl substituted by alkoxy, $C_{2-8}$ alkyl substituted by cyano, and $C_{2-10}$ cycloalkyl containing 1-3 hetero atoms selected from N, O and S;
$R_6$ is selected from the group consisting of $C_{1-8}$ alkyl, $C_{1-8}$ alkyl substituted by hydroxy, halogen.

11. The compound of claim 10, wherein the compound of formula (I) is selected from at least one of the following compounds:

Compound b1-1: 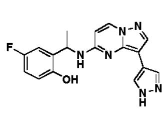; Compound b2-5: 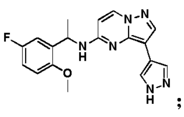; Compound b3-6: 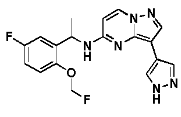; Compound b4-7: 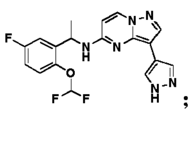; Compound b5-11: 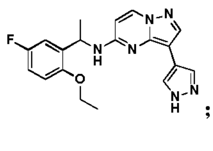; Compound b6-15: 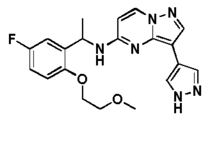;

Compound b7-16: 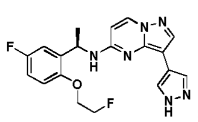; Compound b8-30: 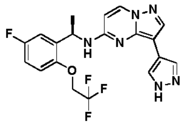; Compound b9-33: 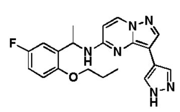;

Compound b10-34: 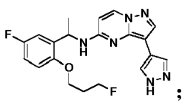; Compound b11-36: 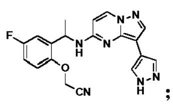; Compound b12-37: 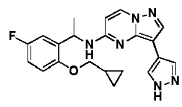;

Compound b13-39: 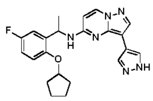; Compound b14-40: 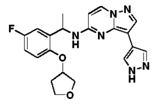; Compound b15-42: 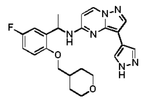; Compound b16-44: 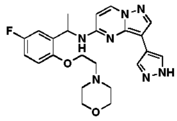.

**12.** A pharmaceutical composition comprising a pharmaceutically acceptable carrier, excipient or diluent, and as an active ingredient a pyrazolopyrimidine compound having a structure represented by formula (I) or a pharmaceutically acceptable salt thereof, or a stereoisomer, a geometric isomer, a tautomer, a nitrogen oxide, a hydrate, a solvatethereof, according to any one of claims 1 to 11.

**13.** A pyrazolopyrimidine compound having a structure represented by formula (I) or a pharmaceutically acceptable salt thereof, or a stereoisomer, a geometric isomer, a tautomer, an oxynitride, a hydrate, a solvate, according to any one of claims 1 to 11, or a pharmaceutical composition according to claim 12 for use in the prevention and/or treatment of tumors;
preferably, the tumor is at least one of breast cancer, large intestine cancer, lung cancer, thyroid cancer, skin cancer, bone cancer, melanoma, leukemia, salivary gland tumor, neuroendocrine tumor, lymphoma, brain tumor, neuroblastoma, ovarian cancer, pancreatic cancer, mesothelioma, esophageal cancer, pulmonary sarcoma, medulloblastoma, glioblastoma, colon cancer, hepatoma, retinoblastoma, renal carcinoma, bladder cancer, osteosarcoma, gastric cancer, uterine cancer, vulval cancer, small intestine cancer, prostate cancer, bile duct cancer, ureter cancer, adrenal cortex cancer, or head and neck cancer.

## Patentansprüche

**1.** Pyrazolopyrimidinverbindung mit einer Struktur, die durch Formel (I) dargestellt ist, oder ein pharmazeutisch unbedenkliches Salz davon oder ein Stereoisomer, ein geometrisches Isomer, ein Tautomer, ein Stickoxid, ein Hydrat, ein Solvat davon,

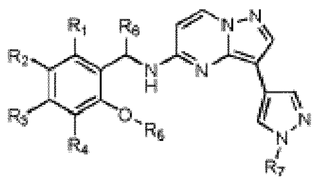

Formel (I),

wobei in der Formel (I)

$R_1$, $R_2$, $R_3$ und $R_4$ jeweils unabhängig aus H, Halogen, $C_{1-12}$-Alkyl, durch 1-6 Halogene substituiertem $C_{1-12}$-Alkyl ausgewählt sind;
$R_5$ aus H, $C_{1-12}$-Alkyl, durch 1-6 Halogene substituiertem $C_{1-12}$-Alkyl, durch Hydroxy substituiertem $C_{1-12}$-Alkyl, durch Alkoxy substituiertem $C_{2-12}$-Alkyl, durch Cyano substituiertem $C_{2-12}$-Alkyl und $C_{2-12}$-Cycloalkyl, enthaltend 1-3 Heteroatome, die aus N, O und S ausgewählt sind, ausgewählt ist;
$R_6$ aus der Gruppe bestehend aus H, $C_{1-12}$-Alkyl, durch Hydroxy substituiertem $C_{1-12}$-Alkyl, Halogen ausgewählt ist;
$R_7$ aus H, $C_{1-12}$-Alkyl, durch 1-6 Halogene substituiertem $C_{1-12}$-Alkyl, durch Hydroxy substituiertem $C_{1-12}$-Alkyl, durch Cyano substituiertem $C_{2-12}$-Alkyl, $C_{2-12}$-Cycloalkyl, enthaltend 1-3 Heteroatome, die aus N, O und S

ausgewählt sind, $C_{2-12}$-Acyl, Sulfonyl ausgewählt ist.

2. Verbindung nach Anspruch 1, wobei in Formel (I)

$R_1$, $R_2$, $R_3$ und $R_4$ jeweils unabhängig aus der Gruppe bestehend aus H, Fluor, Chlor, Brom, $C_{1-8}$-Alkyl, durch 1-6 Halogene, die aus der Gruppe bestehend aus Fluor, Chlor und Brom ausgewählt sind, substituiertem $C_{1-8}$-Alkyl ausgewählt sind;
$R_5$ aus H, $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Alkoxy substituiertem $C_{2-8}$-Alkyl, durch Cyano substituiertem $C_{2-8}$-Alkyl und $C_{2-10}$-Cycloalkyl, enthaltend 1-3 Heteroatome, die aus N, O und S ausgewählt sind, ausgewählt ist;
$R_6$ aus der Gruppe bestehend aus H, $C_{1-8}$-Alkyl, durch Hydroxy substituiertem $C_{1-8}$-Alkyl, Halogen ausgewählt ist;
$R_7$ aus H, $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Cyano substituiertem $C_{2-8}$-Alkyl, $C_{2-10}$-Cycloalkyl, enthaltend 1-3 Heteroatome, die aus N, O und S ausgewählt sind, $C_{2-8}$-Acyl, Sulfonyl ausgewählt ist;
vorzugsweise wobei in der Formel (I)
$R_1$, $R_2$, $R_3$ und $R_4$ jeweils unabhängig aus der Gruppe bestehend aus H, Fluor, Chlor, Brom, $C_{1-6}$-Alkyl, durch 1-4 Halogene, die aus der Gruppe bestehend aus Fluor, Chlor und Brom ausgewählt sind, substituiertem $C_{1-6}$-Alkyl ausgewählt sind;
$R_5$ aus H, $C_{1-6}$-Alkyl, durch 1-4 Halogene, die aus Fluor, Chlor und Brom ausgewählt sind, substituiertem $C_{1-6}$-Alkyl, durch Hydroxy substituiertem $C_{1-6}$-Alkyl, durch Alkoxy substituiertem $C_{2-8}$-Alkyl, durch Cyano substituiertem $C_{2-6}$-Alkyl und $C_{2-8}$-Cycloalkyl, enthaltend 1-3 Heteroatome, die aus N, O und S ausgewählt sind, ausgewählt ist;
$R_6$ aus der Gruppe bestehend aus H, $C_{1-6}$-Alkyl, durch Hydroxy substituiertem $C_{1-6}$-Alkyl, Halogen ausgewählt ist;
$R_7$ aus H, $C_{1-6}$-Alkyl, durch 1-4 Halogene, die aus Fluor, Chlor und Brom ausgewählt sind, substituiertem $C_{1-6}$-Alkyl, durch Hydroxy substituiertem $C_{1-6}$-Alkyl, durch Cyano substituiertem $C_{2-6}$-Alkyl, $C_{2-8}$-Cycloalkyl, enthaltend 1-3 Heteroatome, die aus N, O und S ausgewählt sind, $C_{2-6}$-Acyl, Sulfonyl ausgewählt ist.

3. Verbindung nach Anspruch 1 oder 2, wobei in Formel (I)

mindestens eines von $R_1$, $R_2$, $R_3$, $R_4$ und $R_5$ ein F-Atom enthält;
und $R_5$ aus mit 1-6 Halogenen substituiertem $C_{1-12}$-Alkyl ausgewählt ist;
vorzugsweise wobei in der Formel (I)
mindestens eines von $R_1$, $R_2$, $R_3$, $R_4$ und $R_5$ ein F-Atom enthält; und $R_5$ aus mit 1-6 Halogenen, die aus Fluor, Chlor und Brom ausgewählt sind, substituiertem $C_{1-8}$-Alkyl ausgewählt ist.

4. Verbindung nach Anspruch 3, wobei in Formel (I)

$R_1$, $R_2$, $R_3$ und $R_4$ jeweils unabhängig aus H, Halogen, $C_{1-12}$-Alkyl, durch 1-6 Halogene substituiertem $C_{1-12}$-Alkyl ausgewählt sind;
$R_5$ $-CH_2CHF_2$ ist;
$R_6$ aus der Gruppe bestehend aus $C_{1-12}$-Alkyl, durch Hydroxy substituiertem $C_{1-12}$-Alkyl, Halogen ausgewählt ist;
$R_7$ aus H, $C_{1-12}$-Alkyl, durch 1-6 Halogene substituiertem $C_{1-12}$-Alkyl, durch Hydroxy substituiertem $C_{1-12}$-Alkyl, durch Cyano substituiertem $C_{2-12}$-Alkyl, $C_{2-12}$-Cycloalkyl, enthaltend 1-3 Heteroatome, die aus N, O und S ausgewählt sind, $C_{2-12}$-Acyl, Sulfonyl ausgewählt ist;
vorzugsweise wobei in der Formel (I)
$R_1$, $R_2$, $R_3$ und $R_4$ jeweils unabhängig aus der Gruppe bestehend aus H, Fluor, Chlor, Brom, $C_{1-8}$-Alkyl, durch 1-6 Halogene, die aus der Gruppe bestehend aus Fluor, Chlor und Brom ausgewählt sind, substituiertem $C_{1-8}$-Alkyl ausgewählt sind;
$R_5$ $-CH_2CHF_2$ ist;
$R_6$ aus der Gruppe bestehend aus $C_{1-8}$-Alkyl, durch Hydroxy substituiertem $C_{1-8}$-Alkyl, Halogen ausgewählt ist;
$R_7$ aus H, $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Cyano substituiertem $C_{1-8}$-Alkyl, $C_{2-10}$-Cycloalkyl, enthaltend 1-3 Heteroatome, die aus N, O und S ausgewählt sind, $C_{2-8}$-Acyl, Sulfonyl ausgewählt ist;
vorzugsweise wobei in der Formel (I)
$R_1$, $R_3$ und $R_4$ jeweils unabhängig aus der Gruppe bestehnd aus H, Fluor, Chlor, Brom, $C_{1-8}$-Alkyl, durch 1-6

Halogene, die aus Fluor, Chlor und Brom ausgewählt sind, substituiertem $C_{1-8}$-Alkyl ausgewählt sind;
$R_2$ H oder F ist;
$R_5$ -$CH_2CHF_2$ ist;
$R_6$ aus der Gruppe bestehend aus $C_{1-8}$-Alkyl, durch Hydroxy substituiertem $C_{1-8}$-Alkyl, Halogen ausgewählt ist;
$R_7$ aus H, $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Cyano substituiertem $C_{2-8}$-Alkyl, $C_{2-10}$-Cycloalkyl, enthaltend 1-3 Heteroatome, die aus N, O und S ausgewählt sind, $C_{2-8}$-Acyl, Sulfonyl ausgewählt ist.

**5.** Verbindung nach Anspruch 4, wobei die Verbindung der Formel (I) aus mindestens einer der folgenden Verbindungen ausgewählt ist:

Verbindung 1: 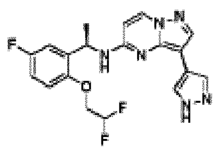; Verbindung 2: 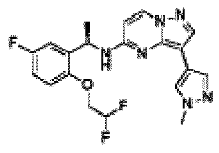;

Verbindung 3: 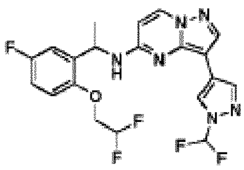; Verbindung 4: 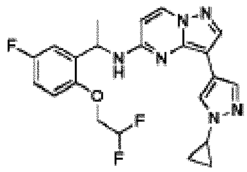;

Verbindung 5: 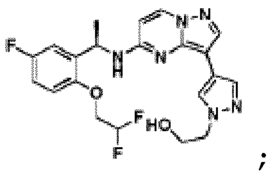; Verbindung 6: 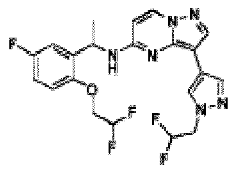;

Verbindung 7: 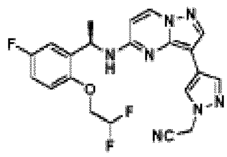; Verbindung 8: 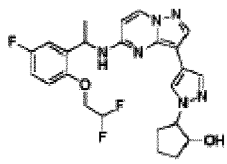;

Verbindung 9: 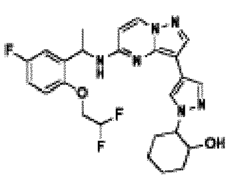; Verbindung 10: 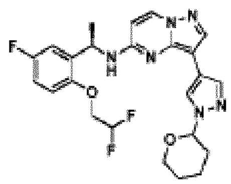;

Verbindung 11: 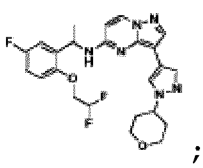; Verbindung 12: 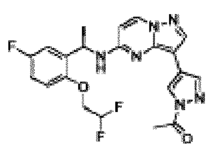;

Verbindung 13: 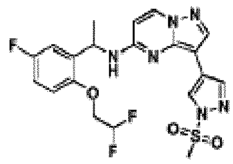 ; Verbindung 14: 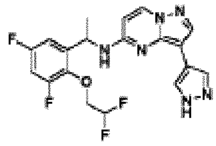 ;

Verbindung 15: 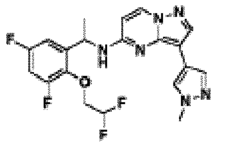 ; Verbindung 16: 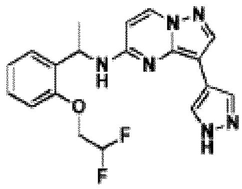 ;

Verbindung 17: 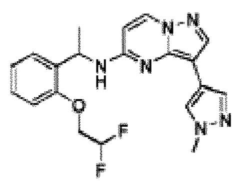 .

**6.** Verbindung nach Anspruch 1 oder 2, wobei in Formel (I)

$R_7$ aus $C_{1-12}$-Alkyl, durch 1-6 Halogene substituiertem $C_{1-12}$-Alkyl, durch Hydroxy substituiertem $C_{1-12}$-Alkyl, durch Cyano substituiertem $C_{2-12}$-Alkyl, $C_{2-12}$-Cycloalkyl, enthaltend 1-3 Heteroatome, die aus N, O und S ausgewählt sind, $C_{2-12}$-Acyl, Sulfonyl ausgewählt ist;
vorzugsweise wobei in der Formel (I)
$R_7$ aus $C_{1-6}$-Alkyl, durch 1-4 Halogene, die aus Fluor, Chlor und Brom ausgewählt sind, substituiertem $C_{1-6}$-Alkyl, durch Hydroxy substituiertem $C_{1-6}$-Alkyl, durch Cyano substituiertem $C_{2-6}$-Alkyl, $C_{2-8}$-Cycloalkyl, enthaltend 1-3 Heteroatome, die aus N, O und S ausgewählt sind, $C_{2-6}$-Acyl, Sulfonyl ausgewählt ist.

**7.** Verbindung nach Anspruch 6, wobei in Formel (I)

$R_1$, $R_3$ und $R_4$ jeweils unabhängig aus der Gruppe bestehend aus H, Halogen, $C_{1-12}$-Alkyl, durch 1-6 Halogene substituiertem $C_{1-12}$-Alkyl ausgewählt sind;
$R_2$ Halogen ist;
$R_5$ aus H, $C_{1-12}$-Alkyl, durch 1-6 Halogene substituiertem $C_{1-12}$-Alkyl, durch Hydroxy substituiertem $C_{1-12}$-Alkyl, durch Alkoxy substituiertem $C_{2-12}$-Alkyl, durch Cyano substituiertem $C_{2-12}$-Alkyl und $C_{2-12}$-Cycloalkyl, enthaltend 1-3 Heteroatome, die aus N, O und S ausgewählt sind, ausgewählt ist;
$R_6$ aus der Gruppe bestehend aus $C_{1-12}$-Alkyl, durch Hydroxy substituiertem $C_{1-12}$-Alkyl, Halogen ausgewählt ist;
$R_7$ aus $C_{1-12}$-Alkyl, durch 1-6 Halogene substituiertem $C_{1-12}$-Alkyl, durch Hydroxy substituiertem $C_{1-12}$-Alkyl, durch Cyano substituiertem $C_{2-12}$-Alkyl, $C_{2-12}$-Cycloalkyl, enthaltend 1-3 Heteroatome, die aus N, O und S ausgewählt sind, $C_{2-12}$-Acyl, Sulfonyl ausgewählt ist;
vorzugsweise wobei in der Formel (I)
$R_1$, $R_3$ und $R_4$ jeweils unabhängig aus der Gruppe bestehend aus H, Fluor, Chlor, Brom, $C_{1-8}$-Alkyl, durch 1-6 Halogene, die aus Fluor, Chlor und Brom ausgewählt sind, substituiertem $C_{1-8}$-Alkyl ausgewählt sind;
$R_2$ F ist;
$R_5$ aus H, $C_{1-8}$-Alkyl, durch 1-6 Halogenatome, die aus Fluor, Chlor und Brom ausgewählt sind, substituiertem $C_{1-8}$-Alkyl, durch Hydroxy substituiertem $C_{1-8}$-Alkyl, durch Alkoxy substituiertem $C_{1-8}$-Alkyl, durch Cyano substituiertem $C_{2-8}$-Alkyl und $C_{2-10}$-Cycloalkyl, enthaltend 1-3 Heteroatome, die aus N, O und S ausgewählt sind, ausgewählt ist;
$R_6$ aus der Gruppe bestehend aus $C_{1-8}$-Alkyl, durch Hydroxy substituiertem $C_{1-8}$-Alkyl, Halogen ausgewählt ist;
$R_7$ aus $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Cyano substituiertem $C_{1-8}$-Alkyl, $C_{2-10}$-Cycloalkyl, enthaltend 1-3 Heteroatome, die aus N, O und S ausgewählt sind, $C_{2-8}$-Acyl, Sulfonyl ausgewählt ist.

**8.** Verbindung nach Anspruch 7, wobei in Formel (I)

$R_1$, $R_3$ und $R_4$ alle H sind; $R_2$ F ist;
$R_5$ aus H, $C_{1-8}$-Alkyl, durch 1-6 Halogenatome, die aus Fluor, Chlor und Brom ausgewählt sind, substituiertem $C_{1-8}$-Alkyl, durch Hydroxy substituiertem $C_{1-8}$-Alkyl, durch Alkoxy substituiertem $C_{2-8}$-Alkyl, durch Cyano substituiertem $C_{2-8}$-Alkyl und $C_{2-10}$-Cycloalkyl, enthaltend 1-3 Heteroatome, die aus N, O und S ausgewählt sind, ausgewählt ist;
$R_6$ aus der Gruppe bestehend aus $C_{1-8}$-Alkyl, durch Hydroxy substituiertem $C_{1-8}$-Alkyl, Halogen ausgewählt ist;
$R_7$ aus $C_{1-8}$-Alkyl, durch 1-6 Halogene, die aus Fluor, Chlor und Brom ausgewählt sind, substituiertem $C_{1-8}$-Alkyl, durch Hydroxy substituiertem $C_{1-8}$-Alkyl, durch Cyano substituiertem $C_{2-8}$-Alkyl, $C_{2-10}$-Cycloalkyl, enthaltend 1-3 Heteroatome, die aus N, O und S ausgewählt sind, $C_{2-8}$-Acyl, Sulfonyl ausgewählt ist;
vorzugsweise wobei die Verbindung der Formel (I) aus mindestens einer der folgenden Verbindungen ausgewählt ist:

Verbindung a1-2: 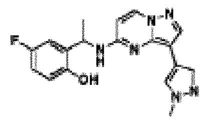 ; Verbindung a2-3: 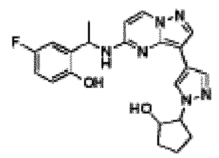

; Verbindung a3-4: 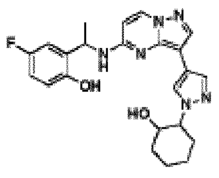 ; Verbindung a4-8: 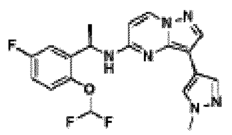 ;

Verbindung a5-9: 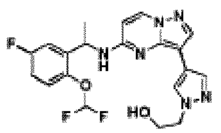 ; Verbindung a6-10: 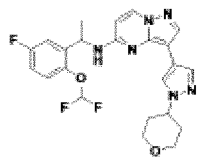 ;

Verbindung a7-12: 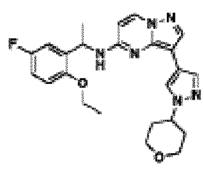 ; Verbindung a8-13: 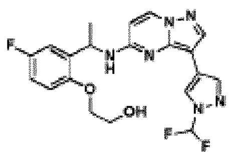 ;

Verbindung a9-14: 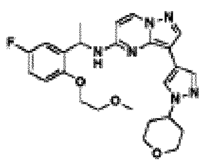 ; Verbindung a10-17: 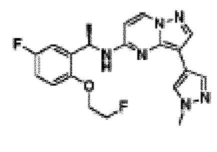 ;

Verbindung a11-18: 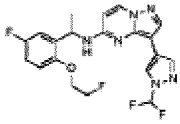 ; Verbindung a12-19: 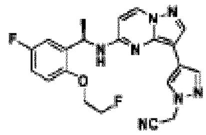 ;

Verbindung a13-20: 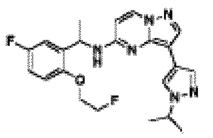 ; Verbindung a14-21: 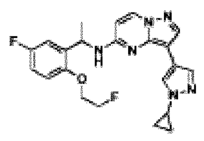 ;

Verbindung a15-22: 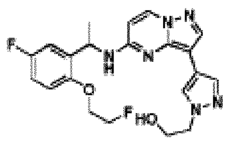 ; Verbindung a16-23: 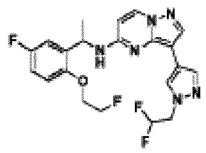

; Verbindung a17-24: 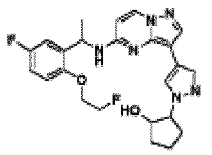 ; Verbindung a18-25: 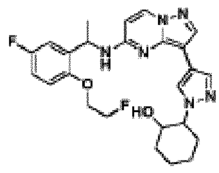

; Verbindung a19-26: 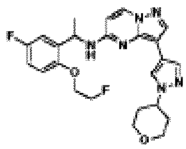 ; Verbindung a20-27: 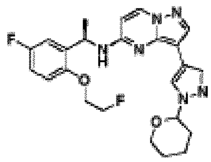

; Verbindung a21-28: 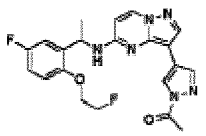 ; Verbindung a22-29: 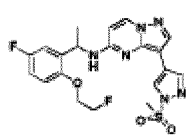

; Verbindung a23-31: 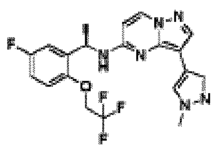 ; Verbindung a24-32:

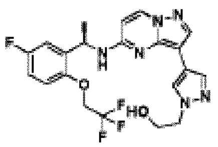 ; Verbindung a25-35: 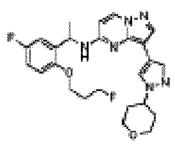 ; Verbindung a26-38:

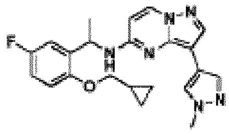 ; Verbindung a27-41: 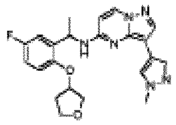 ; Verbindung a28-43:

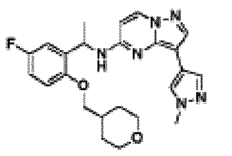.

**9.** Verbindung nach Anspruch 1 oder 2, wobei in Formel (I) $R_7$ H ist.

**10.** Verbindung nach Anspruch 9, wobei in Formel (I)

$R_1$, $R_3$ und $R_4$ jeweils unabhängig aus der Gruppe bestehend aus H, Halogen, $C_{1-12}$-Alkyl, durch 1-6 Halogene substituiertem $C_{1-12}$-Alkyl ausgewählt sind;
$R_2$ Halogen ist;
$R_5$ aus H, $C_{1-12}$-Alkyl, durch 1-6 Halogene substituiertem $C_{1-12}$-Alkyl, durch Hydroxy substituiertem $C_{1-12}$-Alkyl, durch Alkoxy substituiertem $C_{2-12}$-Alkyl, durch Cyano substituiertem $C_{2-12}$-Alkyl und $C_{2-12}$-Cycloalkyl, enthaltend 1-3 Heteroatome, die aus N, O und S ausgewählt sind, ausgewählt ist;
$R_6$ aus der Gruppe bestehend aus $C_{1-12}$-Alkyl, durch Hydroxy substituiertem $C_{1-12}$-Alkyl, Halogen ausgewählt ist;
vorzugsweise wobei in der Formel (I)
$R_1$, $R_3$ und $R_4$ jeweils unabhängig aus der Gruppe bestehend aus H, Fluor, Chlor, Brom, $C_{1-8}$-Alkyl, durch 1-6 Halogene, die aus Fluor, Chlor und Brom ausgewählt sind, substituiertem $C_{1-8}$-Alkyl ausgewählt sind;
$R_2$ F ist;
$R_5$ aus H, $C_{1-8}$-Alkyl, durch 1-6 Halogenatome, die aus Fluor, Chlor und Brom ausgewählt sind, substituiertem $C_{1-8}$-Alkyl, durch Hydroxy substituiertem $C_{1-8}$-Alkyl, durch Alkoxy substituiertem $C_{2-8}$-Alkyl, durch Cyano substituiertem $C_{2-8}$-Alkyl und $C_{2-10}$-Cycloalkyl, enthaltend 1-3 Heteroatome, die aus N, O und S ausgewählt sind, ausgewählt ist;
$R_6$ aus der Gruppe bestehend aus $C_{1-8}$-Alkyl, durch Hydroxy substituiertem $C_{1-8}$-Alkyl, Halogen ausgewählt ist;
vorzugsweise wobei in der Formel (I)
$R_1$, $R_3$ und $R_4$ alle H sind; $R_2$ F ist;
$R_5$ aus H, $C_{1-8}$-Alkyl, durch 1-6 Halogenatome, die aus Fluor, Chlor und Brom ausgewählt sind, substituiertem $C_{1-8}$-Alkyl, durch Hydroxy substituiertem $C_{1-8}$-Alkyl, durch Alkoxy substituiertem $C_{2-8}$-Alkyl, durch Cyano substituiertem $C_{2-8}$-Alkyl und $C_{2-10}$-Cycloalkyl, enthaltend 1-3 Heteroatome, die aus N, O und S ausgewählt sind, ausgewählt ist;
$R_6$ aus der Gruppe bestehend aus $C_{1-8}$-Alkyl, durch Hydroxy substituiertem $C_{1-8}$-Alkyl, Halogen ausgewählt ist.

**11.** Verbindung nach Anspruch 10, wobei die Verbindung der Formel (I) aus mindestens einer der folgenden Verbindungen ausgewählt ist:

Verbindung b1-1: 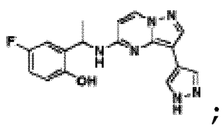 ; Verbindung b2-5: 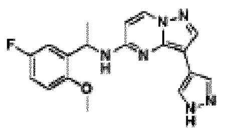

; Verbindung b3-6: 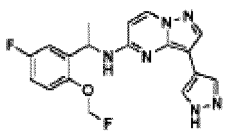 ; Verbindung b4-7: 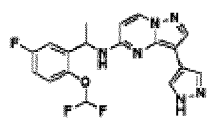 ;

Verbindung b5-11: 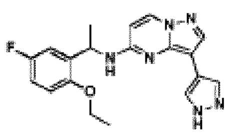 ; Verbindung b6-15: 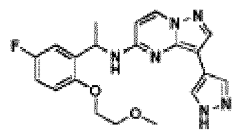

; Verbindung b7-16: 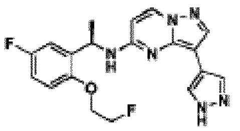 ; Verbindung b8-30: 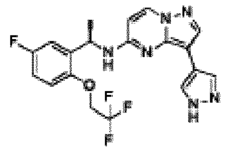

; Verbindung b9-33: 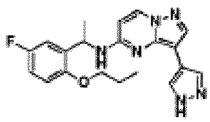 ; Verbindung b10-34: 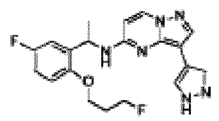

; Verbindung b11-36: 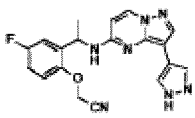 ; Verbindung b12-37: 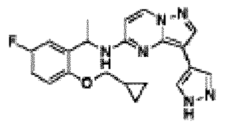

; Verbindung b13-39: 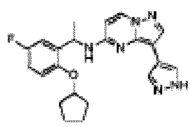 ; Verbindung b14-40: 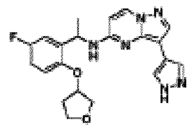

; Verbindung b15-42: 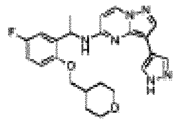 ; Verbindung b16-44: 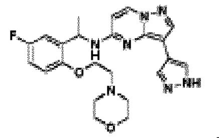 .

**12.** Pharmazeutische Zusammensetzung, umfassend einen pharmazeutisch unbedenklichen Trägerstoff, einen pharmazeutisch unbedenklichen Hilfsstoff oder ein pharmazeutisch unbedenkliches Verdünnungsmittel und als einen Wirkbestandteil eine Pyrazolopyrimidinverbindung mit einer Struktur, die durch Formel (I) dargestellt ist, oder ein pharmazeutisch unbedenkliches Salz davon oder ein Stereoisomer, ein geometrisches Isomer, ein Tautomer, ein Stickoxid, ein Hydrat, ein Solvat davon nach einem der Ansprüche 1 bis 11.

**13.** Pyrazolopyrimidinverbindung mit einer Struktur, die durch Formel (I) dargestellt ist, oder ein pharmazeutisch unbedenkliches Salz davon oder ein Stereoisomer, ein geometrisches Isomer, ein Tautomer, ein Stickoxid, ein Hydrat, ein Solvat davon nach einem der Ansprüche 1 bis 11 oder pharmazeutische Zusammensetzung nach Anspruch 12 zur Verwendung bei der Verhinderung und/oder Behandlung von Tumoren;
vorzugsweise wobei der Tumor mindestens einer bzw. eines bzw. eine von Brustkrebs, Dickdarmkrebs, Lungenkrebs, Schilddrüsenkrebs, Hautkrebs, Knochenkrebs, Melanom, Leukämie, Speicheldrüsentumor, neuroendokrinem Tumor, Lymphom, Hirntumor, Neuroblastom, Eierstockkrebs, Bauchspeicheldrüsenkrebs, Mesotheliom, Speiseröhrenkrebs, Lungensarkom, Medulloblastom, Glioblastom, Darmkrebs, Hepatom, Retinoblastom, Nierenkarzinom, Blasenkrebs, Osteosarkom, Magenkrebs, Gebärmutterkrebs, Vulvakrebs, Dünndarmkrebs, Prostatakrebs, Gallengangskrebs, Harnröhrenkrebs, Nebennierenrindenkrebs oder Kopf-Hals-Krebs ist.

## Revendications

**1.** Composé pyrazolopyrimidine ayant une structure représentée par la formule (I), ou un sel pharmaceutiquement acceptable de celui-ci, ou un stéréoisomère, un isomère géométrique, un tautomère, un oxyde d'azote, un hydrate, ou un solvate de celui-ci,

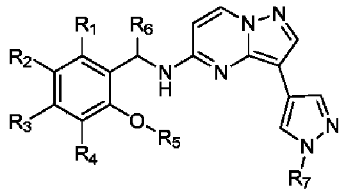

formule (I)

dans lequel, dans la formule (I),

chacun de $R_1$, $R_2$, $R_3$ et $R_4$ est indépendamment choisi parmi H, un halogène, un alkyle en $C_1$ à $C_{12}$, un alkyle en $C_1$ à $C_{12}$ substitué par 1 à 6 halogènes ;
$R_5$ est choisi parmi H, un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par alcoxy, un alkyle en $C_2$ à $C_{12}$ substitué par cyano, et un cycloalkyle en $C_2$ à $C_{12}$ contenant 1 à 3 hétéroatomes choisis parmi N, O et S ;
$R_6$ est choisi dans le groupe constitué par H, un alkyle en $C_1$ à $C_{12}$, un alkyle en $C_1$ à $C_{12}$ substitué par hydroxy, un halogène ;
$R_7$ est choisi parmi H, un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par cyano, un cycloalkyle en $C_2$ à $C_{12}$ contenant 1 à 3 hétéroatomes choisis parmi N, O et S, un acyle en $C_2$ à $C_{12}$, sulfonyle.

**2.** Composé selon la revendication 1, dans lequel, dans la formule (I),

chacun de $R_1$, $R_2$, $R_3$ et $R_4$ est indépendamment choisi dans le groupe constitué par H, le fluor, le chlore, le brome, un alkyle en $C_1$ à $C_8$, un alkyle en $C_1$ à $C_8$ substitué par 1 à 6 halogènes choisis dans le groupe constitué par le fluor, le chlore et le brome ;
$R_5$ est choisi parmi H, un alkyle en $C_1$ à $C_8$, un alkyle en $C_1$ à $C_8$ substitué par 1 à 6 atomes d'halogène choisis parmi le fluor, le chlore et le brome, un alkyle en $C_1$ à $C_8$ substitué par hydroxy, un alkyle en $C_2$ à $C_8$ substitué par alcoxy, un alkyle en $C_2$ à $C_8$ substitué par cyano, et un cycloalkyle en $C_2$ à $C_{10}$ contenant 1 à 3 hétéroatomes choisis parmi N, O et S ;
$R_6$ est choisi dans le groupe constitué par H, un alkyle en $C_1$ à $C_8$, un alkyle en $C_1$ à $C_8$ substitué par hydroxy, un halogène ;
$R_7$ est choisi parmi H, un alkyle en $C_1$ à $C_8$, un alkyle en $C_1$ à $C_8$ substitué par 1 à 6 halogènes choisis parmi le fluor, le chlore et le brome, un alkyle en $C_1$ à $C_8$ substitué par hydroxy, un alkyle en $C_2$ à $C_8$ substitué par cyano, un cycloalkyle en $C_2$ à $C_{10}$ contenant 1 à 3 hétéroatomes choisis parmi N, O et S, un acyle en $C_2$ à $C_8$, sulfonyle ;
de préférence, dans la formule (I),
chacun de $R_1$, $R_2$, $R_3$ et $R_4$ est indépendamment choisi dans le groupe constitué par H, le fluor, le chlore, le brome, un alkyle en $C_1$ à $C_6$, un alkyle en $C_1$ à $C_6$ substitué par 1 à 4 halogènes choisis dans le groupe constitué par le fluor, le chlore et le brome ;
$R_5$ est choisi parmi H, un alkyle en $C_1$ à $C_6$, un alkyle en $C_1$ à $C_6$ substitué par 1 à 4 halogènes choisis parmi le fluor, le chlore et le brome, un alkyle en $C_1$ à $C_6$ substitué par hydroxy, un alkyle en $C_2$ à $C_8$ substitué par alcoxy, un alkyle en $C_2$ à $C_6$ substitué par cyano, et un cycloalkyle en $C_2$ à $C_8$ contenant 1 à 3 hétéroatomes choisis parmi N, O et S ;
$R_6$ est choisi dans le groupe constitué par H, un alkyle en $C_1$ à $C_6$, un alkyle en $C_1$ à $C_6$ substitué par hydroxy, un halogène ;
$R_7$ est choisi parmi H, un alkyle en $C_1$ à $C_6$, un alkyle en $C_1$ à $C_6$ substitué par 1 à 4 halogènes choisis parmi le fluor, le chlore et le brome, un alkyle en $C_1$ à $C_6$ substitué par hydroxy, un alkyle en $C_2$ à $C_6$ substitué par cyano, un cycloalkyle en $C_2$ à $C_8$ contenant 1 à 3 hétéroatomes choisis parmi N, O et S, un acyle en $C_2$ à $C_6$, sulfonyle.

**3.** Composé selon la revendication 1 ou 2, dans lequel, dans la formule (I),

au moins l'un de $R_1$, $R_2$, $R_3$, $R_4$ et $R_5$ contient un atome F ;
et $R_5$ est choisi parmi les radicaux alkyle en $C_1$ à $C_{12}$ substitué par 1 à 6 halogènes ;
de préférence, dans la formule (I),
au moins l'un de $R_1$, $R_2$, $R_3$, $R_4$ et $R_5$ contient un atome F ;
et $R_5$ est choisi parmi les radicaux alkyle en $C_1$ à $C_8$ substitués par 1 à 6 halogènes choisis parmi le fluor, le

chlore et le brome.

**4.** Composé selon la revendication 3, dans lequel, dans la formule (I),

chacun de $R_1$, $R_2$, $R_3$ et $R_4$ est indépendamment choisi parmi H, un halogène, un alkyle en $C_1$ à $C_{12}$, un alkyle en $C_1$ à $C_{12}$ substitué par 1 à 6 halogènes ;
$R_5$ est $-CH_2CHF_2$ ;
$R_6$ est choisi dans le groupe constitué par un alkyle en $C_1$ à $C_{12}$, un alkyle en $C_1$ à $C_{12}$ substitué par hydroxy, un halogène ;
$R_7$ est choisi parmi H, un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par cyano, un cycloalkyle en $C_2$ à $C_{12}$ contenant 1 à 3 hétéroatomes choisis parmi N, O et S, un acyle en $C_2$ à $C_{12}$, sulfonyle ;
de préférence, dans la formule (I),
chacun de $R_1$, $R_2$, $R_3$ et $R_4$ est indépendamment choisi dans le groupe constitué par H, le fluor, le chlore, le brome, un alkyle en $C_1$ à $C_8$, un alkyle en $C_1$ à $C_8$ substitué par 1 à 6 halogènes choisis dans le groupe constitué par le fluor, le chlore et le brome ;
$R_5$ est $-CH_2CHF_2$ ;
$R_6$ est choisi dans le groupe constitué par un alkyle en $C_1$ à $C_8$, un alkyle en $C_1$ à $C_8$ substitué par hydroxy, un halogène ;
$R_7$ est choisi parmi H, un alkyle en $C_1$ à $C_8$, un alkyle en $C_1$ à $C_8$ substitué par 1 à 6 halogènes choisis parmi le fluor, le chlore et le brome, un alkyle en $C_1$ à $C_8$ substitué par hydroxy, un alkyle en $C_1$ à $C_8$ substitué par cyano, un cycloalkyle en $C_2$ à $C_{10}$ contenant 1 à 3 hétéroatomes choisis parmi N, O et S, un acyle en $C_2$ à $C_8$, sulfonyle ;
de préférence, dans la formule (I),
chacun de $R_1$, $R_2$, $R_3$ et $R_4$ est indépendamment choisi dans le groupe constitué par H, fluoro, chloro, bromo, un alkyle en $C_1$ à $C_8$, un alkyle en $C_1$ à $C_8$ substitué par 1 à 6 halogènes choisis parmi fluoro, chloro et bromo ;
$R_2$ est H ou F ;
$R_5$ est $-CH_2CHF_2$ ;
$R_6$ est choisi dans le groupe constitué par un alkyle en $C_1$ à $C_8$, un alkyle en $C_1$ à $C_8$ substitué par hydroxy, un halogène ;
$R_7$ est choisi parmi H, un alkyle en $C_1$ à $C_8$, un alkyle en $C_1$ à $C_8$ substitué par 1 à 6 halogènes choisis parmi le fluor, le chlore et le brome, un alkyle en $C_1$ à $C_8$ substitué par hydroxy, un alkyle en $C_2$ à $C_8$ substitué par cyano, un cycloalkyle en $C_2$ à $C_{10}$ contenant 1 à 3 hétéroatomes choisis parmi N, O et S, un acyle en $C_2$ à $C_8$, sulfonyle.

**5.** Composé selon la revendication 4, dans lequel le composé de formule (I) est au moins l'un choisi parmi les composés suivants :

Composé 1 : 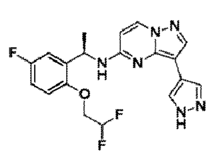
Composé 2 : 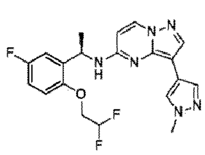
Composé 3 : 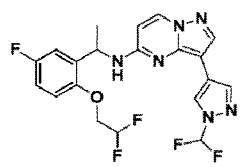

Composé 4 : 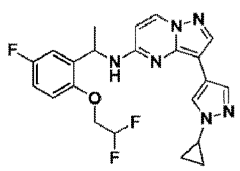
Composé 5 : 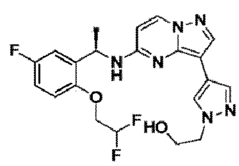
Composé 6 : 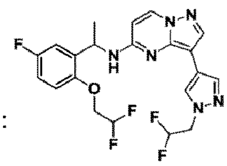

Composé 7 : 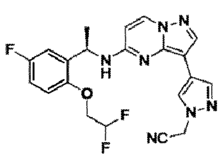
Composé 8 : 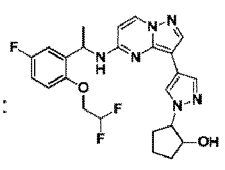
Composé 9 : 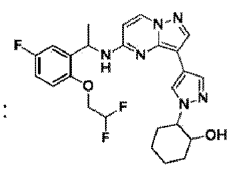

Composé 10 : 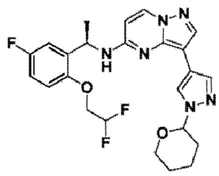 Composé 11 : 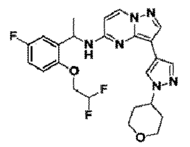 Composé 12 : 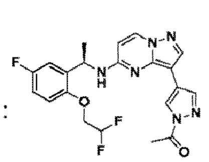

Composé 13 : 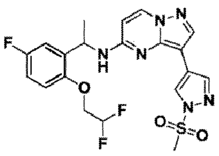 Composé 14 : 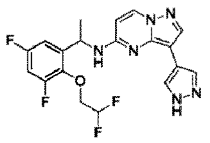 Composé 15 : 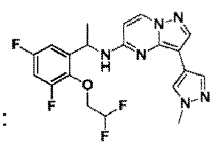

Composé 16 : 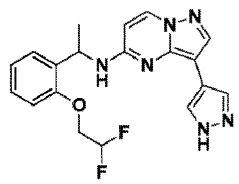 Composé 17 : 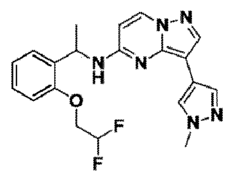

**6.** Composé selon la revendication 1 ou 2, dans lequel, dans la formule (I),

$R_7$ est choisi parmi un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par cyano, un cycloalkyle en $C_2$ à $C_{12}$ contenant 1 à 3 hétéroatomes choisis parmi N, O et S, un acyle en $C_2$ à $C_{12}$, sulfonyle ;
de préférence, dans la formule (I),
$R_7$ est choisi parmi un alkyle en $C_1$ à $C_6$, un alkyle en $C_1$ à $C_6$ substitué par 1 à 4 halogènes choisis parmi le fluor, le chlore et le brome, un alkyle en $C_1$ à $C_6$ substitué par hydroxy, un alkyle en $C_2$ à $C_6$ substitué par cyano, un cycloalkyle en $C_2$ à $C_8$ contenant 1 à 3 hétéroatomes choisis parmi N, O et S, un acyle en $C_2$ à $C_6$, sulfonyle.

**7.** Composé selon la revendication 6, dans lequel, dans la formule (I),

chacun de $R_1$, $R_3$ et $R_4$ est indépendamment choisi dans le groupe constitué par H, un halogène, un alkyle en $C_1$ à $C_{12}$, un alkyle en $C_1$ à $C_{12}$ substitué par 1 à 6 halogènes ;
$R_2$ est un halogène ;
$R_5$ est choisi parmi H, un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par alcoxy, un alkyle en $C_2$ à $C_{12}$ substitué par cyano, et un cycloalkyle en $C_2$ à $C_{12}$ contenant 1 à 3 hétéroatomes choisis parmi N, O et S ;
$R_6$ est choisi dans le groupe constitué par un alkyle en $C_1$ à $C_{12}$, un alkyle en $C_1$ à $C_{12}$ substitué par un hydroxy, un halogène ;
$R_7$ est choisi parmi un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par cyano, un cycloalkyle en $C_2$ à $C_{12}$ contenant 1 à 3 hétéroatomes choisis parmi N, O et S, un acyle en $C_2$ à $C_{12}$, sulfonyle ;
de préférence, dans la formule (I),
chacun de $R_1$, $R_3$ et $R_4$ est indépendamment choisi dans le groupe constitué par H, fluoro, chloro, bromo, un alkyle en $C_1$ à $C_8$, un alkyle en $C_1$ à $C_8$ substitué par 1 à 6 halogènes choisis parmi fluoro, chloro et bromo ;
$R_2$ est F ;
$R_5$ est choisi parmi H, un alkyle en $C_1$ à $C_8$, un alkyle en $C_1$ à $C_8$ substitué par 1 à 6 atomes d'halogène choisis parmi le fluor, le chlore et le brome, un alkyle en $C_1$ à $C_8$ substitué par hydroxy, un alkyle en $C_1$ à $C_8$ substitué par alcoxy, un alkyle en $C_2$ à $C_8$ substitué par cyano, et un cycloalkyle en $C_2$ à $C_{10}$ contenant 1 à 3 hétéroatomes choisis parmi N, O et S ;
$R_6$ est choisi dans le groupe constitué par un alkyle en $C_1$ à $C_8$, un alkyle en $C_1$ à $C_8$ substitué par hydroxy, un halogène ;
$R_7$ est choisi parmi un alkyle en $C_1$ à $C_8$, un alkyle en $C_1$ à $C_8$ substitué par 1 à 6 halogènes choisis parmi le fluor, le chlore et le brome, un alkyle en $C_1$ à $C_8$ substitué par hydroxy, un alkyle en $C_1$ à $C_8$ substitué par cyano, un cycloalkyle en $C_2$ à $C_{10}$ contenant 1 à 3 hétéroatomes choisis parmi N, O et S, un acyle en $C_2$ à $C_8$, sulfonyle.

**8.** Composé selon la revendication 7, dans lequel, dans la formule (I),

$R_1$, $R_3$ et $R_4$ sont tous H ; $R_2$ est F ;

$R_5$ est choisi parmi H, un alkyle en $C_1$ à $C_8$, un alkyle en $C_1$ à $C_8$ substitué par 1 à 6 atomes d'halogène choisis parmi le fluor, le chlore et le brome, un alkyle en $C_1$ à $C_8$ substitué par hydroxy, un alkyle en $C_2$ à $C_8$ substitué par alcoxy, un alkyle en $C_2$ à $C_8$ substitué par cyano, et un cycloalkyle en $C_2$ à $C_{10}$ contenant 1 à 3 hétéroatomes choisis parmi N, O et S ;

$R_6$ est choisi dans le groupe constitué par un alkyle en $C_1$ à $C_8$, un alkyle en $C_1$ à $C_8$ substitué par hydroxy, un halogène ;

$R_7$ est choisi parmi un alkyle en $C_1$ à $C_8$, un alkyle en $C_1$ à $C_8$ substitué par 1 à 6 halogènes choisis parmi le fluor, le chlore et le brome, un alkyle en $C_1$ à $C_8$ substitué par hydroxy, un alkyle en $C_2$ à $C_8$ substitué par cyano, un cycloalkyle en $C_2$ à $C_{10}$ contenant 1 à 3 hétéroatomes choisis parmi N, O et S, un acyle en $C_2$ à $C_8$, sulfonyle ;

de préférence le composé de formule (I) est au moins l'un choisi parmi les composés suivants :

Composé a1-2 : 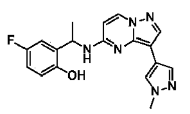 Composé a2-3 : 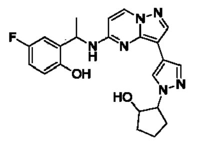 Composé a3-4 : 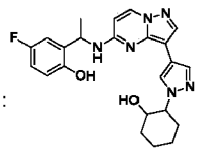

Composé a4-8 : 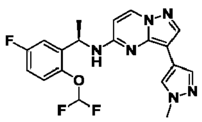 Composé a5-9 : 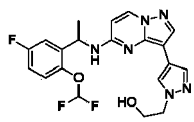 Composé a6-10 : 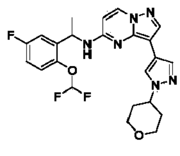

Composé a7-12 : 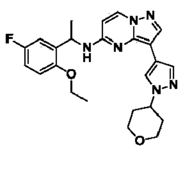 Composé a8-13 ; 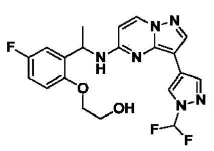 Composé a9-14 ; 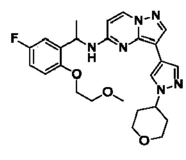

Composé a10-17 : 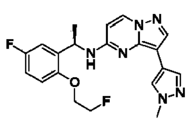 Composé a11-18 : 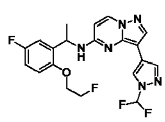 Composé a12-19 : 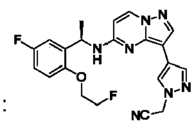

Composé a13-20 : 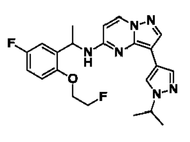 Composé a14-21 : 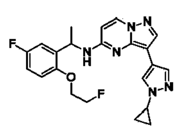 ; Composé a15-22 : 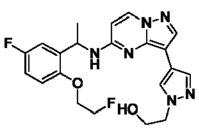

Composé a16-23 : 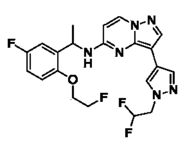 Composé a17-24 : 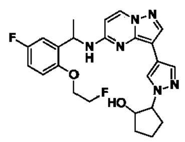 Composé a18-25 : 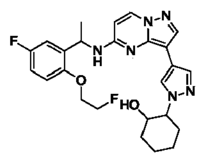

Composé a19-26 : 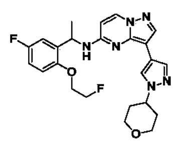 Composé a20-27 : 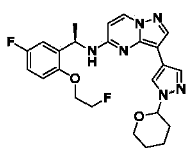 Composé a21-28 : 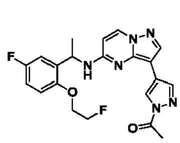

Composé a22-29 : 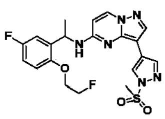 Composé a23-31 : 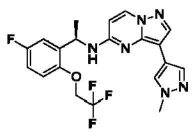 Composé a24-32 : 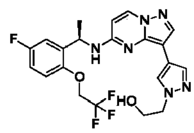

Composé a25-35 : 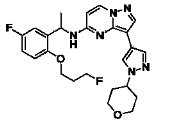 Composé a26-38 : 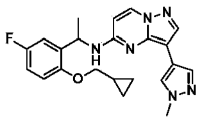 Composé a27-41 : 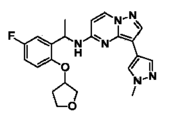

Composé a28-43 : 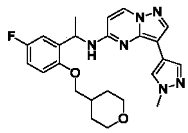

**9.** Composé selon la revendication 1 ou 2, dans lequel, dans la formule (I), $R_7$ est H.

**10.** Composé selon la revendication 9, dans lequel, dans la formule (I),

chacun de $R_1$, $R_3$ et $R_4$ est indépendamment choisi dans le groupe constitué par H, un halogène, un alkyle en $C_1$ à $C_{12}$, un alkyle en $C_1$ à $C_{12}$ substitué par 1 à 6 halogènes ;
$R_2$ est un halogène ;
$R_5$ est choisi parmi H, un alkyle en $C_1$ à $C_{12}$, un alkyle en $C_1$ à $C_{12}$ substitué par 1 à 6 halogènes, un alkyle en $C_1$ à $C_{12}$ substitué par hydroxy, un alkyle en $C_2$ à $C_{12}$ substitué par alcoxy, un alkyle en $C_2$ à $C_{12}$ substitué par cyano, et un cycloalkyle en $C_2$ à $C_{12}$ contenant 1 à 3 hétéroatomes choisis parmi N, O et S ;
$R_6$ est choisi dans le groupe constitué par un alkyle en $C_1$ à $C_{12}$, un alkyle en $C_1$ à $C_{12}$ substitué par hydroxy, un halogène ;
de préférence, dans la formule (I),
chacun de $R_1$, $R_3$ et $R_4$ est indépendamment choisi dans le groupe constitué par H, fluoro, chloro, bromo, un alkyle en $C_1$ à $C_8$, un alkyle en $C_1$ à $C_8$ substitué par 1 à 6 halogènes choisis parmi fluoro, chloro et bromo ;
$R_2$ est F ;
$R_5$ est choisi parmi H, un alkyle en $C_1$ à $C_8$, un alkyle en $C_1$ à $C_8$ substitué par 1 à 6 atomes d'halogène choisis parmi le fluor, le chlore et le brome, un alkyle en $C_1$ à $C_8$ substitué par hydroxy, un alkyle en $C_2$ à $C_8$ substitué par alcoxy, un alkyle en $C_2$ à $C_8$ substitué par cyano, et un cycloalkyle en $C_2$ à $C_{10}$ contenant 1 à 3 hétéroatomes choisis parmi N, O et S ;
$R_6$ est choisi dans le groupe constitué par un alkyle en $C_1$ à $C_8$, un alkyle en $C_1$ à $C_8$ substitué par hydroxy, un halogène ;
de préférence, dans la formule (I),
$R_1$, $R_3$ et $R_4$ sont tous H ; $R_2$ est F ;
$R_5$ est choisi parmi H, un alkyle en $C_1$ à $C_8$, un alkyle en $C_1$ à $C_8$ substitué par 1 à 6 atomes d'halogène choisis parmi le fluor, le chlore et le brome, un alkyle en $C_1$ à $C_8$ substitué par hydroxy, un alkyle en $C_2$ à $C_8$ substitué par alcoxy, un alkyle en $C_2$ à $C_8$ substitué par cyano, et un cycloalkyle en $C_2$ à $C_{10}$ contenant 1 à 3 hétéroatomes choisis parmi N, O et S ;
$R_6$ est choisi dans le groupe constitué par un alkyle en $C_1$ à $C_8$, un alkyle en $C_1$ à $C_8$ substitué par hydroxy, un halogène.

**11.** Composé selon la revendication 10, dans lequel le composé de formule (I) est au moins l'un choisi parmi les composés suivants :

Composé b1-1 : 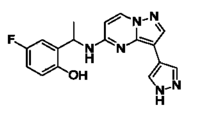 Composé b2-5 : 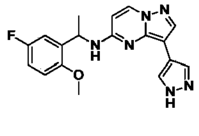 Composé b3-6 : 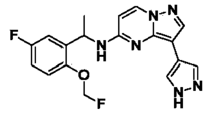

Composé b4-7 : 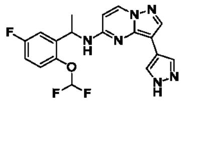 Composé b5-11 : 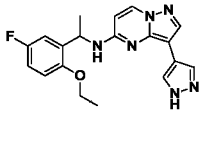 Composé b6-15 : 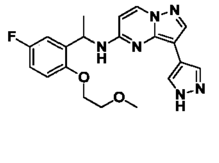

Composé b7-16 : 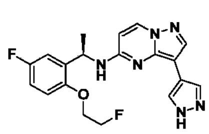 Composé b8-30 : 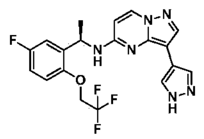 Composé b9-33 : 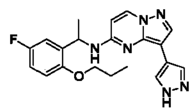

Composé b10-34 : 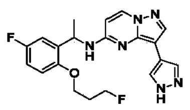 Composé b11-36 : 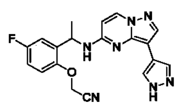 Composé b12-37 : 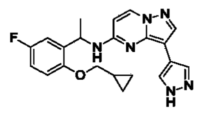

Composé b13-39 : 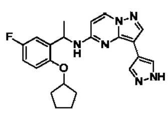 Composé b14-40 : 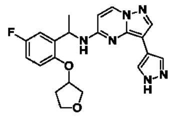 Composé b15-42 : 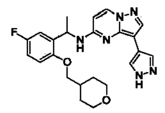

Composé b16-44 : 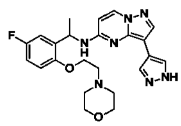

**12.** Composition pharmaceutique comprenant un véhicule, excipient ou diluant pharmaceutiquement acceptable, et, en tant que principe actif, un composé pyrazolopyrimidine ayant une structure représentée par la formule (I) ou un sel pharmaceutiquement acceptable de celui-ci, ou un stéréoisomère, un isomère géométrique, un tautomère, un oxyde d'azote, un hydrate, ou un solvate de celui-ci, selon l'une quelconque des revendications 1 à 11.

**13.** Composé pyrazolopyrimidine ayant une structure représentée par la formule (I), ou un sel pharmaceutiquement acceptable de celui-ci, ou un stéréoisomère, un isomère géométrique, un tautomère, un oxynitrure, un hydrate, ou un solvate, selon l'une quelconque des revendications 1 à 11, ou composition pharmaceutique selon la revendication 12, pour une utilisation dans la prévention et/ou le traitement de tumeurs ;
de préférence laquelle tumeur est au moins l'un parmi un cancer du sein, un cancer du gros intestin, un cancer du poumon, un cancer de la thyroïde, un cancer de la peau, un cancer des os, un mélanome, une leucémie, une tumeur des glandes salivaires, une tumeur neuroendocrinienne, un lymphome, une tumeur cérébrale, un neuroblastome, un cancer ovarien, un cancer du pancréas, un mésothéliome, un cancer de l'œsophage, un sarcome pulmonaire, un médulloblastome, un glioblastome, un cancer du côlon, un hépatome, un rétinoblastome, un carcinome rénal, un cancer de la vessie, un ostéosarcome, un cancer gastrique, un cancer utérin, un cancer vulvaire, un cancer de l'intestin grêle, un cancer de la prostate, un cancer du canal cholédoque, un cancer de l'uretère, un cancer du cortex surrénal, ou un cancer de la tête et du cou.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010048314 A
- WO 2012116217 A
- WO 2011146336 A
- WO 2010033941 A
- WO 2018077246 A
- WO 2016097869 A1
- WO 2007147647 A
- WO 2007025540 A


### Non-patent literature cited in the description


- **BAILEY et al.** *EXPERT OPIN. THER. PATENTS,* 2017, vol. 27, 733-751
- **RUSSO M et al.** *Cancer Discovery,* 2016, vol. 6 (1), 36-44
- **DRILON A. et al.** *Annals of Oncology,* 2016, vol. 27 (5), 920-926
- **CUI J. et al.** *J. Med. chem.,* 2011, vol. 54, 6342-6363